# EUROPÄISCHE PATENTSCHRIFT

(11) **EP 2 054 406 B1**
(45) Veröffentlichungstag und Bekanntmachung des Hinweises auf die Patenterteilung: **07.10.2015**
(21) Anmeldenummer: 07786668.9
(22) Anmeldetag: 13.08.2007
(51) Int. Cl.: C07D 405/04, A61K 31/513, A61P 35/00

(54) **NUKLEOSIDE ZUR UNTERDRÜCKUNG ODER REDUZIERUNG DER RESISTENZBILDUNG BEI DER ZYTOSTATIKA-BEHANDLUNG**
NUCLEOSIDES FOR SUPPRESSING OR REDUCING THE DEVELOPMENT OF RESISTANCE IN CYTOSTATIC THERAPY
NUCLÉOSIDE POUR LA SUPPRESSION OU LA RÉDUCTION DE LA FORMATION DE RÉSISTANCE LORS DU TRAITEMENT CYTOSTATIQUE

(30) Priorität: 11.08.2006 DE 102006037786
(43) Veröffentlichungstag der Anmeldung: 06.05.2009
(73) Patentinhaber: Resprotect GmbH, 01307 Dresden (DE)
(72) Erfinder: FAHRIG, Rudolf, 01326 Dresden (DE); LOHMANN, Dieter, 01445 Radebeul (DE); ROLFS, Andreas, 99817 Eisenach (DE); DIEKS, Henrik, 14197 Berlin (DE); TEUBNER, Janek, 12459 Berlin (DE); HEINRICH, Jörg-Christian, 01309 Dresden (DE)
(74) Vertreter: Pfenning, Meinig & Partner GbR
(86) Internationale Anmeldenummer: PCT/EP2007/007147
(87) Internationale Veröffentlichungsnummer: WO 2008/017515

(56) Entgegenhaltungen:
- EP-A- 0 097 039
- EP-A- 0 104 857
- EP-A2- 0 368 668
- WO-A-02/39952
- WO-A-90/15064
- WO-A-96/23506
- WO-A-98/42351
- WO-A-2004/084917
- WO-A-2005/012327
- DE-A1- 3 233 198

## Beschreibung

Die Erfindung betrifft spezielle Nukleoside, sowie Arzneimittel, die diese Nukleoside enthalten. Weiterhin betrifft die Erfindung die Verwendung derartiger Nukleoside zur Herstellung eines Arzneimittels, insbesondere zur Unterdrückung oder Reduzierung der Resistenzenbildung bei der Zytostatika-Behandlung.

Standardtherapie für Krebserkrankungen ist die Chemotherapie. Zytostatika beeinflussen die Zellteilung und sind dementsprechend besonders toxisch für schnell wachsende Tumorzellen. Zytostatika induzieren Apoptose, d.h. sie führen zum Zelltod der Tumorzellen. Leider ist die resistenzfreie Behandlungsperiode mit den gegenwärtig auf dem Markt befindlichen Zytostatika meist nicht lang genug, um den Tumor gänzlich zu vernichten. Zur Verbesserung dieser Situation wurden "Chemosensitizer" entwickelt, die bestehender Resistenz entgegenwirken.

Wird die Resistenz durch die Amplifikation (Vervielfachung) und Überexpression des "multi-drug resistance"-Gens (MDR-1) hervorgerufen, kann diese durch Inaktivierung von dessen Genprodukt (P-Glykoprotein) vermindert werden (Takara K, Sakaeda T, Okumura K. An update on overcoming MDR1-mediated multidrug resistance in cancer chemotherapy. Curr. Pharm. Des. 2006; 12 (3) : 273-86) .

Schwere Nebenwirkungen standen bisher einem Einsatz von P-Glykoprotein-Hemmern im Wege. Substanzen der dritten Generation sind wahrscheinlich wegen ihrer toxischen Wirkung nur für eine Kurzzeitbehandlung einsetzbar und auch nur bei den wenigen Tumoren, deren Resistenz ausschließlich auf der Wirkung des "multi-drug resistance"-Gens beruht. Darüber hinaus wurden Inhibitoren der Rezeptoren für Tyrosinkinase oder der Überexpression einzelner Onkogene entwickelt. Behandelt werden können allerdings immer nur einige wenige geeignete Tumoren (Desoize B., Jardillier J., Multicellular resistance: a paradigm for clinical resistance? Crit Rev Oncol Hematol. 2000; 36:193-207).

Aus der EP 0 806 956 sind 5-substituierte Nukleoside zur Hemmung der Resistenzbildung bei der Zytostatika-Behandlung bekannt. Bei den hier ausgeführten Verbindungen handelt es sich um (E)-5'-(2-bromovinyl)-2'-deoxyuridin (BVDU) und (E)-5'-(2-bromovinyl)uracil (BVU).

Aus WO 2004/084917 A, WO 02/39952 A, WO 2005/012327 A, WO 90/15064 A, EP-A-0 104 857, EP-A-0 097 039, DE 32 33 198 A1 WO 98/42351 und EP-A2-0 368 668 sind weitere BVDV-Derivate bekannt.

Diese verhindern die Resistenzbildung und bekämpfen nicht schon existierende Resistenzen. Im Gegensatz zu den seit Jahrzehnten bekannten und meist erfolglosen Versuchen, schon existierende Chemoresistenzen zu umgehen oder zu mindern, gibt es für diesen technologischen Ansatz weltweit keine Konkurrenz (Fahrig, R., Heinrich, J.C., Nickel, B., Wilfert, F., Leisser, C., Krupitza, G., Praha, C., Sonntag, D., Fiedler, B., Scherthan, H., and Ernst, H. Inhibition of induced chemoresistance by co-treatment with (E)-5-(2-bromovinyl)-2'-deoxyuridine (RP101). Cancer Res. 63 (2003) 5745 -5753). Das erste Arzneimittel BVDU zeigte in zwei klinischen Studien mit Pankreas (Bauchspeicheldrüsen)-Krebspatienten eine statistisch signifikante Wirkung. Die Wirkung der Kobehandlung von Zytostatika mit BVDU war wirkungsvoller als jede andere bisher beschriebene Chemotherapie.

Daher war es Aufgabe der vorliegenden Erfindung, Substanzen bereitzustellen, die eine gegenüber den aus dem Stand der Technik bekannten Verbindungen höhere Wirksamkeit im Hinblick auf die Unterdrückung oder Reduzierung der Resistenzenbildung bei der Zytostatika-Behandlung aufweisen.

Diese Aufgabe wird durch die Nukleoside mit den Merkmalen des Anspruchs 1 und das Arzneimittel mit den Merkmalen des Anspruchs 4 gelöst. In Anspruch 6 wird die Verwendung derartiger Nukleoside bereitgestellt. Die weiteren abhängigen Ansprüche zeigen vorteilhafte Weiterbildungen auf.

Erfindungsgemäß werden Nukleoside der allgemeinen Formel I bereitgestellt,

Nukleoside der allgemeinen Formel I 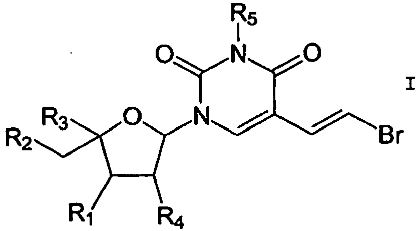 mit
R₁ = Halogen,
R₂ ausgewählt aus der Gruppe bestehend aus H, Halogen, OR₈, CN,
N₃, NR₆R₇ und über ein Sauerstoffatom gebundenen Prodrugresten, wobei
   der Prodrugrest ausgewählt ist aus der Gruppe bestehend aus 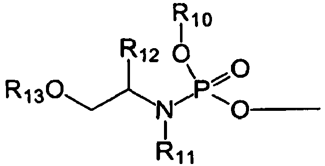 mit
   R₁₀ und R₁₁ unabhängig voneinander geradkettiger oder verzweigter C₁-C₈-Alkyl, Aryl, der auch Heteroatome aufweisen kann, R₁₂ = geradkettiger oder verzweigter C₁-C₈-Alkyl, Aralkylen, Aryl, der auch Heteroatome aufweisen kann, R₁₃ = geradkettiger oder verzweigter C₁-C₈-Alkyl, 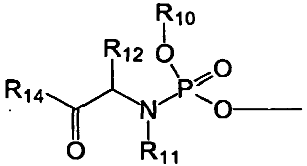 mit
   R₁₀ und R₁₁ unabhängig voneinander geradkettiger oder verzweigter C₁-C₈-Alkyl, Aryl, der auch Heteroatome aufweisen kann, R₁₂ = geradkettiger oder verzweigter C₁-C₈-Alkyl, Aralkylen, Aryl, der auch Heteroatome aufweisen kann, R₁₃ = geradkettiger oder verzweigter C₁-C₈-Alkyl, R₁₄ = O-C₁-C₈-Alkyl, NH-C₁-C₈-Alkyl, N-(C₁-C₈-Alkyl)₂, 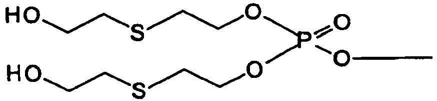 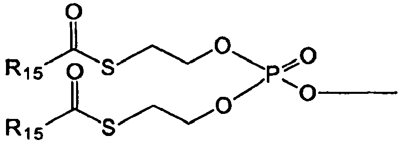 mit
   R₁₅ = geradkettiger oder verzweigter C₁-C₈-Alkyl, 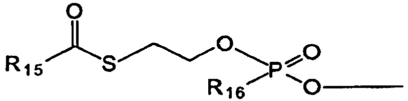 mit
   R₁₅ = geradkettiger oder verzweigter C₁-C₈-Alkyl, R₁₆ = geradkettiger oder verzweigter C₁-C₈-Alkyl, O-Aryl 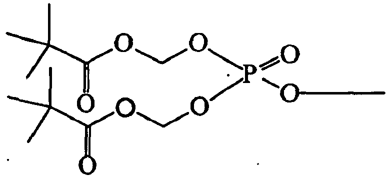 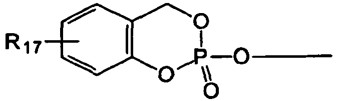 mit
   R₁₇ = geradkettiger oder verzweigter C₁-C₈-Alkyl, Halogen, 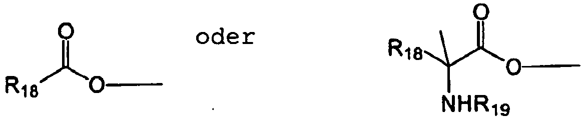 mit
   R₁₈ = geradkettig oder verzweigt C₁-C₈-Alkyl, Aryl oder Heteroaryl, R₁₉ = Alkyl-CO-, Aryl-CO-, Aminosäure oder Peptid, 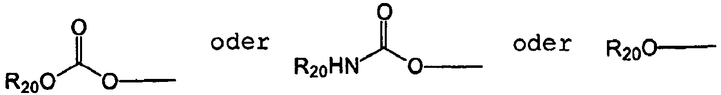 mit
   R₂₀ = geradkettig oder verzweigt C₁-C₈-Alkyl,
   R₃ = H, geradkettiges oder verzweigtes C₁-C₈-Alkyl, geradkettiges oder verzweigtes C₁-C₈-Alkylen,
   R₄ = H, Halogen, OR₈, N₃, NR₆R₇ oder R₄ gemeinsam mit R₁ eine zweite Bindung zwischen den zu R₁ und R₄ benachbarten C-Atomen darstellen,
   R₅ = H, C₁-C₈-Alkyl oder Aryl und
   R₆, R₇ und R₈ unabhängig voneinander H, geradkettiges oder verzweigtes C₁-C₈-Alkyl oder Acetyl, wobei die Verbindungen mit folgenden Resten ausgeschlossen sind:
   R₁ = F, R₂ = OH, R₃ = R₄ = R₅ = H,
   R₁ = F, R₂ = O-Acetyl, R₃ = R₄ = R₅ = H,
   R₁ = F, R₂ = Triphosphat, R₃ = R₄ = R₅ = H,
   R₁ = Cl, R₂ = OH, R₃ = R₄ = R₅ = H.

Ebenso werden Arzneimittel, die Nukleoside der allgemeinen Formel I enthalten, bereitgestellt.

Hinsichtlich der Verwendung betrifft diese insbesondere die Unterdrückung bzw. Reduzierung der Resistenzenbildung bei der Zytostatika-Behandlung. Hierbei werden Nukleoside der allgemeinen Formel I eingesetzt, 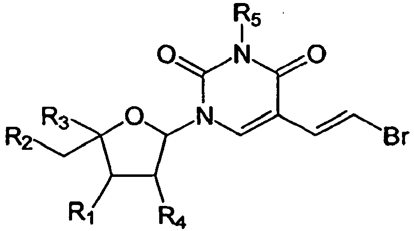 mit
R₁ = Halogen,
R₂ ausgewählt aus der Gruppe bestehend aus H, Halogen, OR₈, CN, N₃, NR₆R₇ und über ein Sauerstoffatom gebundenen Prodrugresten,
R₃ = H, geradkettiges oder verzweigtes C₁-C₈-Alkyl, geradkettiges oder verzweigtes C₁-C₈-Alkylen,
R₄ = H, Halogen, OR₈, N₃, NR₆R₇ oder R₄ gemeinsam mit
R₁ eine zweite Bindung zwischen den zu R₁ und R₄ benachbarten C-Atomen darstellen,
R₅ = H, C₁-C₈-Alkyl oder Aryl und
R₆, R₇ und R₈ unabhängig voneinander H, geradkettiges oder verzweigtes C₁-C₈-Alkyl oder Acetyl, sowie mindestens einem Zytostatikum.

Weitere Verwendungen betreffen die resistenzenfreie Therapie von durch Bakterien, Plasmodien oder Leishmanien verursachten Infektionskrankheiten.

Die Anwendung kann dabei sowohl in einer einzigen Formulierung, d.h. als Kombinationspräparat, oder auch in getrennten Formulierungen erfolgen. Bei der getrennten Formulierung ist auch eine zeitlich versetzte Verabreichung der beiden Formulierungen möglich.

Hinsichtlich der Galenik bestehen keinerlei Beschränkungen, sodass sämtliche aus dem Stand der Technik bekannten Formulierungsformen eingesetzt werden können. Hierzu zählen beispielsweise Tabletten, Kapseln, Sprays, Dragees, Emulsionen, Liquida sowie Ampullen. Entgegen der aktuellen und nicht sehr erfolgreichen Vorgehensweise, bestehende Resistenzen zu bekämpfen, greifen die erfindungsgemäß aufgeführten Substanzen schon viel früher ins Geschehen ein. Sie verhindern die Entstehung von Resistenzen. Es werden also die Ursachen und nicht die Symptome einer Krankheit bekämpft.

Anhand der nachfolgenden Figuren und Beispiele soll der erfindungsgemäße Gegenstand näher erläutert werden, ohne diesen auf die hier gezeigten speziellen Ausführungsformen einschränken zu wollen.

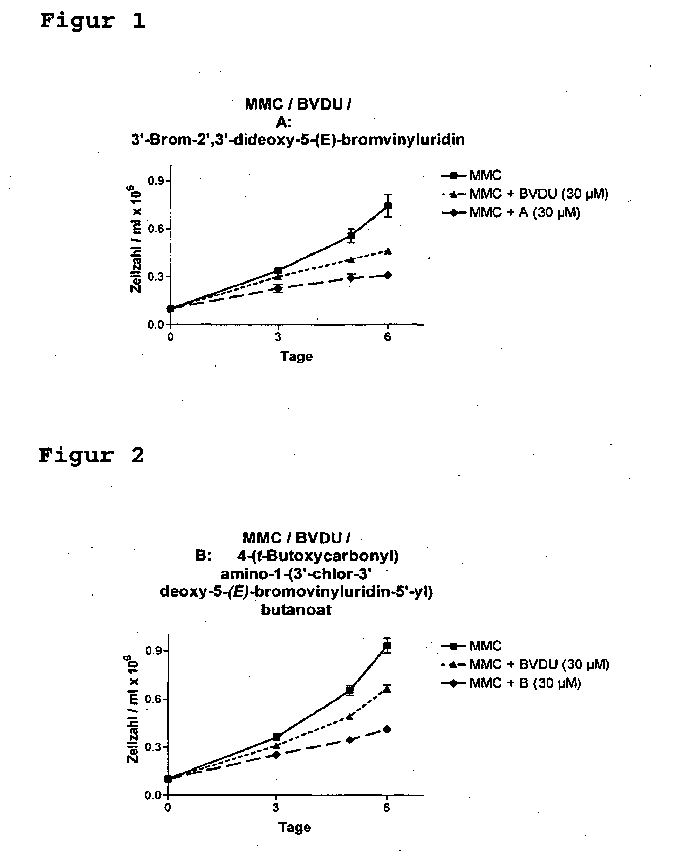

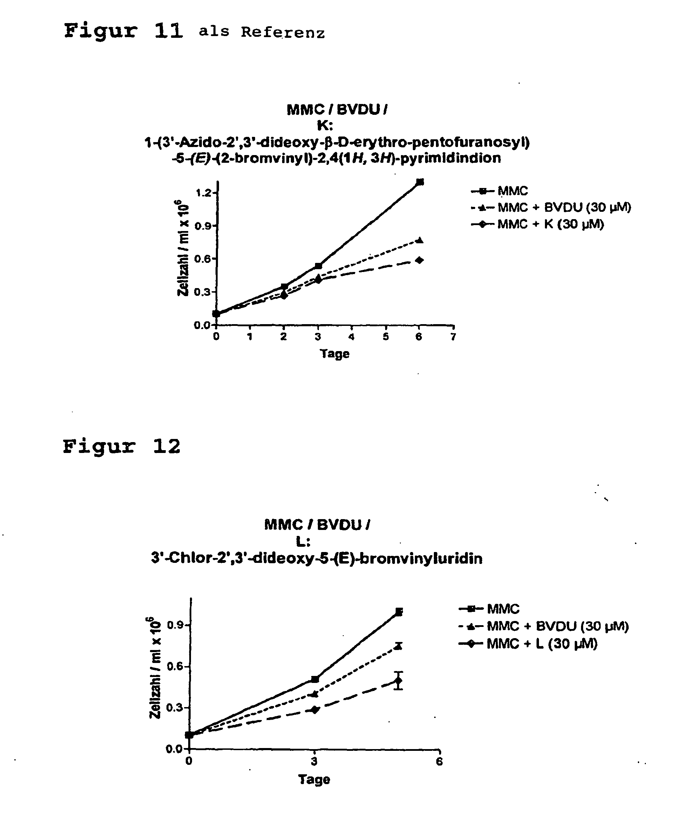

Die Figuren 1 bis 3, 7, 8 und 12 zeigen den Einfluss erfindungsgemäßer Verbindungen in Kombination mit Mitomycin C (MMC) im Vergleich zur alleinigen Verabreichung von MMC bzw. zur Verabreichung von MMC mit BVDU auf die Zellzahl von AH13r-Zellen im zeitlichen Verlauf. AH13r-Zellen wurden dabei steigenden Dosen des Zytostatikums MMC ausgesetzt. Aus diesen Figuren ist zu erkennen, dass die Wirkung von MMC zusammen mit den erfindungsgemäßen Verbindungen im Vergleich zu MMC und BVDU deutlich stärker ist.

### Beispiele

### Allgemeines:

Die Edukte 5'-O-(4-Chlorbenzoyl)-2'-desoxy-5-(E)-bromvinyluridin (P. Herdewijn, J. Balzarini, E. De Clerq, R. Pauwels, M. Baba, J. Med. Chem. 1987, 30, 1270-1278), 5'-O-Methylsulfonyl-2'-desoxy-5-(E)-bromvinyluridin (P. M. Reddy, T. C. Bruice; Bioorg. Med. Chem. Lett. 2003, 13; 1281-1286), 5'-O-Trityl-2'-desoxy-5-ethyl-uridin (C. K. Chu, R. F: Raymond, M. K: Ahn, V. Giliyar, Z. P. Gu, J. Med. Chem. 1989, 32, 612-617), S-(2-Hydroxyethyl)-thiopivaloat (I. Lefebrve, C. Périgaud, A. Pompon, A.-M. Aubertin, J.-L. Girardet, A. Kirn, G. Gilles und J.-L. Imbach, J. Med. Chem. 1995, 38, 3941-3950), 5-(E)-Bromvinyluridin (E. De Clercq, C. Desgranges, P. Herdewijn, A. S. Jones, M. J. McLean, R. T. Walker, J. Med Chem. 1986, 29, 213-217), 5-Ioduracil (J. Asakura, M. J. Robins, J. Org. Chem.1990, 55, 4928-4933), 2'-Desoxy-2'-fluorouridin (Y. Saito, K. Utsumi, T. Maruyama, T. Kimura, I. Yamamoto, D. D. Richman, Chem. Pharm. Bull. 1994, 42, 595-598), 5-Iod-2'-desoxy-2'-fluorouridin (T. Kniess, M. Grote, B. Noll, B. Johannsen, Z. Naturforsch. 2003, 58b, 226-230), 3-(2'-Desoxy-2'-fluorouridin-5-yl)-acrylsäuremethylester (J. Matulic-Adamic, A. T. Daniher, A. Karpeisky, P. Haeberli, D. Sweedler, L. Beigelman, Bioorg. Med. Chem. Lett. 2000, 10, 1299-1302), 3,5-Di-O-benzoyl-2-desoxy-2-fluoro-α-D-arabinosylbromid, 1-(2'-Desoxy-2'-fluor-β-D-arabinofuranosyl)-5-ethyluracil (H. G. Howell, P. R. Brodfuehrer, S. R. Brundige, D. A. Benigni, C. Sapino, J. Org. Chem. 1988, 53, 85-88), 5-(E)-Bromvinyluracil (P. J. Barr, A. S. Jones, G. Verhelst, R. T. Walker, J. Chem. Soc. Perkin Transactions 1, 1981, 565-570) und 1-(2,3,6-Tri-O-acetyl-β-D-arabinofuranosyl)-5-ioduracil (M. J. Robins, S. Manfredini, S. G. Wood, R. J. Wanklin, B. A. Rennie, S. L. Sacks, J. Med. Chem.1991, 34, 2275-2280), 5'-Amino-2',5'-didesoxy-5-(E)-bromvinyluridin, 5'-Brom-2',5'-didesoxy-5-(E)-bromvinyluridin, 5'-Chlor-2',5'-didesoxy-5-(E)-bromvinyluridin, 5'-Azido-2',5'-dides-oxy-5-(E)-bromvinyluridin 3'-Chlor-2',3'-didesoxy-5-(E)-bromvinyluridin und 3'-Azido-2',3'-didesoxy-5-(E)-bromvinyluridin (R. Busson, L. Colla, H. Vanderhaeghe, E. De Clercq, Nucleic Acids Research, Symposium Series, 1981, 9, 49-52), 3'-O-(t-Butyldi-methylsilyl)-2'-desoxy-5-(E)-bromvinyluridin (P. Herdewijn, R. Ramamurthy, E. De Clercq, W. Pfleiderer, Helv. Chim. Acta, 1989, 72, 1739-1748), 2'-Desoxy-3'-methoxy-5-(E)-bromvinyluridin (M. Ashwell, A. S. Jones, A. Kumar, J. R. Sayers, R. T. Walker, Tetrahedron, 1987, 43, 20, 4601-4608) wurden analog den genannten Literaturangaben synthetisiert.

Alle weiteren Edukte waren kommerziell erhältlich.

Die säulenchromatographische Reinigung der Substanzen erfolgte mit den angegebenen Laufmitteln auf Kieselgel 60 (FLUKA, 0,040 - 0,063 mm). Für DünnschichtChromatographie wurden Kieselgelfolien (Merck, Kieselgel 60 F₂₅₄) verwendet.

Die ³¹P-NMR-Spektren wurden mit 85 % Phosphorsäure als externer Standard gemessen.

### 1. 3'-Substituierte BVDU-Derivate

### 1.1. 3'-Halogen-BVDU

### 1.1.1. 2',3'-Didesoxy-3'-brom-5-(E)-bromvinyl-uridin

### 1.1.1.1. 2,3'-Anhydro-2'-desoxy-5'-O-benzoyl-5-(E)-bromvinyluridin

Eine Lösung von 13,8 g (68 mmol) Diisopropylazodicarbonester und 8,3 g (68 mmol) Benzoesäure in 75 ml DMF werden innerhalb 155 min in eine Lösung von 15,0 g (45 mmol) BVDU und 17,8 g (68 mmol) Triphenylphosphin zugetropft. Danach lässt man noch 30 min bei Raumtemperatur rühren und gibt nochmals 17,8 g Triphenylphosphin hinzu. Dazu tropft man innerhalb 10 min eine Lösung von 13,8 g (68 mmol) Diisopropylazodicarbonester in 10 ml DMF und lässt 3 h bei Raumtemperatur rühren. DC-Kontrolle (Dichlormethan/Methanol 5:1) ergibt, dass die Reaktion beendet ist. Das Reaktionsgemisch wird in 1,5 l Diethylether eingegossen, der ausgefallene Niederschlag abgesaugt und mit Diethylether gewaschen. Nach Trocknung beträgt die Ausbeute 10,1 g (53,5 %).

### 1.1.1.2. 2',3'-Didesoxy-3'-brom-5'-O-benzoyl-5-(E)-bromvinyluridin

3,0 g (7,16 mmol) 2,3'-Anhydro-2'-desoxy-5'-O-benzoyl-5-(E)-bromvinyluridin und 2,40 g (15,0 mmol) Pyridiniumhydrobromid werden in 20 ml DMF suspendiert und auf 100 °C erhitzt. Nach 3 h ist die Reaktion beendet (DC-Kontrolle mit Dichlormethan/Methanol 20:1). Die erhaltene Lösung wird mit 200 ml Wasser verdünnt und mit Essigester extrahiert. Die vereinigten Essigesterphasen werden zweimal mit je 100 ml 0,5 M Salzsäure und dreimal mit 100 ml Kochsalzlösung gewaschen, mit Magnesiumsulfat getrocknet, abfiltriert und zuerst mit Dichlormethan/Essigester 12:1, dann mit Chloroform/Methanol 25:1 mehrfach säulenchromatographisch gereinigt. Die Ausbeute beträgt 2,0 g (55,8 %) eines farblosen Schaums.

### 1.1.1.3. 2',3'-Didesoxy-3'-brom-5-(E)-bromvinyl-uridin

1,92 g (3,84 mmol) des 2',3'-Didesoxy-3'-brom-5'-O-benzoyl-5-(E)-bromvinyluridin werden 25 ml THF gelöst und mit 10 ml Wasser und mit 9,6 ml (19,2 mmol) 2 M Natronlauge versetzt. Nach 2,5 h ist die Umsetzung beendet (DC-Kontrolle mit Chloroform/Methanol 15:1). Der Ansatz wird in 50 ml gesättigte Kochsalzlösung eingegossen und mit Essigester extrahiert. Die vereinigten Extrakte werden mit Magnesiumsulfat getrocknet. Nach Filtration und Abdestillieren des Lösungsmittels werden durch wiederholte säulenchromatogra-phische Reinigung (Chloroform/Methanol 15:1) 1,3 g (81,2 %) eines weißen, festen Schaums erhalten.

¹H-NMR (500MHz, DMSO-d₆) : 2,45 (m, 1H); 2,72 (m, 1H); 3,71 (m, 2H); 4,19 (m, 1H); 4,61 (m, 1H); 5,31 (s, 1H); 6,17 (t, 1H); 6,83 (d, 1H); 7,25 (d, 1H); 8,07 (s, 1H); 11,60 (s, 1H) ppm.

Fig. 1 zeigt die Ergebnisse dieser erfindungsgemäßen Verbindung in Kombination mit Mitomycin C (MMC) im Vergleich zu MMC alleine und MMC in Kombination mit BVDU.

### 1.1.2. 1-(3'-Chlor-2,3-didesoxy-β-D-threo-pentofu-ranosyl)-5-(E)-(2-bromvinyl)-2,4-(1H,3H)-pyrimidindion

### 1.1.2.1. 1-(5'-O-(4-Chlorbenzoyl)-3'-Chlor-2',3'-didesoxy-β-D-threo-pentofuranosyl)-5-(E)-(2-bromvinyl)-2,4-(1H,3H)-pyrimidindion

2,0 g (4,24 mmol) 5'-O-(4-Chlorbenzoyl)-2'-desoxy-5-(E)-bromvinyluridin werden in 40 g HMPT gelöst und dazu werden 1,6 g (13,44 mmol) Thionylchlorid zugefügt. Nach 45 min ist die Reaktion beendet. Es wird mit Wasser versetzt und mit Essigester extrahiert. Nach Trocknung mit Natriumsulfat, Filtration und Abrotieren des Lösungsmittels wird säulenchromatographisch gereinigt (Cyclohexan/ Essigester 7:3). Man erhält 1,32 g (64 %) eines farblosen Feststoffes vom Schmelzpunkt 89-91 °C.

### 1.1.2.2. 1-(3'-Chlor-2',3'-didesoxy-β-D-threo-pento-furanosyl)-5-(E)-(2-bromvinyl)-2,4-(1H ,3H)-pyrimidindion

0,9 g (1,84 mmol) 1-(5'-O-(4-Chlorbenzoyl)-3'-Chlor-2',3'-didesoxy-β-D-threo-pentofuranosyl)-5-(E)-(2-bromvinyl)-2,4-(1H,3H)-pyrimidindion werden in 50 ml Methanol gelöst und 0,15 g (2,78 mmol) Natriummethylat zugesetzt. Nach 2 h ergibt die DC-Kontrolle (Dichlormethan/Methanol 95:5), dass die Reaktion beendet ist. Man gibt Ionenaustauscher (DOWEX-H⁺ 50 WX 4, (Merck, 105238), aktiviert unter Methanol) hinzu, filtriert und wäscht mit Methanol nach. Nach abrotieren von Methanol erfolgt säulenchromatographische Reinigung (Essigester). Man erhält 0,43 g (67 %) eines farblosen Feststoffes vom Schmelzpunkt 183-185 °C.

¹H-NMR (300 MHz, DMSO-d₆): 2,37 (m, 1H); 3,00 (m, 1H); 3,77 (m, 2H); 4,20 (m, 1H); 4,76 (m, 1H); 5,00 (t, 1H); 6,00 (m, 1H); 6,95 (d, 1H); 7,27 (d, 1H); 7,92 (s, 1H); 11,63 (s, 1H) ppm.

### 1.2. 5'-O-Derivate von 3'-Halogen-BVDU

### 1.2.1. 5'-O-Acylderivate von 3'-Halogen-BVDU

### 1.2.1.1. 5'-O-Pivaloyl-3'-chlor-2',3'-desoxy-5-(E)-bromvinyl-uridin

1,25 g (3,55 mmol) 2',3'-Didesoxy-3'-chlor-5-(E)-bromvinyluridin, 0,05 g (0,41 mmol) 4-Dimethylaminopyridin werden in THF/Pyridin (je 5 ml) gelöst, auf 0 °C gekühlt und mit 0,54 g (4,44 mol) Pivaloylchlorid versetzt. Man lässt auf Raumtemperatur aufwärmen. Nach 2 h ist die Reaktion beendet (DC-Kontrolle mit Dichlormethan/Methanol 20:1). Nach Eingießen in eine Lösung aus Citronensäure in 50 ml Wasser wird mit Essigester extrahiert und die vereinigten Extrakte mit Phosphatpuffer gewaschen und Magnesiumsulfat getrocknet. Nach Filtration und abrotieren des Lösungsmittels erfolgt säulenchromatographische Reinigung (Dichlormethan/Methanol 75:1). Man erhält 1,33 g (86,4 %) eines farblosen Feststoffes vom Schmelzpunkt 136 °C.

¹H-NMR (300 MHz, DMSO-d₆) : 1,14 (s, 9H) ; 2,57 (m, 1H); 2,71 (m, 1H); 4,28 (m, 3H); 4,70 (m, 1H); 6,23 (m, 1H); 6,90 (d, 1H) ; 7,29 (s, 1H); 7,76 (s, 1H); 11, 65 (s, 1H) ppm.

### 1.2.1.2. 5'-O-Ethoxycarbonyl-(E)-5-(2-bromvinyl)-3'-chlor-2',3'-didesoxy-uridin)

310 mg (0,88 mmol) 2',3'-Didesoxy-3'-chlor-5-(E)-bromvinyluridin wird in Dichlormethan/Pyridin (je 3 ml) gelöst und bei 0 °C mit 108 mg (1 mmol) Ethoxyca-bonylchlorid versetzt. Nach 2 h ist die Reaktion beendet (DC-Kontrolle mit Chloroform/Methanol 25:1). Man verdünnt mit 20 ml Dichlormethan, wäscht mit 1 M Salzsäure und mit Phosphatpuffer. Nach Trocknung über Magnesiumsulfat, Filtration und Abrotieren des Lösungsmittels erfolgt säulenchromatographische Reinigung mit Chloroform/Methanol 25:1. Man erhält 200 mg (53,6 %) eines farblosen Feststoffes vom Schmelzpunkt 181 °C.

¹H-NMR (500MHz, DMSO-d₆): 1,23 (t, 3H); 2,65 (m, 2H); 4,15 (q, 2H); 4,37 (m, 3H); 4,71 (m, 1H); 6,22 (m, 1H); 6,87 (d, 1H); 7,29 (d, 1H); 7,78 (s, 1H); 11,64 (s, 1H) ppm.

### 1.2.2. Aminosäureester von 3'-Halogen-BVDU

### 1.2.2.1. 5'-O-(t-Butoxycarbonylaminoacetyl)-3'-chlor-2',3'-didesoxy-5-(E)-bromvinyluridin

1,06 g (3 mmol) 2',3'-Didesoxy-3'-chlor-5-(E)-bromvinyluridin, 0,58 g (3,3 mmol) N-BOC-Glycin, 0,45 g (3,3 mmol) 1-Hydroxybenzotriazol und 0,99 g (3,3 mmol) 1-(3-Dimethylaminopropyl)-3-ethylcarbodiimid Methiodid werden in 25 ml Dichlormethan gelöst. Nach 8 h wird noch mal jeweils die gleiche Menge N-BOC-Glycin, 1-Hydroxybenzotriazol und 1-(3-Dimethylaminopropyl)-3-ethylcarbodiimid Methiodid zugegeben. Nach weiteren 16 h ist die Reaktion beendet (DC-Kontrolle mit Dichlormethan/Methanol 25:1). Die Dichlormethanlösung wird mit Wasser gewaschen und mit Natriumsulfat getrocknet. Nach Filtration und Abrotieren des Lösungsmittels erfolgt säulenchromatographische Reinigung (Dichlormethan/Methanol 25:1). Man erhält 1,03 g (68 %) eines farblose n Schaums.

¹H-NMR (300MHz, DMSO-d₆): 1,37 (s, 9H); 2,57 (m, 1H); 2,71 (m, 1H); 3,72 (m, 2H); 4,23 (m, 1H); 4,33 (m, 2H); 4,69 m, 1H); 6,24 (m, 1H); 6,93 (d, 1H); 7,26 (t, 1H); 7,31 (d, 1H); 7,77 (s, 1H); 11,66 (s, 1H) ppm.

### 1.2.2.2. 5'-O-(Ammoniumacetyl-3'-chlor-2',3'-dides-oxy-5-(E)-bromvinyluridin-trifluoracetat

0,81 g (1,6 mmol) 5'-O-(t-Butoxycarbonylaminoacetyl)-3'-chlor-2',3'-didesoxy-5-(E)-bromvinyluridin werden in 20 ml Trifluoressigsäure gelöst. Nach 45 min ist die Abspaltung der BOC-Schutzgruppe vollständig (DC-Kontrolle mit Dichlormethan/ Methanol 95:5). Trifluoressigsäure wird abrotiert, der Rückstand in wenig Methanol aufgenommen und mit Diethylether behandelt. Der ausgefallene Niederschlag wird abgesaugt, mit Diethylether gewaschen und erneut aus Methanol/Diethylether umgefällt. Nach Abfiltrieren, waschen mit Diethylether und Trocknen beträgt die Ausbeute 0,38 g (45,7 %) eines farblosen Pulvers.

¹H-NMR (300MHz, DMSO-d₆): 2,59 (m, 1H); 2,75 (m, 1H); 3,87 (m, 2H); 4,26 (m, 1H); 4,44 (m, 2H); 4,73 (m, 1H); 6,24 (m, 1H); 6,93 (d, 1H); 7,32 (d, 1H); 7,80 (s, 1H); 8,32 (s, 3H); 11,68 (s, 1H) ppm.

### 1.2.2.3. 4-(t-Butoxycarbonyl)amino-1-(3'-chlor-2',3'-didesoxy-5-(E)-bromvinyluridin-5'-yl)butanoat

2,0 g (9,84 mmol) N-BOC-4-Aminobuttersäure und 1,2 g (9,84 mmol) 4-Dimethylaminopyridin werden in 20 ml Dichlormethan gelöst. Dazu gibt man 3,46 g (9,84 mmol) 2',3'-Didesoxy-3'-chlor-5-(E)-bromvinyluridin und nach Kühlung auf 0°C 2,03 g (9,84 mmol) N,N'-Dicyclohexylcarbodiimid. Nach Aufwärmen auf Raumtemperatur und nach 20 h Rühren bei Raumtemperatur ist die Reaktion beendet (DC-Kontrolle mit Dichlormethan/Methanol 20:1). Der Ansatz wird filtriert und einrotiert. Nach Lösen in Essigester wird auf -20 °C gekühlt, der Niederschlag abfiltriert und mit wenig auf -20 °C gekühltem Essigester gewaschen. Das Filtrat wird einrotiert und mit Dichlormethan/Essigester (4:1) mehrfach säulenchromatographisch gereinigt. Man erhält 3,2 g (60,6 %) eines farblosen Schaums.

¹H-NMR (500 MHz, DMSO-d₆) : 1,36 (s, 9H) ; 1,62 (m, 2H) ; 2,31 (m, 2H); 2,52 (m, 1H) ; 2,72 (m, 1H) ; 2,92 (m, 2H); 4,31 (m, 3H); 4,71 (m, 1H); 6,22 (m, 1H); 6,79 (m, 1H); 6,92 (d, 1H); 7,31 (m, 1H); 7,77 (s, 1H); 11,64 (s, 1H).

Fig. 2 zeigt die Ergebnisse dieser erfindungsgemäßen Verbindung in Kombination mit Mitomycin C (MMC) im Vergleich zu MMC alleine und MMC in Kombination mit BVDU.

### 1.2.2.4. 4-Amonium-1-(3'-chlor-2',3'-didesoxy-5-(E)-bromvinyluridin-5'-yl)butanoat-trifluor-acetat

310 mg (0,577 mmol) 4-(t-Butoxycarbonyl)amino-1-(3'-chlor-2',3'-didesoxy-5-(E)-bromvinyluridin-5'-yl)butanoat werden in 5 ml Dichlormethan gelöst und mit 5 ml Trifluoresigsäure versetzt. Nach 30 min Rühren bei Raumtemperatur ist der Umsatz vollständig (DC-Kontrolle Dichlormethan/Methanol 20:1). Dichlormethan und Trifluoressigsäure werden abdestilliert und der Rückstand mit 20 ml Diethylether versetzt. Man lässt 2 h rühren, bis ein feiner, pulvriger Niederschlag entsteht, filtriert, wäscht mit Diethylether und trocknet im Vakuum. Man erhält 250 mg (78,7 %) eines farblosen Feststoffes vom Schmelzpunkt 89 °C.

¹H-NMR (500 MHz, DMSO-d₆): 1,82 (m, 2H); 2,49 (m, 2H); 2,59 (m, 1H) ; 2,72 (m, 1H) ; 2, 81 (m, 2H) ; 4, 22 (m, 1H); 4,32 (m, 2H); 4,69 m, 1H); 6,22 (m, 1H); 6,94 (d, 1H); 7,29 (d, 1H); 7,73 (s, 3H); 7,78 (s, 1H) ; 11,65 (s, 1H).

### 1.2.2.5. 5'-O-(N-t-Butyloxycarbonyl-L-valinoyl)-3'-chlor-2',3'-didesoxy-5-(E)-bromvinyluridin

Bei 0°C werden 1,00 g (2,84 mmol) 3'-Chlor-2',3'-didesoxy-5-(E)-bromvinyluridin in 20 ml Dichlormethan vorgelegt. Dann werden 618 mg (2,84 mmol, 1,0 Äq.) N-t-Butyloxycarbonyl-L-valin, 347 mg (2,84 mmol, 1,0 Äq.) N,N-Dimethylaminopyridin sowie 587 mg (2,84 mmol, 1,0 Äq.) N,N'-Dicyclohexylcarbodiimid zugegeben und anschließend wird das Reaktionsgemisch 20 h bei Raumtemperatur gerührt. Der entstandene Niederschlag wird abfiltriert und mit Dichlormethan gewaschen. Das Filtrat wird mit verdünnter Zitronensäurelösung, NaH-CO₃-Lösung sowie NaCl-Lösung gewaschen und anschließend mit Na₂SO₄ getrocknet. Das Solvens wird am Rotationsverdampfer entfernt. Säulenchromatographische Reinigung (CHCl₃/MeOH, 95/5) liefert 1,09 g (1,98 mmol, 70 %) 5'-O-(N-t-Butyloxycarbonyl-L-valinoyl)-3'-chlor-2',3'-didesoxy-5-(E)-bromvinyluridin als weißen Feststoff mit einem Schmelzpunkt von 75-76°C.

¹H-NMR (500 MHz, DMSO-d₆): 0,85 (d, 3H); 0,87 (d, 3H); 1,38 (s, 9H); 1,98 (m, 1H); 2,54 (m, 1H); 2,73 (m, 1H); 3,81 (t, 1H); 4,26 (m, 2H); 4,34 (m, 1H); 4,68 (q, 1H); 6,24 (t, 1H); 6,91 (d, 1H); 7, 21 (d, 1H); 7,31 (d, 1H); 7,76 (s, 1H); 11,67 (s, 1H) ppm.

### 1.2.2.6. (3'-Chlor-2',3'-didesoxy-5'-O-L-valinoyl-5-(E)-bromvinyluridin) trifluoracetat

Bei 0°C werden 600 mg (1,09 mmol) 5'-O-(N-t-Butyloxy-carbonyl-L-valinoyl)-3'-chlor-2',3'-didesoxy-5-(E)-bromvinyluridin in 5 ml Dichlormethan vorgelegt, 1,0 ml (14 mmol) Trifluoressigsäure werden zugegeben und anschließend wird das Reaktionsgemisch 3 h bei Raumtemperatur gerührt. Das Solvens wird am Rotationsverdampfer entfernt. Das Rohprodukt wird mit 5 ml Diethylether versetzt und 20 h bei Raumtemperatur gerührt. Das Solvens wird abdekantiert und der Rückstand am Rotationsverdampfer für 1 h bei 40°C belassen. Es resultieren 540 mg (956 µmol, 88 %) (3'-Chlor-2',3'-didesoxy-5'-O-L-valinoyl-5-(E)-bromvinyl-uridin) trifluoracetat als ein weißer Feststoff mit einem Schmelzpunkt von 110-112°C.

¹H-NMR (500 MHz, DMSO-d₆): 0,95 (d, 3H); 0,97 (d, 3H); 2,17 (m, 1H); 2,60 (m, 1H); 2,79 (m, 1H); 3,93 (bs, 1H); 4,27 (m, 1H); 4,46 (m, 2H); 4,74 (q, 1H); 6,26 (dd, 1H); 6,91 (d, 1H); 7,32 (d, 1H); 7,80 (s, 1H); 8,34 (bs, 3H); 11,69 (s, 1H) ppm.

### 1.2.2.7. 5'-O-(N-t-Butyloxycarbonyl-L-valyl-L-valinoyl)-3'-chlor-2',3'-didesoxy-5-(E)-brom-vinyluridin

Bei 0°C werden 1,00 g (2,84 mmol) 3'-Chlor-2',3'-didesoxy-5-(E)-bromvinyluridin in 20 ml Dichlormethan vorgelegt. Dann werden 880 mg (2,78 mmol, 1,0 Äq.) N-t-Butyloxycarbonyl-L-valyl-L-valin, 347 mg (2,84 mmol, 1,0 Äq.) N,N-Dimethylamino-pyridin sowie 587 mg (2,84 mmol, 1,0 Äq.) N,N'-Dicyclohexylcarbodiimid zugegeben und anschließend wird das Reaktionsgemisch 3 d bei Raumtemperatur gerührt. Der entstandene Niederschlag wird abfiltriert und mit Dichlormethan gewaschen. Das Filtrat wird mit verdünnter Zitronensäurelösung, NaHCO₃-Lösung sowie NaCl-Lösung gewaschen und anschließend mit Na₂SO₄ getrocknet. Das Solvens wird am Rotationsverdampfer entfernt. Säulenchromatographische Reinigung (Dichlormethan/Essigester, 3/1) liefert 610 mg (939 µmol, 33 %) 5'-O-(N-t-Butyloxycarbonyl-L-valyl-L-valinoyl)-3'-chlor-2',3'-didesoxy-5-(E)-bromvinyluridin als weißen Feststoff mit einem Schmelzpunkt von 102-103°C.

¹H-NMR (500 MHz, DMSO-d₆): 0,81-0,90 (m, 12H); 1,37 (s, 9H); 1,91 (m, 1H); 2,06 (m, 1H); 2,54 (m, 1H); 2,74 (m, 1H); 3,88 (t, 1H); 4,19 (t, 1H); 4,23-4,35 (m, 3H); 4,68 (q, 1H); 6,24 (t, 1H); 6,64 (d, 1H); 6,91 (d, 1H); 7,31 (d, 1H); 7,77 (s, 1H); 8,03 (d, 1H); 11,67 (s, 1H) ppm.

### 1.2.2.8. [3'-Chlor-2',3'-didesoxy-5'-O-(L-valyl-L-valinoyl)-5-(E)-bromvinyluridin] trifluoracetat

Bei 0°C werden 300 mg (462 µmol) 5'-O-(N-t-Butyloxy-carbonyl-L-valyl-L-valinoyl)-3'-chlor-2',3'-didesoxy-5-(E)-bromvinyluridin in 5 ml Dichlormethan vorgelegt, 0,5 ml (6,7 mmol) Trifluoressigsäure werden zugegeben und anschließend wird das Reaktionsgemisch 3 h bei Raumtemperatur gerührt. Das Solvens wird am Ro-tationsverdampfer entfernt. Das Rohprodukt wird mit 5 ml Diethylether versetzt und 1 h bei Raumtemperatur gerührt. Das Solvens wird abdekantiert und der Rückstand am Rotationsverdampfer für 1 h bei 40°C belassen. Es resultieren 280 mg (422 µmol, 91 %) [3'-Chlor-2',3'-didesoxy-5'-O-(L-valyl-L-valinoyl)-5-(E)-bromvinyluridin] trifluoracetat als ein weißer Feststoff mit einem Schmelzpunkt von 169-170°C.

¹H-NMR (500 MHz, DMSO-d₆): 0,91-0,96 (m, 12H); 2,08-2,14 (m, 2H); 2,59 (m, 1H); 2,76 (m, 1H); 3,71 (t, 1H); 4,22-4,26 (m, 2H); 4,31-4,38 (m, 2H); 4,68 (q, 1H); 6,25 (t, 1H); 6,90 (d, 1H); 7,32 (d, 1H); 7,81 (s, 1H); 8,06 (bs, 3H); 8,56 (d, 1H); 11,68 (s, 1H) ppm.

### 1.2.2.9. 3'-Azido-5'-O-(N-t-butyloxycarbonyl-L-valyl-L-valinoyl)-2-,3'-didesoxy-5-(E)-bromvinyluridin

Bei 0°C werden 1,02 g (2,84 mmol) 3'-Azido-2',3'-didesoxy-5-(E)-bromvinyluridin in 20 ml Dichlormethan vorgelegt. Dann werden 880 mg (2,78 mmol, 1,0 Äq.) N-t-Butyloxycarbonyl-L-valyl-L-valin, 347 mg (2,84 mmol, 1,0 Äq.) N,N-Dimethylamino-pyridin sowie 587 mg (2,84 mmol, 1,0 Äq.) N,N'-Dicyclohexylcarbodiimid zugegeben und anschließend wird das Reaktionsgemisch 24 h bei Raumtemperatur gerührt. Der entstandene Niederschlag wird abfiltriert und mit Dichlormethan gewaschen. Das Filtrat wird mit verdünnter Zitronensäurelösung, NaHCO₃-Lösung sowie NaCl-Lösung gewaschen und anschließend mit Na₂SO₄ getrocknet. Das Solvens wird am Rotationsverdampfer entfernt. Säulenchromatographische Reinigung (Dichlormethan /Essigester, 3/1) liefert 550 mg (838 µmol, 30 %) 3'-Azido-5'-O-(N-t-butyloxy-carbonyl-L-valyl-L-valinoyl)-2',3'-didesoxy-5-(E)-bromvinyluridin als weißen Feststoff mit einem Schmelzpunkt von 95-97°C.

¹H-NMR (500 MHz, DMSO-d₆): 0,81-0,91 (m, 12H); 1,37 (s, 9H); 1,91 (m, 1H); 2,07 (m, 1H); 2,38 (m, 1H); 2,51 (m, 1H); 3,88 (t, 1H); 4,01 (q, 1H); 4,19 (t, 1H); 4,30 (m, 2H); 4,45 (q, 1H); 6,12 (t, 1H); 6,64 (d, 1H); 6,92 (d, 1H); 7,31 (d, 1H); 7,77 (s, 1H); 8,04 (d, 1H); 11,66 (s, 1H) ppm.

### 1.2.2.10. [3'-Azido-2',3'-didesoxy-5'-O-(L-valyl-L-valinoyl)-5-(E)-bromvinyluridin] trifluoracetat

Bei 0°C werden 200 mg (305 µmol) 3'-Azido-5'-O-(N-t-butyloxy-carbonyl-L-valyl-L-valinoyl)-2',3'-didesoxy-5-(E)-bromvinyluridin in 4 ml Dichlormethan vorgelegt, 0,33 ml (4,47 mmol) Trifluoressigsäure werden zugegeben und anschließend wird das Reaktionsgemisch 3 h bei Raumtemperatur gerührt. Das Solvens wird am Rotationsverdampfer entfernt. Das Rohprodukt wird mit 5 ml Diethylether versetzt und 1 h bei Raumtemperatur gerührt. Das Solvens wird abdekantiert und der Rückstand am Rotationsverdampfer für 1 h bei 40°C belassen. Es resultieren 200 mg (298 µmol, 98 %) [3'-Azido-2',3'-didesoxy-5'-O-(L-valyl-L-valinoyl)-5-(E)-bromvinyluridin] trifluoracetat als ein weißer Fest-stoff mit einem Schmelzpunkt von 113-115°C.

¹H-NMR (500 MHz, DMSO-d₆): 0,91-0,96 (m, 12H); 2,08-2,14 (m, 2H); 2,40 (m, 1H); 2,53 (m, 1H); 3,72 (t, 1H); 4,00 (q, 1H); 4,25 (m, 1H); 4,31-4,38 (m, 2H); 4,45 (q, 1H); 6,13 (t, 1H); 6,90 (d, 1H); 7,31 (d, 1H); 7,80 (s, 1H); 8,08 (m, 3H); 8,58 (d, 1H); 11,67 (s, 1H) ppm.

### 1.2.3. Phosphoramidate von 3'-Halogen-BVDU

### 1.2.3.1. (E)-5-(2-Bromvinyl)-3'-fluor-2',3'-didesoxy-uridin-5'-[phenyl-(methoxy-L-alaninyl)]-phosphat

255 mg (1,20 mmol) Phenyldichlorophosphat und 169 mg (1,20 mmol) L-Alaninmethylester Hydrochlorid werden in 4 ml THF gelöst bzw. suspendiert. Mit einem Trockeneisbad wird auf -78 °C gekühlt und bei dieser Temperatur werden 244 mg (2,40 mmol) Triethylamin zugetropft, das in 4 ml THF gelöst ist. Nach 30 min ist das Zutropfen beendet und man lässt nach und nach auf Raumtemperatur aufwärmen und insgesamt 24 h rühren. Danach gibt man 270 mg (0,80 mmol) 2',3'-Didesoxy-3'-fluor-5-(E)-bromvinyluridin hinzu und kühlt mit einem Trockeneisbad auf -78 °C. Zu der erhaltenen Suspension tropft man eine Lösung von 265 mg (3,22 mmol) N-Methylimidazol in 5 ml THF hinzu. Nach 30 min ist das Zutropfen beendet und man lässt allmählich auf Raumtemperatur aufwärmen. Nach weiteren 48 h ist die Reaktion beendet (DC-Kontrolle mit Chloroform/Essigester 3:2). Der Ansatz wird in ein Gemisch aus 20 ml Phosphatpuffer und 25 ml Essigester gegeben und die wässrige Phase noch zweimal mit je 20 ml Essigester extrahiert. Nach Trocknen mit Magnesiumsulfat, Abfiltrieren und Abrotieren des Lösungsmittels erfolgt säulenchromatographische Reinigung mit Dichlormethan/Essigester 3:2. Man erhält 180 mg (38,8 %) eines farblosen Schaums.

¹H-NMR (500 MHz, DMSO-d₆): 1,23 (d, 3H); 2,35 (m, 1H); 2,49 (m, 1H); 3,58 (s, 3H); 3,85 (m, 1H); 4,21 (m, 2H); 4,39 (m, 1H); 5,32 (m, 1H); 6,18 (m, 2H); 6,84 (d, 1H); 7,19 (m, 3H); 7,29 (d, 1H); 7,36 (m, 2H); 7,87 (s, 1H); 11,67 (s, 1H) ppm.

### ³¹P-NMR (122 MHz, DMSO-d₆): 5,08; 5,29 ppm.

### 1.2.3.2. (E)-5-(2-Bromvinyl)-3'-chlor-2',3'-didesoxy-uridin-5'-[phenyl-(methoxy-L-alaninyl)]-phosphat

1,19 g (5,67 mmol) Phenyldichlorophosphat und 0,79 g (5,67 mmol) L-Alaninmethylester Hydrochlorid werden in 12 ml THF gelöst bzw. suspendiert. Mit einem Trockeneisbad wird auf -78 °C gekühlt und bei dieser Temperatur werden 1,15 g (11,34 mmol) Triethylamin zugetropft, das in 12 ml THF gelöst ist. Nach 30 min ist das Zutropfen beendet und man lässt nach und nach auf Raumtemperatur aufwärmen und insgesamt 24 h rühren.

Danach gibt man 1,0 g (2,84 mmol) 2',3'-Didesoxy-3'-chlor-5-(E)-bromvinyluridin hinzu und kühlt mit einem Trockeneisbad auf -78 °C. Zu der erhaltenen Suspension tropft man eine Lösung von 1,17 g (14,2 mmol) N-Methylimidazol in 12 ml THF hinzu. Nach 30 min ist das Zutropfen beendet und man lässt allmählich auf Raumtemperatur aufwärmen. Nach weiteren 48 h ist die Reaktion beendet (DC-Kontrolle mit Dichlormethan/Essigester 3:2). Der Ansatz wird in ein Gemisch aus 75 ml Phosphatpuffer und 50 ml Essigester gegeben und die wässrige Phase noch zweimal mit je 40 ml Essigester extrahiert. Nach Trocknen mit Magnesiumsulfat, Abfiltrieren und Abrotieren des Lösungsmittels erfolgt säulenchromatographische Reinigung mit Dichlormethan/Essigester 3 : 2 und Chloroform/Aceton 3 : 1. Man erhält 950 mg (56,5 %) eines farblosen Schaums.

¹H-NMR (500MHz, CDCl₃): 1,37 (m, 3H); 2,38 (m, 1H); 2,62 (m, 1H); 3,71, 3,72 (s, 3H); 3,75, 3,85 (m, 1H); 4,09 (m, 1H); 4,41 (m, 4H); 6,24, 6,32 (m, 1H); 6,71 (m, 1H); 7,41 (m, 6H); 7,66, 7,70 (s, 1H); 8,88, 8,92 (s, 1H) ppm.

³¹P-NMR (122MHz, CDCl₃): 2,87; 2,74 ppm.

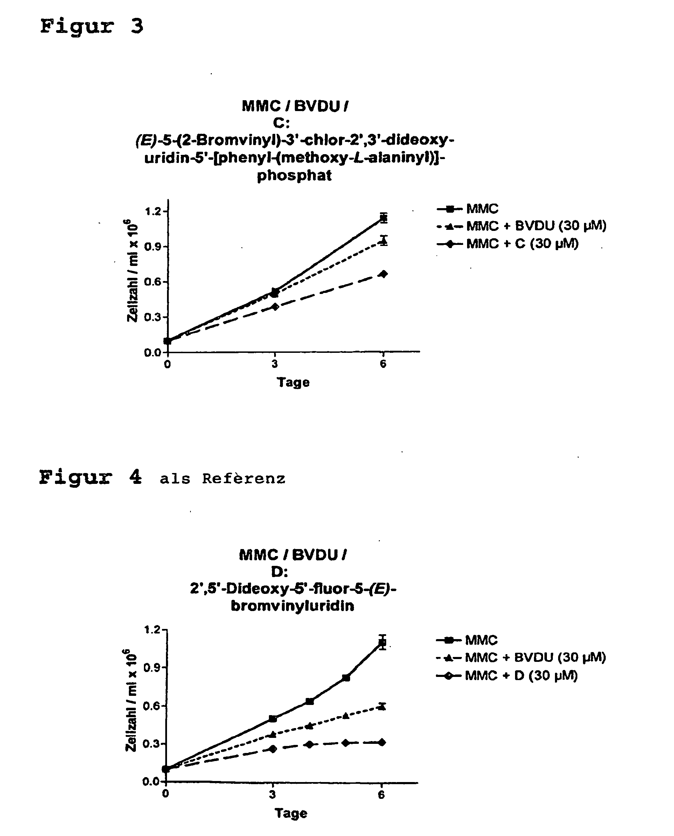

Fig. 3 zeigt die Ergebnisse dieser erfindungsgemäßen Verbindung in Kombination mit Mitomycin C (MMC) im Vergleich zu MMC alleine und MMC in Kombination mit BVDU.

### 1.2.3.3. (E)-5-(2-Bromvinyl)-3'-chlor-2',3'-didesoxy-uridin-5'-[phenyl-(benzyloxy-L-alaninyl)]-phosphat

606 mg (2,87 mmol) Phenyldichlorophosphat und 1010 mg (2,87 mmol) L-Alaninbenzylester 4-Methylbenzolsulfonat werden in 15 ml THF gelöst. Mit einem Trockeneisbad wird auf -78 °C gekühlt und bei dieser Temperatur werden 582 mg (5,75 mmol) Triethylamin zugetropft, das in 5 ml THF gelöst ist. Nach 35 min ist das Zutropfen beendet und man lässt nach und nach auf Raumtemperatur aufwärmen und insgesamt 24 h rühren. Danach gibt man 505 mg (1,44 mmol) 2',3'-Didesoxy-3'-chlor-5-(E)-bromvinyluridin hinzu und kühlt mit einem Trockeneisbad auf -78 °C. Zu der erhaltenen Suspension tropft man eine Lösung von 650 mg (8,0 mmol) N-Methylimidazol in 5 ml THF hinzu. Nach 30 min ist das Zutropfen beendet und man lässt allmählich auf Raumtemperatur aufwärmen. Nach weiteren 48 h ist die Reaktion beendet (DC-Kontrolle mit Chloroform/Methanol 25:1). Der Ansatz wird in ein Gemisch aus 50 ml Phosphatpuffer und 50 ml Essigester gegeben und die wässrige Phase noch zweimal mit je 25 ml Essigester extrahiert. Nach Trocknen mit Magnesiumsulfat, Abfiltrieren und Abrotieren des Lösungsmittels erfolgt säulenchromatographische Reinigung mit Chloroform/Essigester 3:2. Man erhält 520 mg (53,0 %) eines farblosen Schaums.

¹H-NMR (500MHz, DMSO-d₆): 1,25 (2 X d, 3H) ; 2,58 (m, 2H); 3,92 (m, 1H); 4,26 (m, 3H); 4,67 (m, 1H); 5,08 (m, 2H); 6,25 (m, 2H); 6,88 (2 X d, 1H); 7,18 (m, 3H); 7,36 (m, 8H); 7,81 (2 X s, 1H); 11,87 (2 X d, 1H) ppm.

³¹P-NMR (122MHz, DMSO-d₆): 4,40; 4,57 ppm.

### 1.2.3.4. (E)-5-(2-Bromvinyl)-3'-brom-2',3'-didesoxyuridin-5'-[phenyl-(methoxy-L-alaninyl)]-phosphat

401 mg (1,9 mmol) Phenyldichlorophosphat und 265 mg (1,9 mmol) L-Alaninmethylester Hydrochlorid werden in 6 ml THF gelöst bzw. suspendiert. Mit einem Trockeneisbad wird auf -78 °C gekühlt und bei dieser Temperatur werden 385 mg (3,8 mmol) Triethylamin zugetropft, das in 6 ml THF gelöst ist. Nach 30 min ist das Zutropfen beendet und man lässt nach und nach auf Raumtemperatur aufwärmen und insgesamt 24 h rühren. Danach gibt man 500 mg (1,26 mmol) 2',3'-Didesoxy-3'-brom-5-(E)-bromvinyluridin hinzu und kühlt mit einem Trockeneisbad auf -78 °C. Zu der erhaltenen Suspension tropft man eine Lösung von 415 mg (5,05 mmol) N-Methylimidazol in 6 ml THF hinzu. Nach 30 min ist das Zutropfen beendet und man lässt allmählich auf Raumtemperatur aufwärmen. Nach weiteren 48 h ist die Reaktion beendet (DC-Kontrolle mit Chloroform/Essigester 1:1). Der Ansatz wird in ein Gemisch aus 25 ml Phosphatpuffer und 25 ml Essigester gegeben und die wässrige Phase noch zweimal mit je 20 ml Essigester extrahiert. Nach Trocknen mit Magnesiumsulfat, Abfiltrieren und Abrotieren des Lösungsmittels erfolgt säulenchromatographische Reinigung (Dichlormethan/Essigester 3:2). Man erhält 386 mg (48,1 %) eines farblosen Schaums.

¹H-NMR (500MHz, DMSO-d₆): 1,25 (d, 3H); 2,71 (m, 2H); 3,61 (s, 3H); 3,85 (m, 1H); 4,18 (m, 1H); 4,27 (m, 1H); 4,42 (m, 1H); 4,66 (m, 1H); 6,11 (m, 1H); 6,28 (m, 1H); 6,88 (d, 1H); 7,19 (m, 3H); 7,31 (m, 1H); 7,38 (m, 2H); 7,81 (s, 1H); 11,65 (s, 1H) ppm.

³¹P-NMR (122 MHz, DMSO-d₆): 5.04; 5,11 ppm.

### 1.2.3.5. 3'-Azido-5-(E)-bromvinyl-2',3'-didesoxy-uridin]-5'-yl-(methoxy-L-alaninyl]-phenylphosphat

600 mg (2,84 mmol, 2 Äq.) Phenyldichlorophosphat und 397 mg (2,84 mmol, 2 Äq.) L-Alaninmethylester Hydrochlorid werden in 7 ml THF bei -78°C vorgelegt. 576 mg (5,69 mmol, 4 Äq.) Triethylamin werden in 7 ml THF gelöst, innerhalb von 30 min zugetropft und anschließend 20 h bei Raumtemperatur gerührt. Das Reaktionsgemisch wird auf -78°C gekühlt und es werden 509 mg (1,42 mmol) 3'-Azido-2',3'-didesoxy-5-(E)-bromvinyluridin zugegeben. Zu der erhaltenen Suspension wird eine Lösung von 700 mg (8,53 mmol, 6 Äq.) N-Methylimidazol in 7 ml THF innerhalb von 30 min zugetropft und anschließend 20 h bei Raumtemperatur gerührt. Der Ansatz wird in ein Gemisch aus 25 ml Phosphatpuffer und 25 ml Essigester gegeben und die wässrige Phase noch zweimal mit je 20 ml Essigester extrahiert. Die vereinte organische Phase wird über Na₂SO₄ getrocknet, filtriert und das Solvens am Rotationsverdampfer entfernt. Säulenchromatographische Reinigung (Dichlormethan /Essigester, 1/1; Essigester) liefert 250 mg (417 µmol, 29 %) [3'-Azido-5-(E)-bromvinyl-2',3'-didesoxy-uridin]-5'-yl-(methoxy-L-alaninyl]-phenylphosphat als weißen Feststoff.

¹H-NMR (500MHz, DMSO-d₆): 1,20 (d, 3H); 1,23* (d, 3H); 2,38-2,47 (m, 4H); 3,57 (s, 3H); 3,59* (s, 3H); 3,80-3,91 (m, 2H); 4,03 (q, 1H); 4,08 (q, 1H); 4,16-4,21 (m, 1H); 4,23-4,29 (m, 3H); 4,45-4,52 (m, 2H); 6,09-6,16 (m, 4H); 6,86-6,89 (m, 2H); 7,16-7,22 (m, 6H); 7,28-7,32 (m, 2H); 7,34-7,38 (m, 4H); 7,82 (s, 2H); 11,65 (s, 1H); 11,66* (s, 1H) ppm. Die Substanz besteht aus einem Diastereomerengemisch (Verhältnis ca. 1,2:1). Die mit * gekennzeichneten Signale beziehen sich auf das Unterschuss-isomer.

³¹P-NMR (122 MHz, DMSO-d₆): 5,06; 5,20 ppm.

### 1.2.4. Phosphorsäurederivate von 3'-Halogen-BVDU

### 1.2.4.1. 3'-Chlor-2',3'-didesoxy-5-(E)-(2-bromvinyl-uridinyl)-5'-yl-diethylphosphat

500 mg (1,42 mmol) 2',3'-Didesoxy-3'-chlor-5-(E)-bromvinyluridin werden in 10 ml THF und 1 ml Pyridin gelöst. Man kühlt im Eisbad auf 0°C und tropft eine Lösung von 735 mg (4,3 mmol) Diethylchlorphosphat innerhalb 5 min zu. Nach 18 h werden erneut 1 ml Pyridin und 735 mg Diethylchlorphosphat zugesetzt und nach weiteren 18 h ist der Umsatz vollständig (DC-Kontrolle mit Chloroform/Methanol 10:1). Der Ansatz wird einem Gemisch aus 20 ml Phosphatpuffer und 25 ml Essigester zugefügt und die wässrige Phase noch dreimal mit 15 ml Essigester extrahiert. Die vereinigten Extrakte werden mit Magnesiumsulfat getrocknet. Nach Filtration und Abrotieren des Lösungsmittels erfolgt Reinigung durch wiederholte Säulenchromatographie (Chloroform/Methanol 15:1). Das erhaltene Öl erstarrt nach Anreiben mit Cyclohexan. Nach Trocknung erhält man 510 mg (73,6 %) eines farblosen Schaums.

¹H-NMR (300MHz, CDCl₃): 1,38 (m, 6H); 2,55 (m, 1H); 2,67 (m, 1H); 4,21 (m, 5H); 4,32 (m, 2H); 4,49 (m, 1H); 6,35 (t, 1H); 6,78 (d, 1H); 7,44 (d, 1H); 7,74 (s, 1H); 8,91 (s, 1H) ppm.

³¹P-NMR (122 MHz, CDCl₃): 0,75 ppm.

### 1.2.4.2. cycloSaligenyl-5'-O-(E)-(2-bromvinyl)-3'-chlor-2',3'-didesoxy-uridinyl)-phosphat

265 mg (2,13 mmol) 2-Hydroxybenzylalkohol werden in 5 ml THF gelöst und auf -78 °C gekühlt (Aceton/Trockeneis). Danach gibt man 327 mg Phosphoroxychlorid hinzu und schließlich 5 ml einer Lösung von 431 mg Triethylamin in THF. Nach 20 min ist die Zugabe beendet. Man läst noch 45 min bei -78 °C rühren, dann wird das Kältebad entfernt und man lässt auf Raumtemperatur aufwärmen. Es entsteht eine farblose Suspension, die 2,5 h bei Raumtemperatur gerührt wird.

Danach wird erneut auf -78 °C gekühlt und 466 mg (5,68 mmol) N-Methylimidazol, in 2,5 ml THF gelöst, zugegeben. 500 mg (1,42 mmol) 2',3'-Didesoxy-3'-chlor-5-(E)-bromvinyluridin werden in 10 ml THF gelöst und innerhalb 30 min zugetropft. Man lässt allmählich auf Raumtemperatur aufwärmen und rührt noch 18 h bei Raumtemperatur. Die Reaktion ist danach beendet (DC-Kontrolle mit Chloroform/Methanol 20 : 1). Der Ansatz wird einer Mischung aus 20 ml Phosphatpuffer und 20 ml MTBE zugesetzt und mehrfach mit MTBE extrahiert und die vereinigten Extrakte mit Magnesiumsulfat getrocknet. Nach Filtration und Abrotieren des Lösungsmittels erhält man nach säulenchromatographischer Reinigung (Chloroform/Methanol 3 : 2) 210 mg (28,5 %) eines farblosen Schaums.

¹H-NMR (300MHz, CDCl₃) : 2,50 (m, 1H); 2,67 (m, 1H); 4,34 (m, 1H); 4,48 (m, 3H); 5,45 (m, 2H); 6,27 (m, 1H); 6,62 (m, 1H); 7,15 (m, 3H); 7,42 (m, 2H); 7,58 (m, 1H); 8,23 (s, 1H) ppm.

³¹P-NMR(122MHz, CDCl₃): -7,84; -7,99 ppm.

### 1.2.4.3. Phenyl-S-pivaloyl-2-thioethyl-3'-brom-2',3'-didesoxy-5-(E)-bromvinyl-uridin-5'-yl-phosphat

1,50 g (9,24 mmol) S-(2-Hydroxyethyl)-thiopivaloat werden in 80 ml THF gelöst, auf -78 °C gekühlt und mit 0,95 g (9,44 mmol) Triethylamin versetzt. 1,99 g (9,44 mmol) Phenyldichlorophosphat werden in 5 ml THF gelöst und bei -78 °C zugetropft. Man lässt 20 h rühren und dabei allmählich auf Raumtemperatur aufwärmen. Danach wird filtriert, mit THF (2 x 10 ml) gewaschen und das Lösungsmittel abdestilliert. Das gelbliche Öl wird in 50 ml Tetrachlormethan aufgenommen, erneut filtriert und der Rückstand mit Tetrachlormethan gewaschen. Nach Abziehen des Lösungsmittels wird der ölige Rückstand im Vakuum getrocknet. Man erhält 2,55 g (Phenyl-S-(2-Hydroxyethyl)-thiopivaloat)-monochlorphosphat als Rohprodukt, das ohne weitere Reinigung eingesetzt wird.

Das zuvor erhaltene Rohprodukt (2,55.g) wird in 15 ml THF gelöst und danach werden 1,0 g (2,52 mmol) 2',3'-Didesoxy-3'-brom-5-(E)-bromvinyluridin zugesetzt. Nach 5 min rühren bei Raumtemperatur gibt man 1,24 g (15,15 mmol) N-Methylimidazol hinzu und lässt bei Raumtemperatur 3 h rühren. Die Reaktion ist danach beendet (DC-Kontrolle mit Chloroform/Methanol 20 : 1; Dichlorme-than/Aceton 10 : 1). Das Reaktionsgemisch wird in ein Zweiphasengemisch aus 100 ml Phosphatpuffer und 60 ml Essigester eingegossen und die wässrige Phase noch zweimal mit je 50 ml Essigester extrahiert. Die vereinigten Essigesterphasen werden mit 5 % Citronensäure gewaschen, dann mit 5 % Natriumhydrogencarbonatlösung und schließlich mit gesättigter Kochsalzlösung. Nach Trocknung mit Magnesiumsulfat, abfiltrieren und abdestillieren des Lösungsmittels erfolgt säulenchromatographische mit Chloroform/Aceton 10 : 1. Man erhält 920 mg (52,6 %) eines hellgelblichen Schaums.

¹H-NMR (500 MHz, CDCl₃): 8,97 (s, 1H); 7,63 und 7,68 (s, 1H); 7,39 (m, 3H); 7,22 (m, 3H); 6,66 (d, 1H); 6,29 (m, 1H); 4,43 (m, 4H); 4,26 (m, 2H); 3,16 (m, 2H); 2,75 (m, 1H); 2,60 (m, 1H); 1,21 und 1,22 (s, 9H) ppm.

³¹P-NMR (121 MHz, CDCl₃): -5,67; -5,90 ppm.

### 1.3. 3'- bzw. 5'-Aminosäureester des BVDU

### 1.3.1. 5'-O-(N-t-Butyloxycarbonyl-ε-aminocaproyl)-2'-desoxy-5-(E)-bromvinyluridin

Bei 0°C werden 7,72 g (23,2 mmol) 5-(E)-Bromvinyl-2'-desoxyuridin und 9,12 g (34,8 mmol, 1,5 Äq.) Triphenylphosphin in 80 ml DMF vorgelegt. Dann werden 8,04 g (34,8 mmol, 1,5 Äq.) N-t-Butyloxycarbonyl-ε-aminocapronsäure und 7,03 g (34,8 mmol, 1,5 Äq.) Diisopropylazodicarboxylat in 50 ml DMF gelöst und innerhalb von 1 h zutropft. Anschließend wird das Reaktionsgemisch 2 h bei Raumtemperatur gerührt. Das Solvens wird am Rotationsverdampfer entfernt. Säulenchromatographische Reinigung (CHCl₃/MeOH, 95/5, Essigester) liefert 2,98 g (5,45 mmol, 23 %) 5'-O-(N-t-Butyloxycarbonyl-ε-aminocaproyl)-2'-desoxy-5-(E)-bromvinyluridin als weißen Feststoff mit einem Schmelzpunkt von 121 - 122 °C. Als Nebenprodukt konnten 1,05 g (1,92 mmol, 8 %) an 3'-O-(N-t-Butyloxycarbonyl-ε-aminocaproyl)-2'-desoxy-5-(E)-bromvinyluridin als weißen Feststoff mit einem Schmelzpunkt von 204 - 205 °C isoliert werden.

**5'-O-(N-t-Butyloxycarbonyl-ε-aminocaproyl)-2'-desoxy-**5-(E)-bromvinyluridin:
¹H-NMR (500 MHz, DMSO-d₆): 1,20-1,26 (m, 2H); 1,31-1,40 (m, 2H); 1,36 (s, 9H); 1,48-1,53 (m, 2H); 2,15-2,26 (m, 2H); 2,28-2,32 (m, 2H); 2,87 (q, 2H); 3,92 (q, 1H); 4,19-4,25 (m, 3H); 5,43 (d, 1H); 6,15 (t, 1H); 6,74 (t, 1H); 6,93 (d, 1H); 7,30 (d, 1H); 7,31 (d, 1H); 7,77 (s, 1H); 11,62 (s, 1H) ppm.

### 1.3.2. 3'-O-(N-t-Butyloxycarbonyl-ε-aminocaproyl)-2'-desoxy-5-(E)-bromvinyluridin

3'-O-(N-t-Butyloxycarbonyl-ε-aminocaproyl)-2'-desoxy-5-(E)-bromvinyluridin:
¹H-NMR (500 MHz, DMSO-d₆): 1,20-1,26 (m, 2H); 1,31-1,40 (m, 2H); 1,36 (s, 9H); 1,47-1,52 (m, 2H); 2,09-2,32 (m, 6H); 2,87 (q, 2H); 3,93-3,97 (m, 2H); 4,10-4,14 (m, 1H); 4,21-4,28 (m, 5H); 5,42 (d, 1H); 5,45 (d, 1H); 6,09-6,12 (m, 1H); 6,18-6,21 (m, 1H); 6,73 (t, 1H); 6,96 (d, 1H); 6,99 (d, 1H); 7,29-7,36 (m, 2H); 7,86 (s, 1H); 8,01 (s, 1H); 11,58 (s, 1H) ppm. Es liegt ein Gemisch zweier Rotationsisomerer vor.

### 1.3.3. [5'-O-(ε-Aminocaproyl)-2'-desoxy-5-(E)-bromvinyluridin]trifluoracetat

Bei 0°C werden 300 mg (549 µmol) 5'-0-(N-t-Butyloxycarbonyl-ε-aminocaproyl)-2'-desoxy-5-(E)-bromvinyl-uridin in 5 ml Dichlormethan vorgelegt, 0,4 ml (5,6 mmol) Trifluoressigsäure werden zugegeben und anschließend wird das Reaktionsgemisch 2 h bei Raumtemperatur gerührt. Das Solvens wird am Rotationsverdampfer entfernt. Das Rohprodukt wird mit 5 ml Diethylether versetzt und 20 h bei Raumtemperatur gerührt. Das Solvens wird abdekantiert und der Rückstand am Rotationsverdampfer für 1 h bei 40°C belassen. Es resultieren 300 mg (535 µmol, 98 %) [5'-O-(ε-Aminocaproyl)-2'-desoxy-5-(E)-bromvinyluridin] trifluoracetat als ein weißer Feststoff mit einem Schmelzpunkt von 169 - 170 °C.

¹H-NMR (300 MHz, DMSO-d₆): 1,23-1,38 (m, 2H); 1,45-1,58 (m, 4H); 2,12-2,27 (m, 2H); 2,29-2,37 (m, 2H); 2,70-2,80 (m, 2H); 3,89-3,94 (m, 1H); 4,19-4,29 (m, 3H); (d, 1H); 6,16 (t, 1H); 6,94 (d, 1H); 7,30 (d, 1H); 7,69 (bs, 3H); 7,77 (s, 1H); 11,62 (s, 1H) ppm.

### 1.3.4. [3'-O-(ε-Aminocaproyl)-2'-desoxy-5-(E)-bromvinyluridin] trifluoracetat

Bei 0°C werden 300 mg (549 µmol) 5'-O-(N-t-Butyloxy-carbonyl-ε-aminocaproyl)-2'-desoxy-5-(E)-bromvinyl-uridin in 5 ml Dichlormethan vorgelegt, 0,4 ml (5,6 mmol) Trifluoressigsäure werden zugegeben und anschließend wird das Reaktionsgemisch 2 h bei Raumtemperatur gerührt. Das Solvens wird am Rotationsverdampfer entfernt. Das Rohprodukt wird mit 5 ml Diethylether versetzt und 20 h bei Raumtemperatur gerührt. Das Solvens wird abdekantiert und der Rückstand am Rotationsverdampfer für 1 h bei 40°C belassen. Es resultieren 280 mg (500 µmol, 91 %) [3'-O-(ε-Aminocaproyl)-2'-desoxy-5-(E)-bromvinyluridin] trifluoracetat als ein weißer Feststoff der sich bei Temperaturen über 180 °C zersetzt.

¹H-NMR (300 MHz, DMSO-d₆): 1,21-1,35 (m, 2H); 1,45-1,58 (m, 4H); 2,08-2,38 (m, 6H); 2,70-2,80 (m, 2H); 3,91-3,98 (m, 2H); 4,10-4,32 (m, 6H); 5,41-5,49 (m, 2H); 6,08-6,15 (m, 1H); 6,16-6,23 (t, 1H); 6,95 (d, 1H); 7,00 (d, 1H); 7,31 (d, 1H); 7,35 (d, 1H); 7,62 (bs, 3H); 7,86 (s, 1H); 8,01 (s, 1H); 11,59 (s, 1H) ppm. Die Substanz liegt als Gemisch zweier Rotationsisomerer vor.

### 1.4. 3'- bzw. 5'- Acetamidoderivate von BVDU

### 1.4.1. 5'-Acetamido-3'-chlor-2',3',5'-tridesoxy-5-(E)-bromvinyluridin

### 1.4.1.1. 5'-Acetamido-2',5'-didesoxy-5-(E)-bromvinyluridin

1,41 g (4,26 mmol) 5'-Amino-2',5'-didesoxy-5-(E)-bromvinyluridin werden in 15 ml DMF bei 0°C vorgelegt und 674 mg (8,52 mmol) Pyridin zugeben. 401 mg (5,11 mmol) Acetylchlorid in 5 ml DMF werden zugetropft und anschließend weitere 2 h gerührt. Das Reaktionsgemisch wird auf 50 g Eis gegossen und mit konz. Salzsäure auf pH = 7 eingestellt. Das Gemisch wird 3 x mit je 100 ml Essigester extrahiert. Die organische Phase wird mit Na₂SO₄ getrocknet, filtriert und das Solvens am Rotationsverdampfer entfernt. Säulenchromatographische Reinigung (CHCl₃/MeOH, 9/1) liefert 870 mg (2,33 mmol, 55 %) 5'-Acetamido-2',5'-didesoxy-5-(E)-bromvinyluridin als weißen Feststoff.

### 1.4.1.2. 5'-Acetamido-2,3'-anhydro-2',5'-didesoxy-5-(E)-bromvinyluridin

870 mg (2,33 mmol) 5'-Acetamido-2',5'-didesoxy-5-(E)-bromvinyluridin werden in 15 ml DMF vorgelegt und 915 mg (3,49 mmol) PPH₃ zugegeben. Anschließend werden 705 mg (3,49 mmol) Diisopropylazodicarboxylat in 5 ml DMF gelöst und zugetropft. Nach einer Stunde wird das Reaktionsgemisch auf 100 ml Diethylether gegossen, der resultierende Feststoff abgesaugt und 3x mit 20 ml Diethylether gewaschen. Nach Trocknung resultieren 720 mg (2,02 mmol, 87 %) an 5'-Acetamido-2,3'-anhydro-2',5'-didesoxy-5-(E)-bromvinyluridin als weißer Feststoff.

### 1.4.1.3. 5'-Acetamido-3'-chlor-2',3',5'-tridesoxy-5-(E)-bromvinyluridin

720 mg (2,02 mmol) 5'-Acetamido-2,3'-anhydro-2',5'-didesoxy-5-(E)-bromvinyluridin werden in 10 ml DMF vorgelegt und 467 mg (4,04 mmol) Pyridiniumhydrochlorid zugegeben und anschließend 2 h unter Rückfluss erhitzt. Das Solvens wird am Rotationsverdampfer entfernt. Säulenchromatographische Reinigung (CHCl₃/MeOH, 9/1, Dichlormethan/Essigester 1/2) liefert 460 mg (1,17 mmol, 58 %) 5'-Acetamido-3'-chlor-2',3',5'-tridesoxy-5-(E)-bromvinyluridin als weißen Feststoff mit einem Schmelzpunkt von 163-165°C. ¹H-NMR (300 MHz, DMSO-d₆): 1,83 (s, 3H); 2,47-2,73 (m, 2H); 3,36-3,43 (m, 2H); 4,02 (q, 1H); 4,55 (q, 1H); 6,19 (t, 1H); 6,96 (d, 1H); 7,32 (d, 1H); 7,84 (s, 1H); 8,10 (t, 1H); 11,64 (s, 1H).

### 1.4.2. 3'-Acetamido-5'-brom-2',3',5'-tridesoxy-5-(E)-bromvinyluridin

### 1.4.2.1. 3'-Acetamido-2',3'-didesoxy-5-(E)-bromvinyluridin

1,00 g (2,79 mmol) 3'-Azido-2',3'-didesoxy-5-(E)-bromvinyluridin werden in 40 ml (0,1 M, pH = 7,4) Phosphatpufferlösung vorgelegt. 638 mg (8.38 mmol) Thioessigsäure werden zugegeben und bei 60°C 30 h gerührt. Das Solvens wird am Rotationsverdampfer entfernt. Säulenchromatographische Reinigung (CHCl₃/MeOH, 9/1) liefert 470 mg (1,26 mmol, 45 %) 3'-Acetamido-2',3'-didesoxy-5-(E)-bromvinyluridin als weißen Feststoff.

### 1.4.2.2. 3'-Acetamido-5'-o-(methylsulfonyl)-2',3'-didesoxy-5-(E)-bromvinyluridin

500 mg (1,34 mmol) 3'-Acetamido-2',3'-didesoxy-5-(E)-bromvinyluridin werden in 5 ml Pyridin bei 0°C vorgelegt. 160 mg (1,40 mmol) Methansulfonsäurechlorid in 2 ml THF werden zugetropft und anschließend 4 h bei Raumtemperatur gerührt. Das Reaktionsgemisch wird auf Eis gegossen, mit konz. Salzsäure auf pH = 5 eingestellt und 3 x mit Essigester extrahiert. Die organische Phase wird mit verd. Salzsäue und ges. NaCl-Lsg. gewaschen, mit Na₂SO₄ getrocknet und das Solvens wird am Rotationsverdampfer entfernt. Es resultieren 440 mg (973 µmol, 73 %) 3'-Acetamido-5'-O-(methylsulfonyl)-2',3'-didesoxy-5-(E)-bromvinyluridin als weißer Feststoff.

### 1.4.2.3. 3'-Acetamido-5'-brom-2',3',5'-tridesoxy-5-(E)-bromvinyluridin

440 mg (973 µmol) 3'-Acetamido-5'-O-(methylsulfonyl)-2',3'-didesoxy-5-(E)-bromvinyluridin werden in 5 ml DMF vorgelegt. 253 mg (2,92 mmol) LiBr werden zugegeben und das Reaktionsgemisch für 3 h erhitzt. Das Solvens wird am Rotationsverdampfer entfernt. Das Reaktionsgemisch wird in 20 ml Essigester aufgenommen und die organische Phase wird mit NaCl-Lsg. gewaschen. Die wässrige Phase wird 3 x mit Essigester extrahiert. Die vereinte organische Phase wird mit ges. NaCl-Lsg. gewaschen, mit Na₂SO₄ getrocknet und das Solvens wird am Rotationsverdampfer entfernt. Säulenchromatographische Reinigung (Chloroform/Methanol, 9/1) liefert 290 mg (663 µmol, 68 %) 3'-Acetamido-5'-brom-2',3',5'-tridesoxy-5-(E)-bromvinyluridin als weißen Feststoff mit einem Schmelzpunkt von 178-180°C.

¹H-NMR (300 MHz, DMSO-d₆): 1,84 (s, 3H); 2,15-2,45 (m, 2H); 3,70-3,80 (m, 2H); 3,94 (q, 1H); 4,31 (m, 1H); 6,21 (t, 1H); 6,95 (d, 1H); 7,30 (d, 1H); 7,87 (s, 1H); 8,30 (t, 1H); 11,63 (s, 1H).

### 2. 5'-Substituierte BVDU- und BVRU-Derivate als Referenzverbindungen

### 2.1. 5'-Halogen-BVDU

### 2.1.1. 2',5'-Didesoxy-5'-fluor-5-(E)-bromvinyluridin

3,5 g (7,18 mmol) 5'-O-Methylsulfonyl-2'-desoxy-5-(E)-bromvinyluridin und 9,06 g (28,72 mmol) Tetrabutylammoniumfluorid Trishydrat werden in 50 ml DMF gelöst. Nach Zugabe von 20 g Molsieb (3Ǻ) wird auf 40 °C erwärmt. Nach 4,5 h ist die Reaktion beendet (DC-Kontrolle mit Chloroform/Methanol 10 : 1). Man filtriert über Celite, wäscht gründlich mit DMF nach und destilliert DMF ab. Xylol wird als Schlepper zugesetzt. Der Rückstand wird in Essigester gelöst und mit 1 M Salzsäure gewaschen. Die Essigesterphase wird mit Phosphatpuffer, danach mit gesättigter Kochsalzlösung gewaschen. Die vereinigten wässrigen Phasen werden neutralisiert und mit Essigester extrahiert. Alle Essigesterphasen werden vereinigt und über Magnesiumsulfat getrocknet. Nach Filtrieren und Abrotieren des Lösungsmittels erfolgt säulenchromatographische Reinigung (Chloroform/Methanol 15 : 1). Man er hält 1,05 g (43,8 %) eines farblosen Feststoffes vom Schmelzpunkt 208 °C.

¹H-NMR (500 MHz, DMSO-d₆): 2,18 (m, 2H) ; 3,93 (m, 1H); 4,27 (m, 1H); 4,60 (m, 2H); 5,45 (d, 1H); 6,18 (d, 1H); 6,92 (d, 1H); 7,28 (d, 1H); 7,74 (s, 1H); 11,60 (s, 1H) ppm.

Fig. 4 zeigt die Ergebnisse einer Referenzverbindung in Kombination mit Mitomycin C (MMC) im Vergleich zu MMC alleine und MMC in Kombination mit BVDU.

### 2.1.2. 3'-O-Methyl-5'-chlor-2',5'-didesoxy-5-(E)-bromvinyluridin

0,55 g (1,56 mmol) 2',5'-Didesoxy-5'-chlor-5-(E)-bromvinyluridin wird in 9 ml Dioxan gelöst. Dazu werden 3 ml Toluol, 0,03 ml Wasser und 0,46 g (8,2 mmol) Kaliumhy-droxid gegeben. Nach 2 h hat sich eine feine Suspension gebildet. Danach werden 0,44 g (3,12 mmol) Jodmethan zugesetzt. Nach 1h wird dieselbe Menge Jodmethan zugefügt und nach einer weiteren Stunde noch mal. Danach ist die Reaktion beendet (DC-Kontrolle mit Chloroform/Methanol 10 : 1). Der Ansatz wird einem Zweiphasengemisch aus 25 ml Essigester und 25 ml Phosphatpuffer zugefügt und die wässrige Phase mit Essigester extrahiert. Die vereinigten organischen Phasen werden mit Magnesiumsulfat getrocknet. Nach Filtration und Abrotieren des Lösungsmittels erfolgt säulenchromatographische Reinigung (Chloroform/Methanol 60 : 1). Man erhält 430 mg (75,4 %) eines farblosen Feststoffes vom Schmelzpunkt 145 °C.

¹H-NMR (500 MHz, DMSO-d₆): 2,30 (m, 2H); 3,31 (s, 3H); 3,86 (m, 2H); 3,99 (m, 1H); 4,11 (m, 1H); 6,12 (m, 1H); 6,90 (d, 1H); 7,29 (d, 1H); 7,82 (s, 1H); 11,65 (s, 1H) ppm.

### 2.1.3. 3'-O-Methyl-5'-fluor-2',5'-didesoxy-3-methyl-5-(E)-bromvinyl-uridin

1,24 g (3,7 mmol) 2',5'-Didesoxy-5'-fluor-5-(E)-bromvinyluridin werden in 40 ml Dioxan gelöst und mit 15 ml Toluol versetzt. Danach gibt man 1,12 g (20 mmol) Kaliumhydroxid hinzu und 65 µl Wasser. Man lässt 2 h bei Raumtemperatur rühren und erhält eine feine Suspension. Hierzu gibt man 1,57 g Methyljodid und nach 2 h und 4 h Rühren noch mal die gleiche Menge Methyljodid. Nach 16 h Rühren bei Raumtemperatur ist der Umsatz vollständig (DC-Kontrolle mit Chloroform/Methanol 10 : 1). Das Reaktionsgemisch wird in 100 ml Phosphatpufferlösung eingegossen und mit Essigester extrahiert (3 x 50 ml). Die vereinigten Ex-trakte werden mit Magnesiumsulfat getrocknet. Nach Filtration und Abrotieren des Lösungsmittels erfolgt säulenchromatographische Reinigung (Dichlormethan/Aceton 50 : 1; Cyclohexan/Essigester 1 : 1). Aus der konzentrierten Lösung wird das Produkt mit Cyclohexan ausgefällt und getrocknet. Man erhält 0,63 g (46,9 %) eines farblosen Feststoffes vom Schmelzpunkt 95 °C.

¹H-NMR (500 MHz, DMSO-d₆) : 2,24 (m, 1H); 2,35 (m, 1H); 3,18 (s, 3H); 3,30 (s, 3H); 4,05 (m, 1H); 4,19 (m, 1H); 4,61 (m, 1H); 4,71 (m, 1H); 6,15 (t, 1H); 6,94 (d, 1H); 7,33 (d, 1H); 7,82 (s, 1H) ppm.

### 2.1.4. 3'-O-Methyl-5'-fluor-2',5'-didesoxy-5-(E)-bromvinyluridin

### 2.1.4.1. 5'-O-(4,4'-Dimethoxytrityl)-2'-desoxy-5-(E)-bromvinyl-uridin

8,20 g (24,6 mmol) 2'-Desoxy-5-(E)-bromvinyl-uridin werden in 100 ml Pyridin gelöst. Dazu gibt man 0,75 g (6,15 mmol) 4-N,N-Dimethylaminopyridin und 5,06 g (50 mmol) Triethylamin. Man kühlt auf 0 °C und zwei Portionen von je 5 g (insgesamt 10,0 g, 29,51 mmol) 4,4'-Dimethoxytritylchlorid hinzu. Man lässt 30 min rühren, entfernt dann das Kältebad und lässt auf Raumtemperatur aufwärmen. Nach 22 h ist der Umsatz vollständig (DC-Kontrolle mit Dichlormethan/Methanol 15 : 1). Nach Zugabe von 30 ml Ethanol und 20 min rühren wird Pyridin mit Toluol mehrfach abdestilliert. Das erhaltene dunkelbraune Öl wird in Essigester (200 ml) gelöst und mit 5 % Kaliumhydrogencarbonatlösung gewaschen. Die wässrige Phase wird noch dreimal mit Essigester extrahiert und alle Essigesterphasen vereinigt und mit Magnesiumsulfat getrocknet. Nach Filtration und Abdestillieren des Lösungsmittels erfolgt säulenchromatographische Reinigung (Dichlormethan/Methanol 35 : 1 mit 1 % Triethylamin). Man erhält 13,57 g (86,8 %) eines farblosen Schaums.

### 2.1.4.2. 3'-O-Methyl-5'-O-(4,4'-dimethoxytrityl)-2'-desoxy-5-(E)-bromvinyl-uridin

7,5 g (11,8 mmol) 5'-O-(4,4'-Dimethoxytrityl)-2'-desoxy-5-(E)-bromvinyl-uridin werden in einem Gemisch aus 150 ml Dioxan und 50 ml Toluol gelöst und dazu gibt man 3,37 g (60 mmol) Kaliumhydroxid und 0,29 ml Wasser. Nach 2 h rühren bei Raumtemperatur erhält man eine feine farblose Suspension. Man gibt 6,71 g (47,3 mmol) Methyljodid hinzu und lässt 1 h rühren. Danach wird noch einmal dieselbe Menge Methyljodid und nach weiteren 2 h die halbe Menge Methyljodid zugesetzt. Nach 1,5 h ist der Umsatz vollständig (DC-Kontrolle mit Dichlormethan/Methanol 20 :1; Chloroform/Methanol 20 :1)..Der Ansatz wird in 200 ml Phophatpufferlösung eingegossen und danach wird mit Essigester mehrfach extrahiert. Die vereinigten Extrakte werden mit Magnesiumsulfat getrocknet und nach Abrotieren des Lösungsmittels erfolgt säulenchromatographische Reinigung (Dichlormethan /Metha-nol 50 : 1). Man erhält 6,32 g (82,5 %) eines gelblichen Schaums.

### 2.1.4.3. 3'-O-Methyl-2'-desoxy-5-(E)-bromvinyl-uridin

6,32 g (9,72 mmol) 3'-O-Methyl-5'-O-(4,4'-dimethoxytrityl)-2'-desoxy-5-(E)-bromvinyl-uridin werden in 120 ml Chloroform gelöst. Dazu gibt man 60 ml Methanol und nach Kühlung auf 0 °C 1,85 g (9,72 mmol) 4-Methylsulfonsäuremonohydrat. Nach 1 h (DC-Kontrolle mit Dichlormethan/Methanol 15 :1) ist die Reaktion beendet. Man gibt 2,3 g (23 mmol) Kaliumhydrogencarbonat hinzu, rührt 5 min und versetzt mit 200 ml gesättigter Kochsalzlösung. Man extrahiert mit Chloroform und Essigester, vereinigt alle Extrakte und trocknet mit Magnesiumsulfat. Nach Filtration und Abdestillieren des Lösungsmittels erfolgt Extraktion mit Cyclohexan, Filtration und waschen des Rückstandes mit Cyclohexan. Nach Trocknung erhält man 3,33 g (98,5 %) eines farblosen Feststoffes.

### 2.1.4.4. 5'-O-(4-Methylbenzolsulfonyl)-3'-O-Methyl-2'-desoxy-5-(E)-bromvinyl-uridin

3,33 g (9,6 mmol) 3'-O-Methyl-2'-desoxy-5-(E)-bromvinyl-uridin werden in 30 ml Pyridin gelöst und die erhaltene Lösung wird auf 0 °C gekühlt. Danach gibt man 2,2 g 4-Methylbenzolsulfonylchlorid hinzu, lässt 30 min bei 0 °C rühren und dann auf Raumtemperatur aufwärmen. Nach 7 h gibt man noch mal 1,55 g (8,13 mmol) 4-Methylbenzolsulfonylchlorid hinzu und rührt 20 h. Der Umsatz ist dann vollständig (DC-Kontrolle mit Dichlormethan/Methanol 15 : 1). Man gießt auf Eis, rührt 30 min und säuert mit 6 M Salzsäure an. Nach Extraktion mit Essigester werden die vereinigten Extrakte mit 1 M Salzsäure, danach mit Phosphatpuffer gewaschen und mit Magnesiumsulfat getrocknet. Nach Abdestillieren des Lösungsmittels und säulenchromatographischer Reinigung (Cyclohexan/Aceton 2 : 1) erhält man 3,32 g (68,9 %) eines farblosen Feststoffes.

### 2.1.4.5. 3'-O-Methyl-5'-fluor-2',5'-didesoxy-5-(E)-bromvinyluridin

2,5 g (4,98 mmol) 5'-O-(4-Methylbenzolsulfonyl)-3'-O-Methyl-2'-desoxy-5-(E)-brom-vinyl-uridin werden in 40 ml DMF gelöst und es werden 6,27 g (20 mmol) Tetra-n-butylammoniumfluoridtrishydrat und 15 g Molsieb (3 Å) hinzugefügt. Es wird auf 35 °C erwärmt. Nach 1 h ist die Reaktion beendet (DC-Kontrolle Cyclohexan/Essigester 1 : 1). Man filtriert über Celite ab und wäscht mit DMF nach. DMF wird durch abdestillieren mit Xylol entfernt, der Rückstand in Essigester gelöst und mit 1 M Salzsäure (80 ml) gewaschen. Die wässrige Phase wird mit Essigester extrahiert und alle vereinigten Essigesterphasen werden mit Phosphatpufferlösung gewaschen. Nach Trocknen der organischen Phase mit Magnesiumsulfat und abdestillieren des Lösungsmittels erfolgt säulenchromatographische Reinigung mit Cyclohexan/ Essigester 1 : 1. Man 0,99 g (56,9 %) eines farblosen Feststoffes vom Schmelzpunkt 177 °C.

¹H-NMR (500 MHz, DMSO-d₆): 2,23 (m, 1H) ; 2,34 (m, 1H); 3,30 (s, 3H); 4,14 (m, 1H); 4,32 (m, 1H); 4,58 (m, 1H); 4,68 (m, 1H); 6,12 (m, 1H); 6,81 (d, 1H); 7,28 (d, 1H); 7,76 (s, 1H); 11,63 (s, 1H) ppm.

### 2.2. Ester von 5'-Halogen-BVDU

### 2.2.1. 3'-O-Acetyl-5'-Fluor-2'-desoxy-5-(E)-bromvinyl-uridin

300 mg (0,9 mmol) 2',5'-Didesoxy-5'-fluor-5-(E)-bromvinyluridin werden in einer Mischung aus 5 ml Dichlormethan und 1 ml Pyridin suspendiert, auf 0 °C gekühlt, und mit 190 mg (2,4 mmol) Acetylchlorid versetzt. Nach 2 h ist der Umsatz vollständig (DC-Kontrolle mit Chloroform/Methanol 15 : 1). Es wird mit 25 ml Essigester verdünnt und die organische Phase mit 2 X 25 ml 0,1 M Salzsäure gewaschen. Mit Phosphatpuffer wird neutral gewaschen. Nach Trocknung über Magnesiumsulfat, Abfiltrieren und Abrotieren des Lösungsmittels erfolgt säulenchromatographische Reinigung (Dichlormethan/ Essigester 4 : 1). Nach Umfällen aus t-Butylmethylether/Cyclohexan erhält man 90 mg (27 %) eines farblosen Feststoffes vom Schmelzpunkt 81 °C.

¹H-NMR (500 MHz, DMSO-d₆): 2,07 (s, 3H) ; 2,42 (m, 2H); 4,25 (m, 1H); 4,70 (m, 2H); 5,24 (m, 1H); 6,16 (m, 1H); 6,92 (d, 1H); 7,30 (d, 1H); 7,81 (s, 1H); 11,66 (s, 1H) ppm.

### 2.2.2. Aminosäureester von 5'-Halogen-BVDU

### 2.2.2.1. 4-(t-Butoxycarbonyl)amino-1-(5'-fluor-2',5'-didesoxy-5-(E)-bromvinyluridin-3'-yl)butanoat

280 mg (1,33 mmol) N-BOC-4-Aminobuttersäure und 290 mg (2,39 mmol) 4-Dimethylaminopyridin werden in 20 ml Dichlormethan gelöst. Dazu gibt man 400 mg (1,19 mmol) 2',5'-Didesoxy-5'-fluor-5-(E)-bromvinyluridin und 280 mg (1,34 mmol) N,N'-Dicyclohexylcarbodiimid. Nach 2 h ist die Reaktion beendet (DC-Kontrolle mit Chloroform/Methanol 15 : 1). Der Ansatz wird filtriert und einrotiert. Nach Lösen in Essigester wird auf -20 °C gekühlt, der Niederschlag abfiltriert und mit wenig auf -20 °C gekühltem Essigester gewaschen. Das Filtrat wird einrotiert und mit Dichlormethan/Methanol (30 : 1) mehrfach säulenchromatographisch gereinigt. Man erhält 400 mg (64,1 %) eines farblosen Schaums.

¹H-NMR (500 MHz, DMSO-d₆): 1,37 (s, 9H) ; 1,62 (m, 2H); 2,37 (m, 2H); 2,40 (m, 1H); 2,51 (m, 1H) ; 2,94 (m, 2H); 4,25 (m, 1H); 4,68 (m, 2H); 5,24 (m 1H); 6,18 (m, 1H); 6,84 (t, 1H); 6,91 (d, 1H); 7,28 (d, 1H); 7,83 (s, 1H); 11,66 (s, 1H) ppm.

### 2.2.2.2. 4-Amonium-1-(5'-fluor-2',5'-didesoxy-5-(E)-bromvinyluridin-3'-yl)butanoat-trifluoracetat

290 mg (0,56 mmol) Produkt aus 2.2.2.1. werden in 5 ml Dichlormethan gelöst und mit 5 ml Trifluoressigsäure versetzt. Nach 30 min ist die Umsetzung beendet (DC-Kontrolle mit Dichlormethan/Methanol 25 : 1). Nach mehrmaligem Abrotieren mit Dichlormethan wird der Rückstand mit Diethylether behandelt und der farblose Rückstand abfiltriert und mit Diethylether gewaschen. Nach Trocknung erhält man 270 mg (90,6 %) eines farblosen Pulvers mit Schmelzpunkt 129 °C.

¹H-NMR (500 MHz, DMSO-d₆): 11,75 (s, 1H); 7,82 (s, 1H); 7,71 (s (br.), 3H); 7,29 (d, 1H) ; 6,91 (d, 1H) ; 6,18 (m, 1H); 5,27 (m, 1H); 4,74 (m, 1H); 4,67 (m, 1H); 4,24 (m, 1H); 2,83 (m, 2H); 2,44 (m, 3H); 2,37 (m, 1H); 1,80 (m, 2H) ppm.

### 2.2.2.3. 4-(t-Butoxycarbonyl)amino-1-(5'-chlor-2',5'-didesoxy-5-(E)-bromvinyluridin-3'-yl)butanoat

500 mg (1,42 mmol) 5'-Chlor-2',5'-didesoxy-5-(E)-bromvinyl-uridin, 290 mg (1,42 mmol N-BOC-4-Aminobuttersäure) und 175 mg (1,42 mmol) 4-Dimethylaminopyridin werden in 12 ml Dichlormethan gegeben, die erhaltene Suspension wird auf 0 °C gekühlt und danach werden 423 mg (1,42 mmol) 1-(3-Dimethylaminopropyl)-3-ethylcarbodiimid Methiodid zugesetzt. Nach Entfernen des Kältebades lässt man 6 h rühren. Danach ist die Reaktion beendet (DC-Kontrolle mit Chloroform/Methanol 10 : 1). Dichlormethan wird abrotiert und der Rückstand in Essigester aufgenommen. Die organische Phase wird mehrfach mit gesättigter Kochsalzlösung gewaschen. Die vereinigten wässrigen Phasen werden mit Essigester extrahiert. Alle organischen Phasen werden vereinigt und mit Magnesiumsulfat getrocknet. Nach Filtration und Abrotieren des Lösungsmittels erfolgt säulenchromatgraphische Reinigung (Dichlormethan/Methanol 50 : 1; Cyclohexan/Essigester 1 : 1). Man erhält 520 mg (68,2 %) eines farblosen Schaums.

¹H-NMR (500 MHz, DMSO-d₆): 1,37 (s, 9H); 1,64 (m, 2H); 2,36 (m, 3H); 2,51 (m, 1H); 2,94 (m, 2H); 3,91 (m, 2H); 4,21 (m, 1H); 5,24 (m, 1H); 6,18 (m, 1H); 6,82 (t, 1H); 6,91 (d, 1H); 7,29 (d, 1H); 7,87 (s, 1H); 11,67 (s, 1H) ppm.

### 2.2.2.4. 4-Amonium-1-(5'-chlor-2',5'-didesoxy-5-(E)-bromvinyluridin-3'-yl)butanoat-tri-fluoracetat

200 mg (0,372 mmol) 4-(t-Butoxycarbonyl)amino-1-(5'-chlor-2',5'-didesoxy-5-(E)-bromvinyluridin-3'-yl)butanoat werden in 4 ml Dichlormethan gelöst, danach werden 7 ml Trifluoressigsäure zugesetzt. Nach 30 min ist alles umgesetzt (DC-Kontrolle mit Cyclohexan/Essigester 1 : 1). Nach mehrfachem Abrotieren mit Dichlormethan wird der Rückstand mit Diethylether behandelt und der farblose Niederschlag wird abfiltriert und getrocknet. Man erhält 160 mg (78,0 %) eines farblosen Pulvers vom Schmelzpunkt 148 °C.

¹H-NMR (500 MHz, DMSO-d₆) : 11,75 (s, 1H) ; 7,87 (s, 1H); 7,72 (s, 3H); 7,30 (d, 1H); 6,91 (d, 1H); 6,20 (m, 1H); 5,25 (m, 1H); 4,21 (m, 1H); 3,91 (m, 2H); 2,83 (m, 2H); 2,35 (m, 4H); 1,81 (m, 2H) ppm.

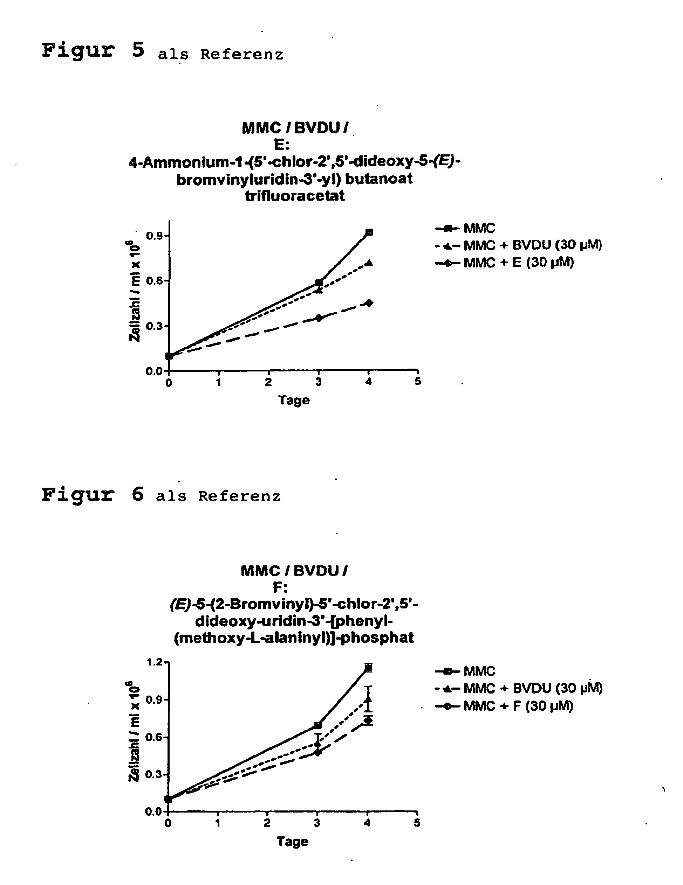

Fig. 5 zeigt die Ergebnisse einer Referenzverbindung in Kombination mit Mitomycin C (MMC) im Vergleich zu MMC alleine und MMC in Kombination mit BVDU.

### 2.2.2.5. 3'-O-(N-t-Butyloxycarbonyl-L-valinoyl)-5'-chlor-2',5'-didesoxy-5-(E)-bromvinyluridin

Bei 0°C werden 1,00 g (2,84 mmol) 5'-Chlor-2',5'-didesoxy-5-(E)-bromvinyluridin in 20 ml Dichlormethan vorgelegt. Dann werden 618 mg (2,84 mmol, 1,0 Äq.) N-t-Butyloxycarbonyl-L-valin, 347 mg (2,84 mmol, 1,0 Äq.) N,N-Dimethylaminopyridin sowie 587 mg (2,84 mmol, 1,0 Äq.) N,N'-Dicyclohexylcarbodiimid zugegeben und anschließend wird das Reaktionsgemisch 20 h bei Raumtemperatur gerührt. Der entstandene Niederschlag wird abfiltriert und mit Dichlormethan gewaschen. Das Filtrat wird mit verdünnter Zitronensäurelösung, NaH-CO₃-Lösung sowie NaCl-Lösung gewaschen und anschließend mit Na₂SO₄ getrocknet. Das Solvens wird am Rotationsverdampfer entfernt. Säulenchromatographische Reinigung (CHCl₃/MeOH, 95/5, Dichlormethan/Essigester, 3/1) liefert 1,00 g (1,82 mmol, 64 %) 3'-O-(N-t-Butyloxycarbonyl-L-valinoyl)-5'-chlor-2',5'-didesoxy-5-(E)-bromvinyluridin als weißen Feststoff mit einem Schmelzpunkt von 87-88°C.

¹H-NMR (500 MHz, DMSO-d₆): 0,89-0,91 (m, 6H); 1,39 (s, 9H); 2,03 (m, 1H); 2,31 (m, 1H); 2,53 (m, 1H); 3,83 (t, 1H); 3,88-3,91 (m, 2H); 4,12 (m, 1H); 5,28 (m, 1H); 6,22 (t, 1H); 6,91 (d, 1H); 7,29 (d, 1H); 7,32 (d, 1H); 7,87 (s, 1H); 11,68 (s, 1H) ppm.

### 2.2.2.6. (5'-Chlor-2',5'-didesoxy-3'-O-L-valinoyl-5-(E)-bromvinyluridin) trifluoracetat

Bei 0°C werden 600 mg (1,09 mmol) 3'-O-(N-t-Butyloxycarbonyl-L-valinoyl)-5'-chlor-2',5'-didesoxy-5-(E)-bromvinyluridin in 5 ml Dichlormethan vorgelegt, 1,0 ml (14 mmol) Trifluoressigsäure werden zugegeben und anschließend wird das Reaktionsgemisch 2 h bei Raumtemperatur gerührt. Das Solvens wird am Rotationsverdampfer entfernt. Das Rohprodukt wird mit 5 ml Diethylether versetzt und 20 h bei Raumtemperatur gerührt. Das Solvens wird abdekantiert und der Rückstand am Rotationsverdampfer für 1 h bei 40°C belassen. Es resultieren 450 mg (797 µmol, 73 %) (5'-Chlor-2',5'-didesoxy-3'-O-L-valinoyl-5-(E)-bromvinyluridin) trifluoracetat als ein weißer Feststoff mit einem Schmelzpunkt von 108-109°C.

¹H-NMR (500 MHz, DMSO-d₆): 0,97-1,01 (m, 6H); 2,19 (m, 1H); 2,37 (m, 1H); 2,58 (m, 1H); 3,89-3,97 (m, 2H); 4,00 (bs, 1H); 4,27 (m, 1H); 5,39 (m, 1H); 6,25 (dd, 1H); 6, 92 (d, 1H); 7,31 (d, 1H); 7,87 (s, 1H);

8,37 (bs, 3H); 11,71 (s, 1H) ppm.

### 2.2.2.7. 3'-O-(N-t-Butyloxycarbonyl-L-valyl-L-valinoyl)-5'-chlor-2',5'-didesoxy-5-(E)-bromvinyluridin

Bei 0°C werden 1,00 g (2,84 mmol) 5'-Chlor-2',5'-didesoxy-5-(E)-bromvinyluridin in 20 ml Dichlormethan vorgelegt. Dann werden 900 mg (2,84 mmol, 1,0 Äq.) N-t-Butyloxycarbonyl-L-valyl-L-valin, 347 mg (2,84 mmol, 1,0 Äq.) N,N-Dimethylamino-pyridin sowie 587 mg (2,84 mmol, 1,0 Äq.) N,N'-Dicyclohexylcarbodiimid zugegeben und anschließend wird das Reaktionsgemisch 24 h bei Raumtemperatur gerührt. Der entstandene Niederschlag wird abfiltriert und mit Dichlormethan gewaschen. Das Filtrat wird mit verdünnter Zitronensäurelösung, NaHCO₃-Lösung sowie NaCl-Lösung gewaschen und anschließend mit Na₂SO₄ getrocknet. Das Solvens wird am Rotationsverdampfer entfernt. Säulenchromatographische Reinigung (Dichlormethan /Essigester, 3/1) liefert 850 mg (1,31 mmol, 46 %) 3'-O-(N-t-Butyloxycarbonyl-L-valyl-L-valinoyl)-5'-chlor-2',5'-didesoxy-5-(E)-bromvinyluridin als weißen Feststoff mit einem Schmelzpunkt von 114-116°C.

¹H-NMR (500 MHz, DMSO-d₆): 0,83-0,93 (m, 12H); 1,37 (s, 9H); 1,91 (m, 1H); 2,09 (m, 1H); 2,30 (m, 1H); 3,88-3,91 (m, 3H); 4,13-4,22 (m, 2H); 5,27 (m, 1H); 6,20 (m, 1H); 6,70 (m, 1H); 6,91 (d, 1H); 7,30 (d, 1H); 7,87 (s, 1H); 8,15 (m, 1H); 11,68 (s, 1H) ppm.

### 2.2.2.8. [5'-Chlor-2',5'-didesoxy-3'-O-(L-valyl-L-valinoyl)-5-(E)-bromvinyluridin] trifluoracetat

Bei 0°C werden 200 mg (308 µmol) 3'-O-(N-t-Butyloxycarbonyl-L-valyl-L-valinoyl)-5'-chlor-2',5'-didesoxy-5-(E)-bromvinyluridin in 5 ml Dichlormethan vorgelegt, 0,35 ml (4,7 mmol) Trifluoressigsäure werden zugegeben und anschließend wird das Reaktionsgemisch 3 h bei Raumtemperatur gerührt. Das Solvens wird am Rotationsverdampfer entfernt. Das Rohprodukt wird mit 5 ml Diethylether versetzt und 1 h bei Raumtemperatur gerührt. Das Solvens wird abdekantiert und der Rückstand am Rotationsverdampfer für 1 h bei 40°C belassen. Es resultieren 180 mg (271 µmol, 88 %) [5'-Chlor-2',5'-didesoxy-3'-O-(L-valyl-L-valinoyl)-5-(E)-bromvinyluridin] trifluoracetat als ein weißer Feststoff mit einem Schmelzpunkt von 135-136°C.

¹H-NMR (500 MHz, DMSO-d₆): 0,92-0,99 (m, 12H); 2,08-2,18 (m, 2H); 2,35 (m, 1H); 2,55 (m, 1H); 3,73 (m, 1H); 3,91 (m, 1H); 4,17 (m, 1H); 4,28 (m, 1H); 5,31 (m, 1H); 6,21 (m, 1H) ; 6,91 (d, 1H); 7,31 (d, 1H); 7,87 (s, 1H); 8,10 (bs, 3H); 8,73 (d, 1H); 11,70 (s, 1H) ppm.

### 2.2.2.9. 5'-Azido-3'-O-(N-t-butyloxycarbonyl-L-valyl-L-valinoyl)-2',5'-didesoxy-5-(E)-bromvinyluridin

Bei 0°C werden 1,10 g (3,07 mmol) 5'-Azido-2',5'-didesoxy-5-(E)-bromvinyluridin in 20 ml Dichlormethan vorgelegt. Dann werden 950 mg (3,07 mmol, 1,0 Äq.) N-t-Butyloxycarbonyl-L-valyl-L-valin, 375 mg (3,07 mmol, 1,0 Äq.) N,N-Dimethylamino-pyridin sowie 634 mg (3,07 mmol, 1,0 Äq.) N,N'-Dicyclohexylcarbodiimid zugegeben und anschließend wird das Reaktionsgemisch 2 d bei Raumtemperatur gerührt. Der entstandene Niederschlag wird abfiltriert und mit Dichlormethan gewaschen. Das Filtrat wird mit verdünnter Zitronensäurelösung, NaHCO₃-Lösung sowie NaCl-Lösung gewaschen und anschließend mit Na₂SO₄ getrocknet. Das Solvens wird am Rotationsverdampfer entfernt. Säulenchromatographische Reinigung (Dichlormethan /Essigester, 2/1) liefert 600 mg (914 µmol, 30 %) 5'-Azido-3'-O-(N-t-butyloxy-carbonyl-L-valyl-L-valinoyl)-2',5'-didesoxy-5-(E)-bromvinyluridin als weißen Feststoff mit einem Schmelzpunkt von 133 - 135°C.

¹H-NMR (300 MHz, DMSO-d₆): 0,83-0,94 (m, 12H); 1,38 (s, 9H); 1,93 (m, 1H); 2,10 (m, 1H); 2,34 (m, 1H); 2,53 (m, 1H); 3,58-3,76 (m, 2H); 3,91 (m, 1H); 4,04-4,24 (m, 2H); 5,24 (m, 1H); 6,22 (m, 1H); 6,72 (m, 1H); 6,94 (d, 1H); 7,32 (d, 1H); 7,92 (s, 1H); 8,14 (m, 1H); 11,70 (s, 1H) ppm. Es liegt ein Gemisch zweier Rotamerer im Verhältnis 1:1 vor, weshalb einige Signale doppelt auftreten.

### 2.2.2.10. [5'-Azido-2',5'-didesoxy-3'-O-(L-valyl-L-valinoyl)-5-(E)-bromvinyluridin]trifluoracetat

Bei 0°C werden 200 mg (305 µmol) 5'-Azido-3'-O-(N-t-butyloxy-carbonyl-L-valyl-L-valinoyl)-2',5'-didesoxy-5-(E)-bromvinyluridin in 5 ml Dichlormethan vorgelegt, 0,33 ml (4,47 mmol) Trifluoressigsäure werden zugegeben und anschließend wird das Reaktionsgemisch 4 h bei Raumtemperatur gerührt. Das Solvens wird am Rotationsverdampfer entfernt. Das Rohprodukt wird mit 5 ml Diethylether versetzt und 20 h bei Raumtemperatur gerührt. Das Solvens wird abdekantiert und der Rückstand am Rotationsverdampfer für 1 h bei 40°C belassen. Es resultieren 160 mg (239 µmol, 78 %) [5'-Azido-2',5'-didesoxy-3'-O-(L-valyl-L-valinoyl)-5-(E)-bromvinyluridin] trifluoracetat als ein weißer Feststoff mit einem Schmelzpunkt von 122 - 124°C.

¹H-NMR (300 MHz, DMSO-d₆): 0,92-0,99 (m, 12H) ; 2,09-2,18 (m, 2H); 2,25-2,40 (m, 1H); 2,50-2,62 (m, 1H); 3,56-3,80 (m, 3H); 4,04-4,15 (m, 1H); 4,22-4,29 (m, 1H); 5,25 (m, 1H); 6,20 (m, 1H); 6,92 (d, 1H); 7,31 (d, 1H); 7,91 (s, 1H); 8,10 (bs, 3H); 8,68 (d, 1H); 11,69 (s, 1H) ppm. Es liegt ein Gemisch zweier Rotamerer im Verhältnis 1:2 vor, weshalb einige Signale doppelt auftreten.

### 2.2.3. Phosphoramidate von 5'-Halogen-BVDU

### 2.2.3.1. (E)-5-(2-Bromvinyl)-5'-chlor-2',5'-didesoxy-uridin-3'-[phenyl-(methoxy-L-alaninyl)]-phosphat

1,55 g (7,35 mmol) Phenyldichlorophosphat und 1,03 g (7,35 mmol) L-Alaninmethylester Hydrochlorid werden in 15 ml Dichlormethan gelöst bzw. suspendiert und auf - 78 °C gekühlt. Triethylamin (1,52 g (15 mmol)) wird in 15 ml Dichlormethan gelöst und bei -78 °C innerhalb 2 h zugetropft. Nach dem Zutropfen auf Raumtemperatur aufwärmen und 18 h rühren lassen. Dichlormethan wird abrotiert, der Rückstand in Diethylether aufgenommen und von Ungelöstem abfiltriert. Diethylether wird danach abgezogen und das ölige Rohprodukt ohne Reinigung weiter verarbeitet.

0,57 g (1,62 mmol) 2',5'-Didesoxy-5'-chlor-5-(E)-bromvinyluridin und das zuvor erhaltene Rohprodukt werden in 15 ml THF gelöst und die Lösung auf -78 °C gekühlt. 0,82 g (10 mmol) N-Methylimidazol werden in 5 ml THF gelöst und innerhalb 20 min zugetropft. Man lässt allmählich auf Raumtemperatur aufwärmen und rührt noch 20 h bei dieser Temperatur. Die Reaktion ist danach vollständig (DC-Kontrolle mit Chloroform/Methanol 10 : 1). Der Ansatz wird einem Zweiphasengemisch aus Phosphatpuffer und Essigester zugesetzt und die wässrige Phase mehrfach mit Essigester extrahiert. Die vereinigten Extrakte werden mit Magnesiumsulfat getrocknet, filtriert und das Lösungsmittel abrotiert. Nach säulenchromatographischer Reinigung (Dichlormethan/Methanol 30 : 1; Chloroform/Aceton 5 :1) werden 0,62 g (64,6 %) eines farblosen Schaums erhalten.

¹H-NMR (500 MHz, CDCl₃): 8,56 (s, 1H); 8,55* (s, 1H); 7,66 (s, 1H); 7,63* (s, 1H); 7,35 (m, 3H); 7,22 (m, 3H); 6,68 (d, 1H); 6,66* (d, 1H); 6,30 (m, 1H); 5,11 (m, 1H); 4,50* (m, 1H); 4,37 (m, 1H); 4,02 (m, 1H); 3,91* (m, 2H); 3,83 (m, 2H); 3,76* (s, 3H); 3,73 (s, 3H); 3,71* (m, 1H); 3,63 (m, 1H); 2,65 (m, 1H); 2,58* (m, 1H); 2,25 (m, 1H); 1,40 (d, 3H) ppm.

Die Substanz besteht aus einem Diastereomerengemisch (Verhältnis ca. 1,2 : 1). Die mit einem * markierten NMR-Signale beziehen sich auf das Diastereomer, das in kleinerem Anteil vorliegt.

³¹P-NMR (202 MHz, CDCl₃): 2,31; 1,61 ppm.

Fig. 6 zeigt die Ergebnisse einer Referenzverbindung in Kombination mit Mitomycin C (MMC) im Vergleich zu MMC alleine und MMC in Kombination mit BVDU.

### 2.2.3.2. [5-(E)-Bromvinyl-5'-fluor-2',5'-didesoxyuridin]-3'-yl-(methoxy-L-alaninyl)-phenyl-phosphat

600 mg (2,84 mmol, 2 Äq.) Phenyldichlorophosphat und 397 mg (2,84 mmol, 2 Äq.) L-Alaninmethylester Hydrochlorid werden in 7 ml THF bei -78°C vorgelegt. 576 mg (5,69 mmol, 4 Äq.) Triethylamin werden in 7 ml THF gelöst, innerhalb von 30 min zugetropft und anschließend 20 h bei Raumtemperatur gerührt. Das Reaktionsgemisch wird auf -78°C gekühlt und es werden 477 mg (1,42 mmol) 2',5'-Didesoxy-5'-fluor-5-(E)-bromvinyluridin zugegeben. Zu der erhaltenen Suspension wird eine Lösung von 700 mg (8,53 mmol, 6 Äq.) N-Methylimidazol in 7 ml THF innerhalb von 30 min zugetropft und anschließend 20 h bei Raumtemperatur gerührt. Der Ansatz wird in ein Gemisch aus 25 ml Phosphatpuffer und 25 ml Essigester gegeben und die wässrige Phase noch zweimal mit je 20 ml Essigester extrahiert. Die vereinte organische Phase wird über Na₂SO₄ getrocknet, filtriert und das Solvens am Rotationsverdampfer entfernt. Säulenchromatographische Reinigung (Dichlormethan /Essigester, 1/1; Essigester) liefert 310 mg (523 µmol, 37 %) [5-(E)-Bromvinyl-5'-fluor-2',5'-didesoxyuridin]-3'-yl-(methoxy-L-alaninyl]-phenyl-phosphat als weißen Feststoff.

¹H-NMR (300 MHz, DMSO-d₆): 1,22 (d, 3H); 1,25* (d, 3H); 2,40-2,59 (m, 4H); 3,61 (s, 3H); 3,62 8s, 3H) ; 3,81-3,98 (m, 2H); 4,19-4,35 (m, 2H); 4,52-4,82 (m, 4H); 5,00-5,12 (m, 2H); 6,13-6,26 (m, 4H); 6,89* (d, 1H); 6,93 (d, 1H); 7,16-7,43 (m, 12H); 7,79* (s, 1H); 7,80 (s, 1H); 11,67* (s, 1H); 11,68 (s, 1H) ppm.

Die Substanz besteht aus einem Diastereomerengemisch (Verhältnis ca. 1,2 : 1). Die mit * gekennzeichneten Signale beziehen sich auf das Unterschussisomer.

³¹P-NMR (122 MHz, DMSO-d₆): 3,91; 4,54 ppm.

### 2.2.3.3. [5'-Azido-5-(E)-bromvinyl-2',5'-didesoxyuridin]-3'-yl-(methoxy-L-

600 mg (2,84 mmol, 2 Äq.) Phenyldichlorophosphat und 397 mg (2,84 mmol, 2 Äq.) L-Alaninmethylester Hydrochlorid werden in 7 ml THF bei -78°C vorgelegt. 576 mg (5,69 mmol, 4 Äq.) Triethylamin werden in 7 ml THF gelöst, innerhalb von 30 min zugetropft und anschließend 20 h bei Raumtemperatur gerührt. Das Reaktionsgemisch wird auf -78°C gekühlt und es werden 509 mg (1,42 mmol) 5'-Azido-2',5'-didesoxy-5-(E)-bromvinyluridin zugegeben. Zu der erhaltenen Suspension wird eine Lösung von 700 mg (8,53 mmol, 6 Äq.) N-Methylimidazol in 7 ml THF innerhalb von 30 min zugetropft und anschließend 20 h bei Raumtemperatur gerührt. Der Ansatz wird in ein Gemisch aus 25 ml Phosphatpuffer und 25 ml Essigester gegeben und die wässrige Phase noch zweimal mit je 20 ml Essigester extrahiert. Die vereinte organische Phase wird über Na₂SO₄ getrocknet, filtriert und das Solvens am Rotationsverdampfer entfernt. Säulenchromatographische Reinigung (Dichlormethan /Essigester, 1/1; Essigester) liefert 160 mg (267 µmol, 19 %) [5'-Azido-5-(E)-bromvinyl-2',5'-didesoxyuridin]-3'-yl-(methoxy-L-alaninyl]-phenyl-phosphat als weißen Feststoff.

¹H-NMR (500 MHz, DMSO-d₆): 1,22 (d, 3H); 1,25* (d, 3H); 2,35-2,58 (m, 4H); 3,57-3,70 (m, 4H); 3,62 (s, 6H); 3,87-3,92 (m, 2H); 4,13-4,18 (m, 2H); 4,94-4,99* (m, 1H); 5,00-5,05 (m, 1H); 6,14-6,22 (m, 4H); 6,90* (d, 1H); 6,93 (d, 1H); 7,16-7,24 (m, 6H); 7,28-7,32 (m, 2H); 7,36-7,42 (m, 4H); 7,88* (s, 1H); 7,89 (s, 1H); 11,69 (s, 2H) ppm.

Die Substanz besteht aus einem Diastereomerengemisch (Verhältnis ca. 1,5 : 1). Die mit * gekennzeichneten Signale beziehen sich auf das Unterschussisomer.

.³¹P-NMR (122 MHz, DMSO-d₆): 3,93; 4,54 ppm.

### 2.3. 5'-Brom-5'-desoxy-5-(E)-bromvinyluridin

### 2.3.1. 2',3'-O-Isopropyliden-5-(E)-bromvinyl-uridin

1,0 g (2,86 mmol) 5-(E)-bromvinyluridin werden in 15 ml Aceton suspendiert. Dazu gibt man 3,13 g (30 mmol) 2,2-Dimethoxypropan und 0,05 g p-Toluolsulfonsäure. Nach 2 h ist die Reaktion vollständig (DC-Kontrolle mit Chloroform/Methanol 20 : 1). Man gibt 0,50 g Kaliumhydrogencarbonat, 20 ml Wasser und 25 ml Essigester zu, trennt die Phasen und extrahiert die wässrige mehrmals mit Essigester. Die vereinigten organischen Phasen werden mit Magnesiumsulfat getrocknet. Nach Abfiltrieren des Trockenmittels und Abdestilieren des Lösungsmittels erfolgt säulenchromatographische Reinigung mit Chloroform/Methanol 30 : 1. Man erhält 0,57 g (51,2 %) eines farblosen Schaums.

### 2.3.2. 2',3'-O-Isopropyliden-5'-brom-5'-desoxy-5-(E)-bromvinyl-uridin

0,57 g (1,46 mmol) 2',3'-O-Isopropyliden-5-(E)-bromvinyl-uridin und 0,81 g (3,1 mmol) Triphenylphosphin werden in 12 ml Pyridin gelöst. Dazu tropft man eine Lösung von 0,92 g (2,77 mmol) Tetrabrommethan in 8 ml Pyridin. Nach 90 min ist der Umsatz vollständig (DC-Kontrolle mit Chloroform/Methanol 30 : 1). Pyridin wird Essigester abdestilliert und der Rückstand säulenchromatographisch mit Dichlormethan/Essigester 12 : 1 gereinigt. Man erhält 380 mg (57,7 %) eines farblosen Schaums.

### 2.3.3. 5'-Brom-5'-desoxy-5-(E)-bromvinyl-uridin

0,38 g (0,84 mmol) 2',3'-O-Isopropyliden-5'-brom-5'-desoxy-5-(E)-bromvinyl-uridin werden in 5 ml Trifluoressigsäure und 1 ml Wasser gelöst und 30 min bei Raumtemperatur gelöst. Der Umsatz ist danach vollständig (DC-Kontrolle mit Chloroform/Methanol 30 : 1). Es wird zur Trockene eingedampft, mehrmals in Methanol aufgenommen und erneut eingedampft. Nach säulenchromatographischer Reinigung erhält man 0,25 g (72,2 %) eines farblosen Feststoffes vom Schmelzpunkt 201 °C.

¹H-NMR (500 MHz, DMSO-d₆): 3,71 (m, 1H); 3,81 (m, 1H); 3,99 (m, 2H); 4,19 (m, 1H); 5,41 (d, 1H); 5,55 (d, 1H); 5,82 (d, 1H); 6,93 (d, 1H); 7,30 (d, 1H); 7,81 (s, 1H); 11,68 (s, 1H) ppm.

### 2.4. 5'- bzw. 3'- Phosphoramidite von BVDU

### 2.4.1. [5-(E)-Bromvinyl-5'-methoxy-2'-desoxyuridin]-3'-yl-(2-cyanoethyl)-diisopropylphosphoramidit

### 2.4.1.1. 3'-O-(t-Butyldimethylsilyl)-5'-methoxy-2'-desoxy-5-(E)-bromvinyluridin

1,33 g (2,97 mmol) 3'-O-(t-Butyldimethylsilyl)-2'-desoxy-5-(E)-bromvinyluridin werden in 17 ml Dioxan und 6 ml Toluol gelöst und 0,88 g (15,6 mmol) KOH sowie 60 µl (3,3 mmol) Wasser zugegeben. Das Reaktionsgemisch wird 2 ½ h bei Raumtemperatur gerührt und anschließend 0,85 g (6,0 mmol) MeI zugeben. Nach weiteren 3 h rühren werden 120 ml Phosphatpufferlösung (pH = 7) zugesetzt und das Gemisch 3 x mit 50 ml Essigester extrahiert. Die vereinte organische Phase wird über MgSO₄ getrocknet, filtriert und das Solvens am Rotationsverdampfer entfernt. Säulenchromatographische Reinigung (Chloroform/Methanol 100/1) liefert 1,04 g (2,25 mmol, 76 %) 3'-O-(t-Butyldimethylsilyl)-5'-methoxy-2'-desoxy-5-(E)-bromvinyluridin als weißen Feststoff.

### 2.4.1.2. 2'-Desoxy-5'-methoxy-5-(E)-bromvinyluridin

2,62 g (5,68 mmol) 3'-O-(t-Butyldimethylsilyl)-5'-methoxy-2'-desoxy-5-(E)-bromvinyluridin werden in 25 ml THF gelöst und 2,69 g (8,52 mmol) Tetrabutylammoniumfluorid in 25 ml THF zugetropft. Das Reaktionsgemisch wird 4 h bei Raumtemperatur gerührt und anschließend das Solvens am Rotationsverdampfer entfernt. Säulenchromatographische Reinigung (Chloroform/Methanol 25/1) liefert 1,73 g (4,98 mmol, 88 %) 2'-Desoxy-5'-methoxy-5-(E)-bromvinyluridin als weißen Feststoff.

### 2.4.1.3. [5-(E)-Bromvinyl-5'-methoxy-2'-desoxy-uridin]-3'-yl-(2-cyanoethyl)-diisopropyl-phosphoramidit

200 mg (574 µmol) 2'-Desoxy-5'-methoxy-5-(E)-bromvinyluridin werden in 5 ml Dichlormethan gelöst und 200 µl (1,15 mmol) Diisopropylethylamin zugegeben. Das Reaktionsgemisch wird auf 0°C gekühlt und dann 192 µl (861 µmol) 2-Cyanoethyl-diisopropylchlorophosphoramidit zugegeben. Nach 3 h bei 0°C wird das Solvens am Rotationsverdampfer entfernt, der Rückstand in 50 ml NaHCO₃-Lsg. aufgenommen und 3 x mit 50 ml Essigester extrahiert. Die vereinte organische Phase wird mit 50 ml ges. NaCl-Lsg. gewaschen, über Na₂SO₄ getrocknet, filtriert und das Solvens am Rotationsverdampfer entfernt. Säulenchromatographische Reinigung (Chloroform/ Methanol 9/1, Cyclohexan/Essigester 1/1) liefert 130 mg (237 µmol, 41 %) [5-(E)-Bromvinyl-5'-methoxy-2'-desoxyuridin]-3'-yl-(2-cyanoethyl)diisopropylphosphoramidit als weißen Feststoff.

¹H-NMR (300 MHz, CDCl₃): 1,19 (d, 12H) ; 2,17-2,24 (m, 1H); 2,41-2,58 (m, 1H); 2,64 (t, 2H); 3,46-3,47 (2x s, 3H); 3,55-3,91 (m, 6H); 4,16-4,23 (m, 1H); 4,57-4,61 (m, 1H); 6,30-6,36 (m, 1H); 6,63 (d, 1H); 7,33 (d, 1H); 7,92-7,94 (2x s, 1H); 8,23 (bs, 1H) ppm.

Die Substanz besteht aus einem Diastereomerengemisch (Verhältnis ca. 1 : 1)

.³¹P-NMR (122 MHz, CDCl₃): 149,45; 149,51 ppm.

### 2.4.2. [5-(E)-Bromvinyl-3'-methoxy-2'-desoxy-uridin]-5'-yl-(2-cyanoethyl)-diisopropyl-phosphoramidit

200 mg (574 µmol) 2'-Desoxy-3'-methoxy-5-(E)-bromvinyluridin werden in 5 ml Dichlormethan gelöst und 200 µl (1,15 mmol) Diisopropylethylamin zugegeben. Das Reaktionsgemisch wird auf 0°C gekühlt und dann 192 µl (861 µmol) 2-Cyanoethyl-diisopropylchlorophosphoramidit zugegeben. Nach 2 h bei 0°C wird das Solvens am Rotationsverdampfer entfernt, der Rückstand in 50 ml NaHCO₃-Lsg. aufgenommen und 3 x mit 50 ml Essigester extrahiert. Die vereinte organische Phase wird mit 50 ml ges. NaCl-Lsg. gewaschen, über Na₂SO₄ getrocknet, filtriert und das Solvens am Rotationsverdampfer entfernt. Säulenchromatographische Reinigung (Chloroform/ Methanol 9/1, Dichlormethan/Essigester 1/1) liefert 120 mg (219 µmol, 38 %) [5-(E)-Bromvinyl-3'-methoxy-2'-desoxyuridin]-5'-yl-(2-cyanoethyl)diisopropylphosphoramidit als weißen Feststoff.

¹H-NMR (300 MHz, CDCl₃) : 1,17-1,26 (m, 12H) ; 1,96-2,17 (m, 1H); 2,45-2,57 (m, 1H); 2,65-2,74 (m, 2H); 3,35-3,37 (2x s, 3H); 3,55-3,65 (m, 2H); 3,81-4,09 (m, 5H); 4,17-4,25 (2x m, 1H); 6,20-6,34 (2x m, 1H); 6,74-6,83 (2x d, 1H); 7,40-7,42 (2x d, 1H); 7,85, 8,10 (2x s, 1H); 8,03 (bs, 1H) ppm.

Die Substanz besteht aus einem Diastereomerengemisch (Verhältnis ca. 1 : 1).

³¹P-NMR (122 MHz, CDCl₃): 150,02; 150,48 ppm.

### 3. 3',5'-Dihalogenderivate von BVDU

### 3.1. 3',5'-Difluor-2',3',5'-tridesoxy-5-(E)-bromvinyl-uridin

### 3.1.1. 5'-O-Trityl-2,3'-anhydro-2'-desoxy-5-ethyl-uridin

17,84 g (35,78 mmol) 5'-O-Trityl-2'-desoxy-5-ethyl-uridin und 14,08 g (53,67 mmol) Triphenylphosphin werden in 170 ml THF gelöst. Zu dieser Lösung wird eine Lösung von 10,85 g (53,67 mmol) Diisopropylazodicarbonester in 120 ml THF innerhalb 40 min zugetropft. Nach 1 h rühren bei Raumtemperatur ist der Umsatz vollständig (DC-Kontrolle mit Chloroform/Methanol 10 : 1). Nach dem Einrotieren erhält man nach säulenchromatographischer Reinigung (Chloroform/Methanol 10 : 1) 13,00 g (75,6 %) eines farblosen Schaums.

### 3.1.2. 1-(5'-O-Trityl-2'-desoxy-β-D-threo-pentofuranosyl)-5-ethyl)-2,4-(1H,3H)-pyrimidindion

14,24 g (29,63 mmol) 5'-O-Trityl-2,3'-anhydro-2'-desoxy-5-ethyl-uridin werden in einem Gemisch aus 500 ml Ethanol und 140 ml Wasser mit 3,03 g (75,85 mmol) Natriumhydroxid 90 min zum Sieden erhitzt. Danach ist die Reaktion beendet (DC-Kontrolle mit Chloroform/Methanol 10 : 1). Die Lösung wird einrotiert, mit Phosphatpuffer und Essigester behandelt und die Phasen getrennt. Die wässrige Phase wird mehrfach mit Essigester extrahiert. Die vereinigten Essigesterphasen werden mit Magnesiumsulfat getrocknet. Nach Filtration und Abrotieren des Lösungsmittels wird das erhaltene Produkt getrocknet und man 13,25 g (90,4 %) eines farblosen Feststoffes.

### 3.1.3. 5'-O-Trityl-3'-fluor-2',3'-didesoxy-5-ethyluridin

7,73 g (15,51 mmol) 1-(5'-O-Trityl-2'-desoxy-β-D-threo-pentofuranosyl)-5-ethyl)-2,4-(1H ,3H)-pyrimidindion werden in 130 ml Dichlormethan gelöst und bei 0 °C wird eine Lösung von 5,0 g (31,02 mmol) Diethylaminoschwefeltrifluorid (DAST) innerhalb 30 min zugetropft. Nach 1 h rühren bei 0 °C ist der Umsatz vollständig (DC-Kontrolle mit Dichlormethan/Methanol 20 : 1). Der Ansatz wird in eiskalte Natriumhydrogen-carbonatlösung (5 %) eingegossen, die Phasen getrennt und die wässrige Phase noch zweimal mit je 50 ml Dichlormethan extrahiert. Die vereinigten organischen Phasen werden mit Magnesiumsulfat getrocknet und nach Filtration und Abrotieren erfolgt säulenchromatographische Reinigung (Dichlormethan/Essigester 5 : 1). Man erhält 5,17 g (66,6 %) eines farblosen Schaums.

### 3.1.4. 3'-Fluor-2',3'-didesoxy-5-ethyl-uridin

4,69 g (9,37 mmol) 5'-O-Trityl-3'-fluor-2',3'-didesoxy-5-ethyl-uridin werden in 70 ml Essigsäure gelöst und 18 ml Wasser langsam zugefügt. Es wird 20 min auf 90 °C erhitzt, danach ist die Reaktion beendet (DC-Kontrolle mit Chloroform/Methanol 15 : 1). Der Ansatz wir 1 h ins Eisbad gestellt, filtriert und der Rückstand mit 80 % Essigsäure gewaschen. Das Filtrat wird mit 400 ml Wasser verdünnt und mehrfach mit Essigester extrahiert. Die vereinigten Extrakte werden mit gesättigter Kochsalzlösung gewaschen und mit Magnesiumsulfat getrocknet. Nach Filtration und Abrotieren des Lösungsmittels erfolgt säulenchromatographische Reinigung (Chloroform/Methanol 15 : 1). Man erhält 1,33 g (55,0 %) eines farblosen Feststoffes.

### 3.1.5. 5'-O-Methylsulfonyl-3'-fluor-2',3'-didesoxy-5-ethyl-uridin

1,33 g (5,15 mmol) 3'-Fluor-2',3'-didesoxy-5-ethyl-uridin werden in 8 ml Pyridin gelöst und dazu wird bei 0 °C eine Lösung von 1,78 g (15,5 mmol) Methylsulfonylchlorid in 2,5 ml THF innerhalb 20 min getropft. Man lässt auf Raumtemperatur aufwärmen. Die Reaktion ist danach beendet (DC-Kontrolle mit Chloroform/Methanol 10 : 1). Der Ansatz wird in Eiswasser eingegossen und 15 min gerührt. Nach Ansäuern (pH ca. 1,5) wird mit Essigester extrahiert und die vereinigten Extrakte mit Phosphatpuffer gewaschen. Nach Trocknen mit Magnesiumsulfat, Filtrieren und Abdestillieren des Lösungsmittels erhält 1,68 g (97,1 %) eines farblosen Schaums.

### 3.1.6. 3',5'-Difluor-2',3',5'-tridesoxy-5-ethyl-uridin

1,68 g (4,99 mmol) 5'-O-Methylsulfonyl-3'-fluor-2',3'-didesoxy-5-ethyl-uridin und 6,30 g (20,00 mmol) Tetrabutylamoniumfluorid Trishydrat werden in 30 ml DMF gelöst und 20 g Molsieb (3 Ǻ) zugesgesetzt. Nach 1,5 h erwärmen auf 40 °C ist die Reaktion beendet (DC-Kontrolle mit Chloroform/Methanol 20 : 1). DMF wird mit Xylol entfernt und der Rückstand in Essigester gelöst. Die Essigesterphase wird mit 1 M Salzsäure und Phosphatpuffer gewaschen. Die vereinigten wässrigen Phasen werden neutralisiert und mit Essigester extrahiert. Alle vereinigten Essigesterphasen werden mit Magnesiumsulfat getrocknet und nach Filtration und Abrotieren des Lösungsmittels mehrfach säulenchromatographisch gereinigt (Chloroform/ Methanol 30 : 1; Chloroform/Essigester 3 : 1). Man erhält 0,52 g (40,0 %) eines farblosen Feststoffes.

### 3.1.7. 3',5'-Difluor-2',3',5'-tridesoxy-5-(E)-bromvinyl-uridin

520 mg (1,99 mmol) 3',5'-Difluor-2',3',5'-tridesoxy-5-ethyl-uridin werden in 15 ml Chloroform gelöst und zum Sieden erhitzt. Es werden 10 mg Azo-bisisobutyronitril (AIBN) zugegeben und eine Lösung von 702 mg Brom in 2 ml Chloroform zugetropft. Während des Zutropfens wird mit einer 500 W Lampe bestrahlt. Nach 40 min ist alles Brom zugegeben und man erhitzt noch 1 h am Rückfluss. Nach dem Abkühlen wird Argon durch die Lösung geleitet und es werden 304 mg Triethylamin zugegeben. Nach 1,5 h rühren bei Raumtemperatur wird mit Phosphatpuffer ausgeschüttelt und die wässrige Phase noch mehrfach mit Essigester extrahiert. Die vereinigten organischen Phasen werden mit Magnesiumsulfat getrocknet. Nach Filtration und Abrotieren des Lösungsmittels erfolgt mehrfache säulenchromatographische Reinigung (Chloroform/Methanol 25 : 1; Chloroform/Essigester 5 : 1) und nachfolgend Umkristallisation aus Ethanol und Methanol/Wasser. Man erhält 211 mg (31, 3 %) eines farblosen Feststoffes vom Schmelzpunkt 177-180 °C.

¹H-NMR (500 MHz, DMSO-d₆) : 2,38 (m, 2H); 4,43 (m, 1H); 4,69 (m, 2H); 5,42 (m, 1H); 6,19 (m, 1H); 6,89 (d, 1H); 7,29 (d, 1H); 7,79 (s, 1H); 11,69 (s, 1H) ppm.

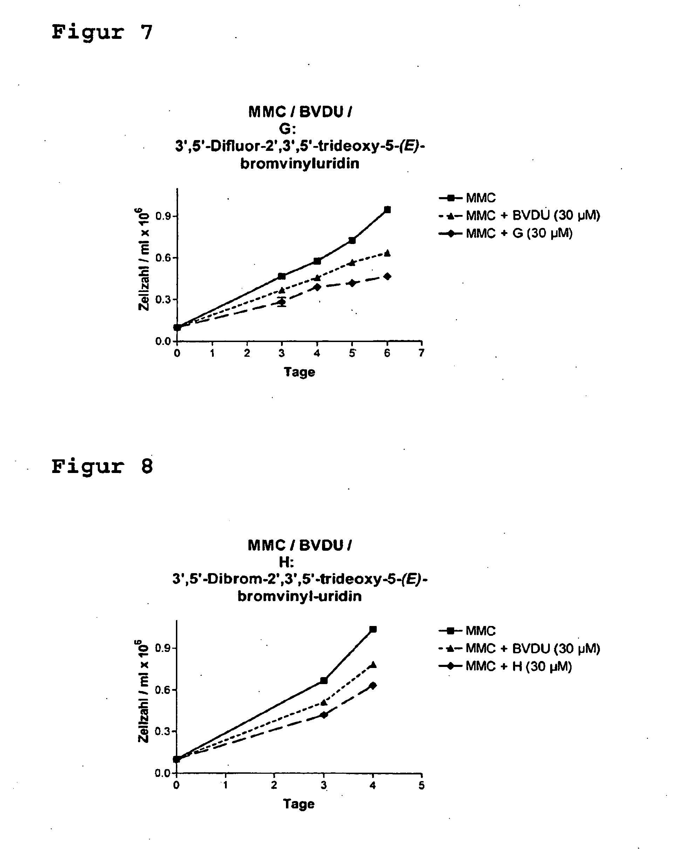

Fig. 7 zeigt die Ergebnisse dieser erfindungsgemäßen Verbindung in Kombination mit Mitomycin C (MMC) im Vergleich zu MMC alleine und MMC in Kombination mit BVDU.

### 3.2. 3'-Fluor-5'-chlor-2',3',5'-tridesoxy-5-(E)-bromvinyluridin

190 mg (0,57 mmol) 3'-Fluor-2',3'-didesoxy-5-(E)-bromvinyl-uridin und 200 mg (0,76 mmol) Triphenyl-phosphin werden in 7 ml DMF gelöst und dazu werden 440 mg (2,83 mmol) Tetrachlormethan gegeben. Nach 18 h rühren bei Raumtemperatur werden 100 mg (0,38 mmol) Triphenylphosphin und 220 mg (1,42 mmol) Tetrachlormethan zugegeben. Nach 22 h ist die Reaktion vollständig (DC-Kontrolle mit Dichlormethan/Essigester 4 : 1). DMF wird durch Destillation mit Xylol entfernt und der Rückstand säulenchromatographisch gereinigt (Dichlormethan/ Essigester 5 : 1). Man erhält 60 mg (30 %) eines kristallinen Feststoffes vom Schmelzpunkt 203 °C.

¹H-NMR (500 MHz, DMSO-d₆): 2,36 (m, 2H); 3,88 (m, 2H); 4,36 (m, 1H); 5,35 (d, 1H); 6,21 (m, 1H); 6,89 (d, 1H); 7,2,8 (d, 1H); 7,85 (s, 1H); 11,69 (s, 1H) ppm.

### 3.3. 3',5'-Dichlor-2',3',5'-tridesoxy-5-(E)-bromvinyluridin

0,70 g (2,0 mmol) 3'-Chlor-2',3'-didesoxy-5-(E)-bromvinyl-uridin und 0,70 g (2,7 mmol) Triphenylphosphin werden in 10 ml DMF gelöst und dazu werden 1,59 g (10,0 mmol) Tetrachlormethan gegeben. Nach 24 h rühren bei Raumtemperatur ist die Reaktion vollständig (DC-Kontrolle mit Dichlormethan/Essigester 4 : 1). DMF wird durch Destillation mit Xylol entfernt und der Rückstand säulenchromatographisch gereinigt (Dichlormethan/ Essigester 4: 1). Man erhält nach Umkristallisation aus n-Hexan/Essigester 180 mg (24,3 %) eines kristallinen Feststoffes vom Schmelzpunkt 184 °C.

¹H-NMR (500MHz, DMSO-d₆): 2,59 (m, 1H); 2,76 (m, 1H); 3,94 (m, 2H); 4,27 (m, 1H); 4,71 (m, 1H); 6,26 (m, 1H); 6,90 (d, 1H); 7,29 (d, 1H); 7,81 (s, 1H); 11,67 (s, 1H) ppm.

### 3.4. 3'-Azido-5'-fluor-2',3,5"-tridesoxy 5-(E)-bromvinyluridin

### 3.4.1. 2',3'-Didesoxy-3'-azido-5'-O-(4-methylphenylsulfonyl)-5-(E)-bromvinyluridin

2,54 g (7,09 mmol) 2',3'-Didesoxy-3'-azido-5-(E)-bromvinyluridin werden bei 0°C in 20 ml Pyridin gelöst und dazu werden 2,56 g (13,42 mmol) 4-Methylphenylsulfonylchlorid gegeben. Man lässt auf Raumtemperatur aufwärmen und rührt 20 h. Die Reaktion ist danach vollständig (DC-Kontrolle mit Chloroform/Methanol 20 : 1). Der Ansatz wird in 80 ml Eiswasser eingegossen und mit 6 M Salzsäure angesäuert. Es wird mit Essigester extrahiert und die vereinigten Extrakte mit gesättigter Kochsalzlösung gewaschen und mit Magnesiumsulfat getrocknet. Nach Filtration und Abrotieren des Lösungsmittels beträgt die Ausbeute 3,32 g (91,5 %) eines farblosen Schaums.

### 3.4.2. 3'-Azido-5'-fluor-2',3,5"-tridesoxy 5-(E)-bromvinyluridin

3,32 g (6,48 mmol) 2',3'-Didesoxy-3'-azido-5'-O-(4-methylphenylsulfonyl)-5-(E)-bromvinyluridin werden in 50 ml DMF gelöst und dazu werden 20 g Molsieb (3 Å) und 8,17 g Tetrabutylammoniumfluorid Trishydrat gegeben. Nach 6 h Erwärmen auf 40 °C ist die Reaktion beendet (DC-Kontrolle mit Cyclohexan/Essigester 1 : 1). DMF wird mit Xylol entfernt und der braune, ölige Rückstand in Essigester gelöst und mit 100 ml 1 M Salzsäure, zweimal mit je 50 ml Phosphatpuffer und mit gesättigter Kochsalzlösung gewaschen. Die wässrigen Phasen werden vereinigt, neutralisiert und mit Essigester extrahiert. Alle vereinigten organischen Phasen werden mit Magnesiumsulfat getrocknet. Nach Filtration und Abrotieren des Lösungsmittels erfolgt säulenchromatographische Reinigung (Dichlormethan/Essigester 5 : 1; Cyclohexan/Essigester 1,2 : 1). Man erhält 0,72 g (19,2 %) eines farblosen Feststoffes vom Schmelzpunkt 135 °C.

¹H-NMR (500 MHz, DMSO-d₆): 2,45 (m, 2H); 4,05 (m, 1H); 4,53 (m, 1H); 4,62 (m, 1H); 4,73 (m, 1H); 6,13 (m, 1H); 6,90 (d, 1H); 7,28 (d, 1H); 7,74 (s, 1H); 11,64 (s, 1H) ppm.

### 3.5. 3'-Chlor-5'-brom-2',3',5'-tridesoxy-5-(E)-bromvinyluridin

### 3.5.1. 2',3'-Didesoxy-3'-chlor-5'-O-methylsulfonyl-5-(E)-bromvinyluridin

1,0 g (2,84 mmol) 3'-Chlor-2',3'-didesoxy-5-(E)-bromvinyl-uridin werden in 5 ml THF/Pyridin (1 : 1) gelöst und bei 0°C werden 1,10 g (9,6 mmol) Methylsulfonylchlorid, gelöst in 5 ml THF, zugetropft. Man lässt auf Raumtemperatur aufwärmen und rührt insgesamt 20 h. Der Umsatz ist danach vollständig (DC-Kontrolle mit Dichlormethan/Methanol 15 : 1). Der Ansatz wird in Eiswasser eingegossen und man lässt 15 min rühren. Es wird mit 2 M Salzsäure angesäuert und das sich abscheidende Öl mit Essigester extrahiert. Die vereinigten Essigesterphasen werden mit Phosphatpuffer gewaschen und mit Magnesiumsulfat getrocknet. Nach Filtration und Abdestillieren des Lösungsmittels erhält man 1,22 g (quantitative Ausbeute) eines farblosen Schaums.

### 3.5.2. 3'-Chlor-5'-brom-2',3',5'-tridesoxy-5-(E)-bromvinyluridin

510 mg (1,18 mmol) 3'-Chlor-5'-O-(methylsulfonyl)-5-(E)-bromvinyl-uridin werden mit 515 mg (6 mmol) Lithiumbromid 3h erhitzt. Danach ist die Reaktion beendet (DC-Kontrolle mit Dichlormethan/Methanol 25 : 1). DMF wird mit Xylol destillativ entfernt und der Rückstand mit Chloroform/Methanol 60 : 1, Dichlormethan/Methanol 40 : 1 und mit Cyclohexan/Essigester 1,2 : 1 säulenchromatographisch gereinigt. Man erhält 240 mg (49,1 %) eines farblosen Feststoffes vom Schmelzpunkt 149 °C.

¹H-NMR (500 MHz, DMSO-d₆): 2,52 (m, 1H) ; 2,76 (m, 1H); 3,73 (m, 1H); 3,81 (m, 1H); 4,27 (m, 1H); 4,68 (m, 1H); 6,27 (m 1H); 6,90 (d, 1H); 7,29 (d, 1H); 7,82 (s, 1H); 11,67 (s, 1H) ppm.

### 3.5.3. 5'-Azido-3'-chlor-2',3'.5'-tridesoxy-5-(E)-bromvinyluridin

3,50 g (8,15 mmol) 3'-Chlor-5'-O-(methylsulfonyl)-2',3'-didesoxy-5-(E)-bromvinyluridin werden mit 1,20 g (24,4 mmol, 3,0 Äq.) Lithiumazid in 20 ml DMF 1 h erhitzt. Das Solvens wird am Rotationsverdampfer entfernt. Säulenchromato-graphische Reinigung (Chloroform/Methanol, 12/1) liefert 2,12 g (5,63 mmol, 69 %) 5'-Azido-3'-chlor-2',3',5'-tridesoxy-5-(E)-bromvinyluridin als weißen Feststoff mit einem Schmelzpunkt von 58 - 60 °C.

¹H-NMR (500 MHz, DMSO-d₆): 2, 58 (m, 1H) ; 2,75 (m, 1H); 3,60 (m, 1H); 3,71 (m, 1H); 4,19 (m, 1H); 4,71 (m, 1H); 6,25 (dd, 1H); 6,93 (d, 1H); 7,30 (d, 1H); 7,86 (s, 1H); 11,69 (s, 1H) ppm.

### 3.5.4. 3'-Azido-5'-chlor-2',3',5'-tridesoxy-5-(E)-bromvinyluridin

### 3.5.4.1. 5'-Chlor-3'-O-(methylsulfonyl)-2',5'-didesoxy-5-(E)-bromvinyluridin

1,00 g (2,84 mmol) 5'-Chlor-2',5'-didesoxy-5-(E)-bromvinyluridin werden in 10 ml Pyridin bei 0°C vorgelegt. 0,34 g (2,99 mmol) Methansulfonsäurechlorid in 5 ml THF werden zugetropft und anschließend 4 h bei Raumtemperatur gerührt. Das Reaktionsgemisch wird auf Eis gegossen, mit konz. Salzsäure auf pH = 5 eingestellt und 3 x mit Essigester extrahiert. Die organische Phase wird mit verd. Salzsäue und ges. NaCl-Lsg. gewaschen, mit Na₂SO₄ getrocknet und das Solvens wird am Rotationsverdampfer entfernt. Es resultieren 1,03 g (2,40 mmol, 84 %) 5'-Chlor-3'-O-(methylsulfo-nyl)-2',5'-didesoxy-5-(E)-bromvinyluridin als weißer Feststoff.

### 3.5.4.2. 2,3'-Anhydro-2'-desoxy-5'-chlor-5-(E)-bromvinyluridin

800 mg (1,86 mmol) 5'-Chlor-3'-O-(methylsulfonyl)-2',5'-didesoxy-5-(E)-bromvinyluridin werden in 10 ml THF vorgelegt. 340 mg (2,23 mmol) 1,8-Diazabicyc-lo[5.4.0]undec-7-en (DBU) werden zugegeben und für 4 h unter Rückfluss erhitzt. Das Solvens wird am Rotationsverdampfer entfernt. Säulenchromato-graphische Reinigung (Chloroform/Methanol, 9/1) liefert 470 mg (1,41 mmol, 76 %) 2,3'-Anhydro-2'-desoxy-5'-chlor-5-(E)-bromvinyluridin als weißen Feststoff.

### 3.5.4.3. 3'-Azido-5'-chlor-2',3',5'-tridesoxy-5-(E)-bromvinyluridin

470 mg (1,41 mmol) 2,3'-Anhydro-5'-chlor-2'-desoxy-5-(E)-bromvinyluridin werden in 10 ml DMF vorgelegt. 345 mg (7,05 mmol) LiN₃ werden zugegeben und das Reaktionsgemisch für 20 h erhitzt. Das Solvens wird am Rotationsverdampfer entfernt. Das Reaktionsgemisch wird in 50 ml Essigester aufgenommen und die organische Phase wird mit NaCl-Lsg. gewaschen. Die wässrige Phase wird 3 x mit Essigester extrahiert. Die vereinte organische Phase wird mit ges. NaCl-Lsg. gewaschen, mit Na₂SO₄ getrocknet und das Solvens wird am Rotationsverdampfer entfernt. Säulenchromatographische Reinigung (Chloroform/Methanol, 9/1) liefert 370 mg (982 µmol, 70 %) 3'-Azido-5'-chlor-2',3',5'-tridesoxy-5-(E)-bromvinyluridin als weißen Feststoff mit einem Schmelzpunkt von 124-125°C.

¹H-NMR (300 MHz, CDCl₃): 2,33-2,57 (m, 2H); 3,58-3,66 (m, 1H); 3,80-3,87 (m, 1H); 3,96-4,02 (m, 1H); 4,21-4,28 (m, 1H); 6,14 (t, 1H); 6,68 (d, 1H); 7,42 (d, 1H); 7,54 (s, 1H); 8,39 (bs, 1H).

### 3.6. 3',5'-Dibrom-2',3',5'-tridesoxy-5-(E)-bromvinyluridin

0,52 g (1,31 mmol) 2',3'-Didesoxy-3'-brom-5-(E)-bromvinyluridin werden in 15 ml Pyridin gelöst und dazu werden 0,69 g (2,62 mmol) Triphenylphosphin gegeben. Zu dieser Lösung tropft man eine Lösung von 0,79 g (2,36 mmol) Tetrabrommethan in 5 ml Pyridin innerhalb 10 min. Nach 1 h ist die Reaktion beendet (DC-Kontrolle mit Chloroform/Methanol 20 : 1). Man gießt auf Eis und säuert mit 37 % Salzsäure an. Es folgt Extraktion mit Essigester (3 x 40 ml), waschen der vereinigten Essigesterphasen mit 1 M Salzsäure und mit gesättigter Kochsalzlösung. Nach Trocknung mit Magnesiumsulfat und Filtration wird die Rohsubstanz säulenchromatographisch gereinigt (Dichlormethan/Essigester 4 : 1) und aus Cyclohexan/Essigester umgefällt. Man erhält nach Trocknung 0,21 g (35,0 %) eines farblosen Feststoffes vom Schmelzpunkt 153 °C.

¹H-NMR (500 MHz, DMSO-d₆): 2,68 (m, 1H); 2,81 (m, 1H); 3,74 (m, 1H) ; 3,82 (m, 1H); 4,38 (m, 1H); 4,65 (m, 1H); 6,27 (m, 1H); 6,91 (d, 1H); 7,29 (d, 1H); 7,82 (s, 1H); 11,66 (s, 1H) ppm.

Fig. 8 zeigt die Ergebnisse dieser erfindungsgemäßen Verbindung in Kombination mit Mitomycin C (MMC) im Vergleich zu MMC alleine und MMC in Kombination mit BVDU.

### 4. 2',3'-Didehydro-2',3',5'-tridesoxy-5'-brom-5-(E)-bromvinyl-uridin als Referenzverbindungen

### 4.1. 2',3'-Didehydro-2',3'-didesoxy-5-(E)-bromvinyl-uridin

### 4.1.1. 1-(2'-Desoxy-β-D-threo-pentofuranosyl)-5-(E)-(2-bromvinyl)-2,4(1H, 3H)-pyrimidindion

20,07 g (47,87 mmol) 2,3'-Anhydro-2'-desoxy-5'-O-benzoyl-5-(E)-bromvinyluridin werden in 400 ml Ethanol suspendiert und mit einer Lösung von 4,78 g (120 mmol) Natriumhydroxid in 95 ml Wasser versetzt. Es wird 2,5 h zum Sieden erhitzt, danach ist die Reaktion beendet (DC-Kontrolle mit Dichlormethan/Methanol 10 : 1). Nach Neutralisation mit Salzsäure wird das Lösungsmittel bis zur Trockne abdestilliert und der Rückstand mehrfach mit Aceton/Essigester-Gemisch (1 : 1) extrahiert. Es werden 13,5 g (84,6 %) Produkt erhalten

### 4.1.2. 5'-O-Benzoyl-1-(2'-desoxy-β-D-threo-pentofuranosyl)-5-(E)-(2-bromvinyl)-2,4(1H, 3H)-pyrimidindion

5,0 g (15,00 mmol) 1-(2'-Desoxy-β-D-threo-pentofuranosyl)-5-(E)-(2-bromvinyl)-2,4(1H, 3H)-pyrimidindion werden in 125 ml Pyridin gelöst und auf 0 °C gekühlt. Dazu tropft man bei dieser Temperatur eine Lösung con 2,53 g (18,0 mmol) Benzoylchlorid in 30 ml Pyridin. Nach 60 min ist das Zutropfen beendet. Man lässt noch 60 min bei 0 °C rühren. Danach ist die Reaktion beendet (DC-Kontrolle mit Dichlormethan/Methanol 20 : 1). Der Ansatz wird in 400 ml Eiswasser eingegossen, mit 4 x 100 ml Essigester extrahiert und die vereinigten Extrakte werden mit 5 % Kaliumhydrogencarbonat und gesättigter Kochsalzlösung gewaschen. Nach Trocknung mit Magnesiumsulfat, abfiltrieren und abdestillieren des Lösungsmittels erfolgt säulenchromatographische Reinigung (Chloroform/Methanol 20 : 1). Man erhält 5,52 g (84,1 %) eines farblosen Feststoffes.

### 4.1.3. 3'-O-(Methylsulfonyl)-5'-O-benzoyl-1-(2'-Desoxy-β-D-threo-pentofuranosyl)-5-(E)-(2-bromvinyl)-2,4(1H, 3H)-pyrimidindion

3,50 g (8,00 mmol) 5'-O-Benzoyl-1-(2'-desoxy-β-D-threo-pentofuranosyl)-5-(E)-(2-bromvinyl)-2,4(1H, 3H)-pyrimidindion werden in 15 ml Pyridin gelöst und bei 0 °C mit 1,14 g (10,00 mmol) Methansulfonsäurechlorid versetzt. Nach 18 h ist die Reaktion beendet (DC-Kontrolle mit Chloroform/Methanol 12 : 1). Danach wird der Ansatz auf Eis gegossen und 20 min stehen gelassen. Man säuert mit 6 M Salzsäure an und extrahiert mit Essigester (3 x 50 ml). Die vereinigten Extrakte werden neutral gewaschen und mit Magnesiumsulfat getrocknet. Nach Abfiltrieren und abrotieren des Lösungsmittels erhält man 3,95 g (95,8 %) eines gelblichen Schaums.

### 4.1.4. 5'-O-Benzoyl-2',3'-didehydro-2',3'-didesoxy-5-(E)-bromvinyl-uridin

3,38 g (6,56 mmol) 3'-O-(Methylsulfonyl)-5'-O-benzoyl-1-(2'-Desoxy-β-D-threo-pentofuranosyl)-5-(E)-(2-bromvinyl)-2,4(1H, 3H)-pyrimidindion werden in 120 ml THF gelöst. Dazu gibt man 6,20 g (19,68 mmol) Tetra-n-butylamoniumfluorid Trishydrat und 25,0 g Molsieb (3 Ǻ) und lässt 20 h bei Raumtemperatur rühren. Die Reaktion ist danach vollständig (DC-Kontrolle mit Dichlormethan/Essigester 3 : 1). Es wird über Celite abfiltriert, mit Essigester gründlich gewaschen, auf 80 ml eingeengt und mit 3 x 80 ml gesättigter Kochsalzlösung gewaschen. Nach Trocknung mit Magnesiumsulfat, abfiltrieren und säulenchromatographischer Reinigung erhält man 2,20 g (80,0 %) eines farblosen Feststoffes.

### 4.1.5. 2',3'-Didehydro-2',3'-didesoxy-5-(E)-bromvinyl-uridin

2,47 g (5,89 mmol) 5'-O-Benzoyl-2',3'-didehydro-2',3'-didesoxy-5-(E)-bromvinyl-uridin werden in 70 ml THF suspendiert und mit 8,8 ml (17,6 mmol) 2 M Natronlauge versetzt. Nach 5 h ist der Umsatz vollständig (DC-Kontrolle mit Dichlormethan/Methanol 20 : 1). Der Ansatz wird in 100 ml Phospatpufferlösung (pH = 7) eingegossen und mit 5 x 50 ml Essigester extrahiert. Nach Trocknung mit Magnesiumsulfat und Abdestillieren des Lösungsmittels erhält man nach säulenchromatographischer Reinigung mit Chloroform/Aceton 3 : 1 1,73 g (93,0 %) eines farblosen Feststoffes vom Schmelzpunkt > 250 °C (Zersetzung).

¹H-NMR (500 MHz, DMSO-d₆): 3,63 (m, 2H); 4,81 (m, 1H); 5,09 (t, 1H); 5,92 (m, 1H); 6,42 (m, 1H); 6,75 (d, 1H); 6,82 (m, 1H); 7,17 (d, 1H); 8,04 (s, 1H); 11,59 (s, 1H) ppm.

### 4.2. 2',3'-Didehydro-2',3',5'-tridesoxy-5'-brom-5-(E)-bromvinyl-uridin

### 4.2.1. 5'-O-(4-Methylphenylsulfonyl)-2',3'-didehydro-2',3'-didesoxy-5-(E)-bromvinyluridin

0,55 g (1,75 mmol) 2',3'-Didehydro-2',3'-didesoxy-5-(E)-bromvinyl-uridin werden in 5 ml Pyridin gelöst und bei 0 °C mit 0,67 g (3,5 mmol) 4-Methylphenylsulfonylchlorid versetzt. Nach 20 h ist der Umsatz vollständig (DC-Kontrolle mit Chloroform/Methanol 20 : 1). Man gießt auf Eis, lässt 20 min rühren und filtriert. Nach gründlichem Waschen mit Wasser wird über Calciumchlorid getrocknet. Nach säulenchromatographischer Reinigung mit Cyclohexan/Aceton 2 :1 erhält man 0,65 g (79,4 %) eines farblosen Feststofffes.

### 4.2.2. 2',3'-Didehydro-2',3',5'-tridesoxy-5'-brom-5-(E)-bromvinyl-uridin

0,65 g (1,39 mmol) 5'-O-(4-Methylphenylsulfonyl)-2',3'-didehydro-2',3'-didesoxy-5-(E)-bromvinyl-uridin und 0,87 g (10,00 mmol) Lithiumbromid werden in 10 ml DMF gelöst und auf 50 °C erwärmt. Nach 3,5 h gibt man noch 400 mg (4,6 mmol) Lithiumbromid hinzu und nach weiteren 60 min ist die Umsetzung vollständig. Man gießt den Ansatz in 40 ml Wasser ein, filtriert den ausgefallenen Niederschlag ab und trocknet über Calciumchlorid. Nach säulenchromatographischer Reinigung mit Cyclohexan/Aceton 2 : 1 erhält man 510 mg (97,3 %) eines farblosen Feststoffes vom Schmelzpunkt > 150 °C.

¹H-NMR (500 MHz, DMSO-d₆): 3,75 (m, 2H); 5,03 (m, 1H); 6,06 (m, 1H); 6,47 (m, 1H); 6,82 (m, 1H); 6,96 (d, 1H); 7,29 (d, 1H); 7,65 (s, 1H); 11,68 (s, 1H) ppm.

### 5. 2'-Fluorderivate von BVDU

### 5.1. 2'-Fluor-2'-desoxy-5-(E)-bromvinyl-uridin

### 5.1.1. 3-(2'-Desoxy-2'-fluorouridin-5-yl)-acrylsäure

2,2 g (6,66 mmol) 3-(2'-Desoxy-2'-fluorouridin-5-yl)-acrylsäuremethylester werden in 20 ml (40 mmol) 2 M Natronlauge gelöst. Nach 3 h wird mit 12 M Salzsäure angesäuert und eine Stunde im Eisbad gekühlt. Nach Filtration, waschen mit Eiswasser und Trocknen im Exsikkator erhält man 1,31 g (62,1 %) eines farblosen Feststoffes.

### 5.1.2. 2'-Fluor-2'-desoxy-5-(E)-bromvinyl-uridin

1,31 g (3,97 mmol) 3-(2'-Desoxy-2'-fluorouridin-5-yl)-acrylsäure werden in 40 ml DMF gelöst und danach werden 1,58 g (16,04 mmol) Kaliumhydrogencarbonat zugesetzt. Nach 20 min rühren bei Raumtemperatur werden 0,86 g (4,81 mmol) N-Bromsuccinimid, gelöst in 20 ml DMF, innerhalb 30 min zugetropft. Nach 4 h rühren bei Raumtemperatur wird abfiltriert, der Rückstand mit DMF gewaschen und DMF mit Xylol abdestilliert. Der Rückstand wird mit Chloroform/Methanol 9 : 1 säulenchromatographisch gereinigt. Nach digerieren in Diethylether und Trocknen erhält man 0,84 g (62,7 %) eines farblosen Produktes mit Schmelzpunkt 182 °C.

¹H-NMR (500 MHz, DMSO-d₆): 3,63 (m, 1H); 3,85 (m, 1H); 3,89 (m, 1H); 4,17 (m, 1H); 5,04 (dd, 1H); 5,35 (t, 1H); 5,61 (d, 1H); 5,88 (d, 1H); 6,75 (d, 1H); 7,21 (d, 1H); 8,19 (s, 1H); 11,65 (s, 1H) ppm.

### 5.2. 2'-Fluor-5'-brom-2',5'-didesoxy-5-(E)-bromvinyl-uridin

0,25 g (0,71 mmol) 2'-Fluor-2'-desoxy-5-(E)-brom-vinyluridin und 0,37 g (1,42 mmol) Triphenylphosphin werden in 10 ml Pyridin gelöst. 0,42 g (1,28 mmol) Tetrabrommethan werden in 5 ml Pyridin gelöst und innerhalb 5 min zugetropft. Nach 1 h ist die Reaktion beendet (DC-Kontrolle mit Chloroform/Methanol 10: l). Nach Entfernen des Pyridins mit Toluol erfolgt säulenchromatographische Reinigung mit Chloroform/Methanol 30 : 1. Man erhält 0.23 g (78 %) eines farblosen Feststoffes vom Schmelzpunkt 223 °C.

¹H-NMR (500 MHz, DMSO-d₆): 3,78 (m, 1H) ; 3,85 (m, 1H); 4,0 (m, 1H); 4,13 (m, 1H); 5,19 (dd, 1H); 5,87 (d, 1H); 5,91 (dd, 1H); 6,89 (d, 1H); 7,29 (d, 1H); 7,77 (s, 1H); 11,72 (s, 1H) ppm.

### 5.3. 5-(E)-2-Bromvinyl)-1-(2'-desoxy-2'-fluor-β-D-arabinofuranosyl)uracil

### 5.3.1. 5-Iod-1-(2'-desoxy-2'-fluor-3',5'-di-O-benzo-yl-β-D-arabinofuranosyl)uracil

7,08 g (29,75 mmol) 5-Ioduracil werden in 105 ml 1,2-Dichlorethan suspendiert. Dazu gibt man 13,32 g (65,47 mmol) N,O-Bis-trimethylsilylacetamid (BSA) und rührt 5 h bei Raumtemperatur. Es entsteht eine klare, farblose Lösung. Dazu gibt man 11,40 g ( 26,9 mmol) 3,5-Di-O-benzoyl-2-desoxy-2-fluoro-α-D-arabinosyl-bromid. Es wird 22 h am Rückfluss erhitzt. Der Umsatz ist danach vollständig (DC-Kontrolle mit Chloroform/Methanol 100 : 1). Der Ansatz wird mit 450 ml Essigester verdünnt und mit 400 ml Phosphatpuffer (pH = 7) behandelt. Die organische Phase wird abgetrennt und die wässrige mit 3 x150 ml Essigester extrahiert. Nach Trocknen der vereinigten organischen Phasen mit Magnesiumsulfat, abfiltrieren des Trockenmittels und abdestillieren des Lösungsmittels wird aus Isopropylalkohol umkristallisiert. Man 8,47 g (54,3 %) eines farblosen Produktes.

### 5.3.2. 5-Iod-1-(2'-desoxy-2'-fluor-β-D-arabinofurano-syl)uracil

8,14 g (14,03 mmol) 5-Iod-1-(2'-desoxy-2'-fluor-3',5'-di-O-benzoyl-β-D-arabino-furanosyl)uracil werden in 250 ml THF gelöst und mit 50 ml (100 mmol) 2 M Natronlauge versetzt. Nach 4 h ist die Reaktion beendet. Die Lösung wird neutralisiert und mit Essigester mehrfach extrahiert. Die vereinigten Extrakte werden mit Magnesiumsulfat getrocknet. Nach Filtration und abrotieren des Lösungsmittels erfolgt säulenchromatographische Reinigung (Chloroform/Methanol 10 : 1). Man erhält 4,80 g (91,8 %) eines farblosen Feststoffes.

### 5.3.3. 3-((2'-desoxy-2'-fluor-β-D-arabinofurano-syl)uracil-5-yl)-acrylsäuremethylester

0,75 g (2,84 mmol) Triphenylphosphin, 0,34 g (1,52 mmol) Palladium(II)acetat und 6,17 g (61 mmol) Triethylamin werden in 160 ml 1,4-Dioxan auf 80 °C erhitzt. Es tritt eine tief dunkelrote Färbung auf. Man erwärmt 10 min bei dieser Temperatur und fügt dann 6,54 g (76 mmol) Acrylsäuremethylester, 4,80 g (12,9 mmol) S-Iod-1-(2'-desoxy-2'-fluor-β-D-arabino-furanosyl)uracil und 40 ml 1,4-Dioxan hinzu. Nach 1 h Erhitzen am Rückfluss ist der Umsatz vollständig (DC-Kontrolle mit Chloroform/Methanol 9 : 1). Nach dem Abkühlen wird über Celite von Ungelöstem abfiltriert und mit THF gründlich nachgewaschen, bis das Filtrat farblos abläuft. Das Filtrat wird abrotiert und der ölige Rückstand wird mit Chloroform/Methanol 10 : 1 säulenchromatographisch gereinigt. Man erhält 2,72 g (54,4 %) eines farblosen Feststoffes.

### 5.3.4. 3-((2'-desoxy-2'-fluor-β-D-arabinofurano-syl)uracil-5-yl)-acrylsäure

2,72 g (8,25 mmol) 3-((2'-desoxy-2'-fluor-β-D-arabinofuranosyl)uracil-5-yl)-acrylsäure-methylester werden in 40 ml (80 mmol) 2 M Natronlauge gelöst. Nach 4 h ist die Reaktion beendet (DC-Kontrolle mit Chloroform/Methanol 8 : 1). Man säuert mit 12 M Salzsäure an und lässt 1 h im Eisbad stehen. Der Niederschlag wird abgesaugt, mit Eiswasser gewaschen und im Exsikkator getrocknet. Man erhält 2,61 g (100 %) eines farblosen Produktes.

### 5.3.5. 5-(E)-(2-Bromvinyl)-1-(2'-desoxy-2'-fluor-β-D-arabinofuranosyl)uracil

2,61 g (7,9 mmol) 3-((2'-desoxy-2'-fluor-β-D-arabinofuranosyl)uracil-5-yl)-acrylsäure werden in 60 ml DMF gelöst und mit 3,2 g (32 mmol) Kaliumhydrogencarbonat versetzt. Man rührt 30 min bei Raumtemperatur und tropft danach eine Lösung von 1,78 g (10 mmol) in 30 ml DMF innerhalb 50 min zu. Nach weiteren 3,5 h rühren bei Raumtemperatur wird von Ungelöstem abfiltriert, der Rückstand mit Aceton/Methanol 1 : 1 gründlich gewaschen und einrotiert. DMF wird durch Destillation mit Xylol entfernt. Nach säulenchromatographischer Reinigung mit Chloroform/Methanol 9 : 1 und Dichlormethan/Essigester 1 : 2 erhält man 1,51 g (54,5 %) eines farblosen Feststoffes vom Schmelzpunkt 202 °C.

¹H-NMR (500 MHz, DMSO-d₆): 3,62 (m, 1H) ; 3, 65 (m, 1H); 3,81 (m, 1H); 4,25 (dq, 1H); 5,06 (dt, 1H); 5,14 (t, 1H); 5,89 (d, 1H); 6,11 (dd, 1H); 6,88 (d, 1H); 7,27 (d, 1H); 7,99 (s, 1H); 11,75 (s, 1H).

### 5.4. 5-(E)-(2-Bromvinyl)-1-(2',5'-didesoxy-2'-fluor-5'-brom-β-D-arabinofuranosyl)-uracil

0,60 g (1,71 mmol) 5-(E)-(2-Bromvinyl)-1-(2'-desoxy-2'-fluor-β-D-arabinofuranosyl)-uracil und 950 mg (3,62 mmol) Triphenylphosphin werden in 10 ml Pyridin gelöst. Danach tropft man eine Lösung von 1,08 g (3,25 mmol) Tetrabrommethan in 10 ml Pyridin innerhalb von 10 min hinzu. Nach 1,5 h ist die Umsetzung vollständig (DC-Kontrolle mit Chloroform/Methanol 10 : 1). Pyridin wird mit Toluol abdestilliert und der Rückstand mit Chloroform/Methanol 30 : 1 säulenchromatographisch gereinigt. Man erhält 0,63 g (89,0 %) eines farblosen Produktes vom Schmelzpunkt 237 °C.

¹H-NMR (300 MHz, DMSO-d₆): 3,79 (m, 2H);4,07 (m, 1H); 4,26 (dq, 1H); 5,09 (ddd, 1H), 6,16 (d, 1H); 6,23 (dd, 1H); 6,98 (d, 1H); 7,30 (d, 1H); 7,74 (d, 1H); 11,80 (s, 1H) ppm.

### 6. 2',2'-Difluorderivate von BVDU als Referenzverbindungen

### 6.1. 1-(2'-Desoxy-2',2'-difluoro-α-D-erythro-pentofuranos-1'-yl)-5-(E)-bromvinyl-uracil und 1-(2'-Desoxy-2'-2'-difluoro-β-D-erythro-pentofuranos-1'-yl)-5-(E)-bromvinyl-uracil

### 6.1.1. 1-(3',5'-Di-O-benzoyl-2',2'-difluoro-α-D-pentofuranos-1'-yl)-5-(E)-bromvinyl-uracil und 1-(3',5'-Di-O-benzoyl-2',2'-difluoro-β-D-pentofuranos-1'-yl)-5-(E)-bromvinyl-uracil

1,89 g (8,70 mmol) 5-(E)-Bromvinyluracil werden in 40 ml 1,2-Dichlorethan suspendiert und man gibt 4,28 g (19,23 mmol) Trimethylsilyltrifluoromethansulfonat und 2,14 g (177 mmol) 2,4,6-Collidin hinzu. Nach 1 h erhält man eine klare Lösung. 3,98 g (8,7 mmol) 2-Desoxy-2,2-difluoro-D-ribofuranose-3,5-dibenzoat-1-methansulfonat (α/β-Gemisch) werden in 25 ml 1,2-Dichlorethan gelöst und innerhalb 20 min zugetropft. Danach tropft man eine Lösung von 1,94 g (8,7 mmol) Trimethylsilyltrifluoromethansulfonat in 15 ml 1,2-Dichlorethan innerhalb 10 min zu und erhitzt 18 h am Rückfluss. DC-Kontrolle (Chloroform/Methanol 10 : 1 und 100 : 1) ergibt, dass der Umsatz vollständig ist. Das Lösungsmittel wird abdestilliert und der ölige Rückstand in 80 ml Essigester gelöst. Nach Waschen mit 2 x 80 ml 1 M Salzsäure, 2 x 80 ml gesättigter Kochsalzlösung und mit 1 x 80 ml Phosphatpuffer erfolgt Trocknung mit Magnesiumsulfat. Nach Filtration und abrotieren des Lösungsmittels wird die Rohsubstanz säulenchromatographisch mit Chloroform/Methanol 125 : 1 gereinigt. Man erhält 3,1 g (61,7 %) eines farblosen Schaums (α/β = 1,2).

Die Trennung der α- und β-Anomeren erfolgt durch wiederholte Säulenchromatogra-phie mit Cyclohexan/Essigester 3 : 1.

Man erhält 1,49 g α-Anomer und 1,15 g β-Anomer.

### 6.1.2. 1-(2'-Desoxy-2',2'-difluoro-α-D-erythro-pentofuranos-1'-yl)-5-(E)-bromvinyl-uracil

1,49 g (2,58 mmol) 1-(3',5'-Di-O-benzoyl-2',2'-difluoro-α-D-pentofuranos-1'-yl)-5-(E)-bromvinyl-uracil werden in 55 ml THF gelöst und 13 ml (26 mmol) 2 M Natronlauge zugegeben. Nach 3h wird neutralisiert und mit Essigester extrahiert. Nach Trocknung mit Magnesiumsulfat, Filtration und Abrotieren des Lösungsmittels erfolgt säulenchromatographische Reinigung mit Chloroform/Methanol 20 :1 und Dichlor-methan/Methanol 20 : 1. Nach Trocknung erhält man 0,82 g (86 %) eines farblosen Feststoffes vom Schmelzpunkt 192 °C.

¹H-NMR (500 MHz, DMSO-d₆): 3,56 (m, 1H); 3,65 (m, 1H); 4,35 (m, 2H); 5,08 (s, 1H); 6,22 (m, 1H); 6,34 (d, 1H); 7,02 (d, 1H); 7,32 (d, 1H) ; 7,85 (s, 1H); 11,81 (s, 1H) ppm.

### 6.1.3. 1-(2'-Desoxy-2',2'-difluoro-β-D-erythro-pentofuranos-1'-yl)-5-(E)-bromvinyl-uracil

1,15 g (1,99 mmol) 1-(3',5'-Di-O-benzoyl-2',2'-difluoro-β-D-pentofuranos-1'-yl)-5-(E)-bromvinyluracil werden in 55 ml THF gelöst und mit 10 ml (20 mmol) Natronlauge versetzt. Die Aufarbeitung und säulenchromatographische Reinigung erfolgt analog wie vorstehend für das α-Anomer beschrieben. Man erhält 740 mg (100 %) eines farblosen Feststoffes mit Schmelzpunkt 189 °C.

¹H-NMR (500 MHz, DMSO-d₆): 3,64 (m, 1H); 3,81 (m, 1H); 3,87 (m, 1H); 4,22 (m, 1H); 5,36 (t, 1H); 6,06 (t, 1H); 6,33 (d, 1H); 6, 83 (d, 1H); 7, 27 (d, 1H); 8,05 (s, 1H); 11,86 (s, 1H) ppm.

### 6.1.4. 1-(5'-Chlor-2',5'-didesoxy-2',2'-difluoro-α-D-erythro-pentofuranos-1'-yl)-5-(E)-bromvinyl-uracil

350 mg (0,95 mmol) 1-(2'-Desoxy-2',2'-difluoro-α-D-erythro-pentofuranos-1'-yl)-5-(E)-bromvinyl-uracil werden in 8 ml DMF gelöst. Man gibt 337 mg (1,29 mmol) Triphenylphosphin hinzu und danach 729 mg (4,74 mmol) Tetrachlormethan. Nach 20 h rühren bei Raumtemperatur ist der Umsatz vollständig (DC-Kontrolle mit Chloroform/Methanol 15 : 1). Nach Zugabe von 2 ml Methanol wird DMF mit Xylol entfernt und der braune Rückstand mit Chloroform/Methanol 30 : i säulenchromatographisch mehrfach gereinigt. Man erhält 240 mg (65,2 %) eines farblosen Produktes vom Schmelzpunkt 208 °C.

¹H-NMR (500MHz, DMSO-d₆) : 3,87 (m, 1H); 3,94 (m, 1H); 4,42 (m, 1H); 4,61 (m, 1H); 6,31 (m, 1H); 6,61 (s (br.), 1H); 6,99 (d, 1H); 7,33 (d, 1H); 7,89 (d, 1H); 11,82 (s (br.), 1H) ppm.

### 6.1.5. 1-(5'-Chlor-2',5'-didesoxy-2',2'-difluoro-β-D-erythro-pentofuranos-1'-yl)-5-(E)-bromvinyl-uracil

520 mg (1,41 mmol) 1-(2'-Desoxy-2',2'-difluoro-β-D-erythro-pentofuranos-1'-yl)-5-(E)-bromvinyl-uracil, 500 mg (1,90 mmol) Triphenylphophin werden mit 1083mg (7,04 mmol) Tetrachlormethan analog wie vorstehend beschrieben umgesetzt und aufgearbeitet. Man erhält nach mehrfacher säulenchromatographischer Reinigung mit Chloroform/Methanol 20 : 1 460 mg (84,1 %) eines farblosen Produktes mit Schmelzpunkt 191 °C.

¹H-NMR (500MHz, DMSO-d₆): 3,99 (m, 2H); 4,05 (m, 1H) ; 4,25 (m, 1H); 6,17 (t, 1H); 6,57 (d, 1H); 6,93 (d, 1H); 7,33 (d, 1H); 7,76 (s, 1H); 11,90 (s, 1H) ppm.

### 7. 1-(β-D-Arabinofuranosyl)-5-(E)-bromvinyl-uracil als Referenzverbindung

### 7.1. 3-(1-(2,3,6-Tri-O-acetyl-β-D-arabinofuranosyl)-uracil-5-yl)-acrylsäuremethylester

0,84 g (3,2 mmol) Palladium(II)acetat, 0,89 g (3,38 mmol) Triphenylphosphin und 7,35 g (73,56 mmol) Triethylamin werden in 135 ml Dioxan auf 70 °C erhitzt. Es entsteht eine tief dunkelrot gefärbte Lösung. Man erwärmt 10 min bei dieser Temperatur, gibt zuerst 22,74 g (264 mmol) Acrylsäuremethylester und danach 9,0 g (18,13 mmol) 1-(2,3,6-Tri-O-acetyl-β-D-arabino-furanosyl)-5-ioduracil sowie 90 ml Dioxan hinzu. Nach 2,5 h Erhitzen am Rückfluss ist die Reaktion beendet (DC-Kontrolle mit Dichlormethan/Essigester 5 : 2). Nach dem Abkühlen wird mit Essigester verdünnt, von Ungelöstem abfiltriert und das Filtrat eingedampft. Der Rückstand wird säulenchromatographisch gereinigt (Dichlormethan/Essigester 5 : 2). Man erhält 6,3 g (76,5 %)eines farblosen Schaums.

### 7.2.3-(1-(β-D-arabinofuranosyl)-uracil-5-yl)-acrylsäure

6,3 g (13,86 mmol) 3-(1-(2,3,6-Tri-O-acetyl-β-D-arabinofuranosyl)-uracil-5-yl)-acrylsäuremethylester werden in 140 ml THF gelöst und mit 140 ml (280 mmol) 2 M Natronlauge versetzt. Nach 5 h wird die Lösung eingeengt und mit 12 M Salzsäure angesäuert. Der Ansatz wird 1 h im Eisbad aufbewahrt und der Niederschlag abgesaugt und mit Eiswasser gewaschen. Nach Trocknen im Exsikkator erhält man 3,45 g (79,4 %) eines farblosen Feststoffes.

### 7.3. 1-(β-D-Arabinofuranosyl)-5-(E)-bromvinyl-uracil

3,46 g (11,01 mmol) 3-(1-(β-D-arabinofuranosyl)-uracil-5-yl)-acrylsäure werden 100 ml DMF gelöst und es werden 5,51 g (55,05 mmol) Kaliumhydrogencarbonat zugegeben. Nach 30 min rühren bei RT wird eine Lösung von 2,37 g (13,4 mmol) N-Bromsuccinimid innerhalb 1 h zugetropft. Nach 4,5 h rühren bei Raumtemperatur wird DMF mit Xylol entfernt und der Rückstand mit Dichlormethan/Methanol 10 : 1 säulenchromatographisch gereinigt. Nach Umkristallisation aus Methanol erhält man ein farbloses, kristallines Produkt (0,84 g (21,8 %).

¹H-NMR (500MHz, DMSO-d₆): 3,63 (m, 2H); 3,74 (m, 1H); 3,91 (m, 1H); 4,03 (m, 1H); 5,11 (t, 1H); 5,45 (d, 1H); 5,55 (d, 1H); 5,97 (d, 1H); 6,87 (d, 1H); 7,24 (d, 1H); 7,89 (s, 1H); 11,57 (s, 1H) ppm.

### 8. 5'-Desoxy-BVDU und 3',5'-Didesoxy-3'-halogen-BVDU als Referenzverbindung

### 8.1. 5'-Desoxy-BVDU

### 8.1.1. 2',5'-Didesoxy-5'-iod-5-ethyl-uridin

8,0 g (31,22 mmol) 2'-Desoxy-5-ethyl-uridin und 10,66 g (40,64 mmol) Triphenylphosphin werden in 50 ml Pyridin gelöst. Dazu gibt man in mehreren Portionen 10,32.g (40,64 mmol) Iod. Nach 4 h ist der Umsatz vollständig (DC-Kontrolle mit Dichlormethan/Methanol 15 : 1). Pyridin wird im Vakuum abdestilliert und der Rückstand in Essigester gelöst und mit 1 M Salzsäure gewaschen. Die wässrige Phase wird mehrfach mit Essigester extrahiert und die vereinigten Essigesterphasen mit Phosphatpuffer gewaschen. Nach Trocknen mit Magnesiumsulfat, abfiltrieren, und waschen mit Essigester wird das Lösungsmittel bis zur Trockne abdestilliert und der Rückstand mit Toluol und Dichlormethan gewaschen. Man erhält 7,5 g (65,6 %) eines schwach gelblichen Produktes.

### 8.1.2. 2',5'-Didesoxy-5-ethyl-uridin

4,68 g (12,78 mmol) 2',5'-Didesoxy-5'-iod-5-ethyl-uridin und 1,08 g (27,0 mmol) Natriumhydroxid werden in 280 ml Ethanol gelöst, mit 780 mg Hydrierkatalysator (Palladium auf Aktivkohle, 10 % Pd) versetzt und 12 h hydriert. Die Reaktion ist danach beendet (DC-Kontrolle mit Dichlormethan/Methanol 10 : 1). Nach Abfiltrieren des Hydrierkatalysators wird bis zur Trockne eingedampft und der Rückstand mit Phosphatpuffer versetzt. Der Niederschlag wird abgesaugt, mit Eiswasser gewaschen und über Kaliumhydroxid getrocknet. Das Filtrat wird mehrfach mit Essigester extrahiert. Die vereinigten Extrakte werden mit Magnesiumsulfat getrocknet. Nach Abfiltrieren des Trockenmittels, waschen mit Essigester wird bis zur Trockne eingedampft. Man erhält insgesamt 2,97 g (96,7 %) eines farblosen Feststoffes.

### 8.1.3. 2',5'-Didesoxy-3'-O-benzoyl-5-ethyl-uridin

3,28 g (13,65 mmol) 2',5'-Didesoxy-5-ethyl-uridin werden in 35 ml Pyridin gelöst und bei 0 °C mit 2,14 g (15,21 mmol) Benzoylchlorid versetzt. Nach 30 min wird das Kältebad entfernt und man lässt noch 1h bei Raumtemperatur rühren. Danach ist der Umsatz vollständig. Pyridin wird mit Toluol entfernt und der Rückstand in Chloroform gelöst. Es wird mir 0,1 M Salzsäure gewaschen und mit Phosphatpuffer neutral gewaschen. Nach trocknen der organischen Phase mit Magnesiumsulfat, abfiltrieren des Trockenmittels und abrotieren des Lösungsmittels erfolgt säulenchromatographische Reinigung mit Dichlormethan/Aceton 10 : 1. Man erhält 3,82 g (81,3 %) eines weißen Schaums.

### 8.1.4. 2',5'-Didesoxy-3'-O-benzoyl-5-(E)-bromvinyl-uridin

2,79 g (8,1 mmol) 2',5'-Didesoxy-3'-O-benzoyl-5-ethyl-uridin und 50 mg AIBN werden in 45 ml Chloroform gelöst und zum Sieden erhitzt. Dazu tropft man innerhalb von 1,5 h eine Lösung 2,88 g (18 mmol) Brom in 15 ml Chloroform. Man erhitzt noch 0,5 h zum Sieden und leitet nach Abkühlen auf Raumtemperatur 1 h Argon durch die Lösung. Danach setzt man der Lösung 2,88 g (19,8 mmol) Triethylamin zu und rührt 1 h bei Raumtemperatur. Chloroform wird abdestilliert und der Rückstand in Essigester aufgenommen und mit gesättigter Kochsalzlösung gewaschen. Nach trocknen der organischen Phase mit Magnesiumsulfat, abfiltrieren des Trockenmittels und abrotieren des Lösungsmittels erfolgt säulenchromatographische Reinigung mit Dichlormethan/Aceton 20 : 1. Man erhält 1,97 g (57,8 %) eines weißen Schaums.

### 8.1.5. 2',5'-Didesoxy-5-(E)-bromvinyluridin

2,48 g (5,88 mmol) 2',5'-Didesoxy-3'-O-benzoyl-5-(E)-bromvinyluridin werden in 25 ml THF gelöst und mit 15 ml (30 mmol) 2 M Natronlauge versetzt. Nach 3 h rühren bei Raumtemperatur ist die Umsetzung beendet (DC-Kontrolle mit Dichlormethan/Aceton 10 :1). Man versetzt mit 80 ml Phosphatpuffer und extrahiert mehrfach mit Essigester. Nach trocknen der organischen Phase mit Magnesiumsulfat, abfiltrieren des Trockenmittels und abrotieren des Lösungsmittels erfolgt säulenchromato-aphische Reinigung mit Chloroform/Methanol 20 : 1. Man erhält 1,65 g (88,7 %) eines farblosen Feststoffes vom Schmelzpunkt 174 °C (Zersetzung).

¹H-NMR (300 MHz, DMSO-d₆): 2,11 (d, 3H); 2,15 (m, 2H); 3,81 (m, 1H); 3,98 (m, 1H); 5,26 (d, 1H); 6,09 (m, 1H); 6,99 (d, 1H); 7,31 (d, 1H); 7,76 (s, 1H); 11,58 (s, 1H) ppm.

### 8.2. 3'-Chlor-2',3',5'-tridesoxy-5-(E)-bromvinyluridin

### 8.2.1. 2,3'-Anhydro-1-(2',5'-didesoxy-β-D-threo-pentofuranosyl)-5-(E)-bromvinyluracil

1,47 g (4,64 mmol) 2',5'-Didesoxy-5-(E)-bromvinyl-uridin und 1,83 g (6,96 mmol) Triphenylphosphin werden in 40 ml THF gelöst und zu dieser Lösung wird eine Lösung von 1,44 g (7,16mmol) Diisopropylazodicarboxylat in 10 ml THF innerhalb 20 min zugetropft. Nach 4 h ist die Reaktion beendet (DC-Kontrolle mit Dichlormethan/Methanol 15 : 1). THF wird abdestilliert und der Rückstand in Toluol aufgenommen. Es wird abfiltriert und der Rückstand mehrfach mit Toluol gewaschen. Man erhält einen farblosen Feststoff (1,19 g (85,7 %).

### 8.2.2. 3'-Chlor-2',3',5'-tridesoxy-5-(E)-bromvinyl-uridin

0,59 g (1,97 mmol) 2,3'-Anhydro-1-(2',5'-didesoxy-β-D-threo-pentofuranosyl)-5-(E)-bromvinyl-uracil und 0,5 g (4,33 mmol) Pyridinhydrochlorid werden in 5 ml DMF suspendiert und auf 90 °C erhitzt. Nach 1 h ist die Reaktion beendet (DC-Kontrolle mit Dichlormethan/Methanol 15 : 1). DMF wird mit Xylol abdestilliert und der Rückstand mit Essigester und gesättigter Kochsalzlösung behandelt. Die wässrige Phase wird mehrfach mit Essigester extrahiert und die vereinigten Essigesterphasen mit Magnesiumsulfat getrocknet. Nach abfiltrieren des Trockenmittels, waschen mit Essigester und abdestillieren des Lösungsmittels erfolgt säulenchromatographische Reinigung mit Chloroform/Methanol 100 : 1 und Chloroform/Essigester 5 : 1. Man erhält 0,25 g (37,8 %) eines farblosen Feststoffes vom Schmelzpunkt 134 °C.

¹H-NMR (300 MHz, DMSO-d₆): 1,34 (d, 3H); 2,58 (m, 1H); 2,66 (m, 1H); 4,03 (m, 1H); 4,44 (m, 1H); 6,17 (dd, 1H); 6,97 (d, 1H); 7,31 (d, 1H); 7,77 (s, 1H); 11,62 (s, 1H) ppm.

### 8.3. 3'-Brom-2',3',5'-tridesoxy-5-(E)-bromvinyl-uridin

0,59 g (1,97 mmol) 2,3'-Anhydro-1-(2',5'-didesoxy-β-D-threo-pentofuranosyl)-5-(E)-bromvinyl-uracil und 0,96 g Pyridinhydrobromid (6,0 mmol) werden in DMF umgesetzt und aufgearbeitet wie vorstehend unter 8.2.2. beschrieben. Das Produkt wird mit Chloroform/Methanol 70 : 1 mehrfach säulenchromatographisch gereinigt und man erhält 0,22 g (29,4 %) eines farblosen Feststoffes vom Schmelzpunkt 128 °C.

¹H-NMR (300MHz, DMSO-d₆): 1,35 (d, 3H); 2,65 (m, 2H); 4,12 (m, 1H); 4,41 (m, 1H); 6,15 (dd, 1H); 6,98 (d, 1H); 7,30 (d, 1H); 7,76 (s, 1H); 11,62 (s, 1H) ppm.

### 9. 1-[2',5'-dideoxy-β-D-glycero-pent-4-enofuranosyl]-5-(E)-bromvinyl-uracil als Referenzverbindung

### 9.1. 2',5'-Dideoxy-5'-iod-5-(E)-bromvinyl-uridin

3,0 g (9,0 mmol) 2'-Deoxy-5-(E)-bromvinyl-uridin und 3,07 g (11,7mmol) Triphenyl-phosphin werden in 30 ml Pyridin gelöst und auf 10 °C gekühlt. Dazu gibt man in mehreren Portionen 2,97 g (11,7 mmol) Iod. Man lässt auf Raumtemperatur aufwärmen. Nach 6 h ist der Umsatz vollständig (DC-Kontrolle mit Dichlormethan/Methanol 15 : 1). Pyridin wird abdestilliert und der Rückstand in Essigester/2-Butanon (2 : 1) aufgenommen und mit Natriumdisulfitlösung gewaschen. Die wässrige Phase wird mehrfach mit Essigester extrahiert und die vereinigten organischen Phasen mit Magnesiumsulfat getrocknet. Nach abfiltrieren des Trockenmittels und abdestillieren des Lösungsmittels wird der Rückstand mit Toluol/Dichlormethan 1 : 1 behandelt und der erhaltene Feststoff getrocknet. Man erhält 3,77 g (94,5 %) eines farblosen Feststoffes.

### 9.2. 2',5'-Dideoxy-3'-O-acetyl-5'-iod-5-(E)-bromvinyl-uridin

2,48 g (5,59 mmol) 2',5'-Dideoxy-5'-iod-5-(E)-bromvinyl-uridin werden in 20 ml Pyridin gelöst und mit 3,20 g (31,34 mmol) Acetanhydrid versetzt. Nach 90 min ist die Reaktion beendet (DC-Kontrolle mit Dichlormethan/Methanol 10 : 1). Der Ansatz wird in Phosphatpufferlösung eingegossen und nach 15 min mehrfach mit Essigester extrahiert. Die vereinigten Extrakte werden mit Magnesiumsulfat getrocknet und nach üblicher Aufarbeitung mit Chloroform/Aceton 10 1 säulenchromatographisch gereinigt. Man erhält 2,14 g (78,7 %) eines farblosen Feststoffes.

### 9.3. 1-[3'-O-Acetyl-2',5'-dideoxy-β-D-glycero-pent-4-enofuranosyl]-5-(E)-bromvinyl-uracil

2,14 g (4,41 mmol) 2',5'-Dideoxy-3'-O-acetyl-5'-iod-5-(E)-bromvinyl-uridin werden in 45 ml Acetonitril gelöst und mit 2,08 g (13,73 mmol) DBU (1,8-Diazabicyclo[5.4.0]undec-7-en) verstzt. Nach 18 h rühren bei Raumtemperatur ist der Umsatz vollständig (DC-Kontrolle mit Cyclohexan/Essigester 1 : 1). In Phosphatpuffer eingießen und mehrfach mit Essigester extrahieren. Die vereinigten Extrakte werden mit Magnesiumsulfat getrocknet und nach üblicher Aufarbeitung mit Cyclohexan/Essigester 1 : 1 und Chloroform/Aceton 8 : 1 säulenchromatographisch gereinigt. Man erhält 1,24 g (77,3 %) eines farblosen Feststoffes vom Schmelzpunkt 148 °C.

¹H-NMR (500MHz, DMSO-d₆): 2,07 (s, 3H) ; 2,44 (m, 1H); 2,71 (m, 1H); 4,27 (dd, 1H); 4,47 (d, 1H); 5,78 (m, 1H); 6,37 (t, 1H); 6,88 (d, 1H); 7,29 (d, 1H); 7,90 (s, 1H); 11,70 (s, 1H) ppm.

### 9.4. 1-[2',5'-dideoxy-β-D-glycero-pent-4-enofuranosyl]-5-(E)-bromvinyl-uracil

0,86 g (2,41 mmol) 1-[3'-O-Acetyl-2',5'-dideoxy-β-D-glycero-pent-4-enofuranosyl]-5-(E)-bromvinyl-uracil werden in 10 ml Methanol gelöst und mit 15 ml einer gesättigten Lösung von Ammoniak in Methanol versetzt. Nach 7 h ist die Umsetzung beendet DC-Kontrolle mit Chloroform/Methanol 15 : 1). Das Lösungsmittel wird abgezogen und der Rückstand säulenchromatographisch mit Chloroform/Methanol 15 : 1 und Dichlormethan/Essigester 1 : 1 gereinigt. Man erhält 210 mg (27,6 %) einer farblosen Substanz vom Schmelzpunkt 128 (Zers.) °C.

¹H-NMR (500MHz, DMSO-d₆) : 2,21 (m, 1H); 2,48 (m, 1H); 4,16 (d, 1H); 4,31 (d, 1H); 4,72 (m, 1H); 5,56 (d, 1H); 6,39 (t, 1H); 6,93 (d, 1H); 7,29 (d, 1H); 7,80 (s, 1H); 11,66 (s, 1H) ppm.

### 10. 5'-Cyano-2',5'-dideoxy-5-(E)-bromvinyl-uridin als Referenzverbindung

### 10.1. 5'-Cyano-2',5'-dideoxy-5-ethyl-uridin

2,54 g (6,93 mmol) 5'-Iod-2',5'-dideoxy-5-ethyl-uridin werden in 50 ml DMSO gelöst und dazu werden 0,52 g (10,61 mmol) Natriumcyanid gegeben. Nach 18 h ist der Umsatz vollständig (DC-Kontrolle mit Essigester). Der Ansatz wird in gesättigte Kochsalzlösung eingegossen und mit Essigester mehrfach extrahiert. Die vereinigten Extrakte werden mit Magnesiumsulfat getrocknet und nach üblicher Aufarbeitung mit Essigester säulenchromatographisch gereinigt. Man erhält 0,96 g (52,1 %) einer farblosen Substanz.

### 10.2. 3'-O-Acetyl-5'-cyano-2',5'-dideoxy-5-ethyl-uridin

0,96 g (3,62 mol) 5'-Cyano-2',5'-dideoxy-5-ethyl-uridin werden in 15 ml Pyridin gelöst und mit 1,7.7 g (17,35 mmol) Acetanhydrid versetzt. Nach 1 h ist der Umsatz vollständig (DC-Kontrolle mit Essigester). Der Ansatz wird in Phosphatpuffer eingegossen und mit Essigester mehrmals extrahiert. Die vereinigten Extrakte werden mit Magnesiumsulfat getrocknet und nach üblicher Aufarbeitung mit Dichlormethan/Essigester säulenchromatographisch gereinigt. Man erhält 0,82 g (73,7 %) einer farblosen Substanz.

### 10.3. 3'-O-Acetyl-5'-cyano-2',5'-dideoxy-5-(E)-bromvinyl-uridin

0,82 g (2,67 mmol) 3'-O-Acetyl-5.'-cyano-2',5'-dideoxy-5-ethyl-uridin und 0,01 g AIBN (Azo-bis-isobutyronitril) werden in 30 ml Chloroform gelöst und zum Sieden erhitzt. Dazu wird eine Lösung von 0,94 g (5,87 mmol) Brom in 10 ml Chloroform zugetropft. Nach 65 min ist das Zutropfen beendet. Man erhitzt noch 1 h am Rückfluß, lässt auf Raumtemperatur abkühlen und gibt 0,66 g (6,52 mmol) Triethylamin hinzu. Nach 1 h wird das Lösungsmittel abdestilliert und der Rückstand mit Essigester und Phospatpuffer behandelt. Die wässrige Phase wird mehrfach mit Essigester extrahiert und die vereinigten Essigesterphasen werden mit Magnesiumsulfat getrocknet. Nach üblicher Aufarbeitung erfolgt säulenchromatographische Reinigung mit Cyclohexan /Essigester 2 : 3. Man erhält 0,62 g (59,7 %) einer farblosen Substanz vom Schmelzpunkt 96 °C.

¹H-NMR (300 MHz, DMSO-d₆) : 2,07 (s, 3H); 2,39 (m, 1H); 2,53 (m, 1H); 3,08 (m, 2H); 4,21 (m, 1H); 5,16 (m, 1H); 6,17 (t, 1H); 6,88 (d, 1H); 7,28 (d, 1H); 7,88 (s, 1H); 11,68 (s, 1H) ppm.

### 10.4. 5'-Cyano-2',5'-dideoxy-5-(β)-bromvinyl-uridin

0,41 g (1,07 mmol) 3'-O-Acetyl-5'-cyano-2',5'-dideoxy-5-(E)-bromvinyl-uridin werden in 20 ml Methanol gelöst und mit 10 ml einer gesättigten Lösung von Chlorwasserstoff in Methanol versetzt. Nach 36 h ist der Umsatz vollständig (DC-Kontrolle mit Chloroform/Methanol 15 : 1). Nach Abdestilieren des Lösungsmittels erfolgt säulenchromatographische Reinigung Chloroform/Methanol 15 : 1. Man erhält 0,35 g (95,9 %) einer farblosen Substanz mit Schmelzpunkt 177 °C (Zers.).

¹H-NMR (300 MHz, DMSO-d₆) : 2,15 (m, 1H); 2,29 (m, 1H); 2,99 (m, 2H) : 3,92 (m, 1H) ; 4,18 (m, 1H); 5,55 (d, 1H); 6,19 (t, 1H); 6,91 (d, 1H); 7,29 (d, 1H); 7, 80 (s, 1H) ; 11, 63 (s, 1H) ppm.

### 11. Substituierte 1-(2'Desoxy-β-D-threo-pentofura-nosyl)-5-(E)-(2-bromvinyl)-2,4-(1H, 3H)-pyrimid-indione als Referenzverbindungen

### 11.1. 1-(5'-Iod-2',5'-didesoxy-β-D-threo-pentofura-nosyl)-5-(E)-(2-bromvinyl)-2,9-(1H,3H)-pyrimidindion

### 11.1.1. 1-(2'-Desoxy-β-D-threo-pentofuranosyl)-5-(E)-(2-bromvinyl)-2,4-(1H ,3H)-pyrimidindion

15,8 g (37,6 mmol) 2,3'-Anhydro-5'-O-benzoyl-2'-desoxy-5-(E)-bromvinyluridin werden mit 65,7 ml (132 mmol, 3,5 Äq.) 2 M Natronlauge in 600 ml eines Gemisches aus Ethanol/Wasser (5/1) 1 h unter Rückfluss erhitzt. Nach dem Abkühlen des Reaktionsgemisches auf Raumtemperatur wird mit konz. Salzsäure neutralisiert und anschließend das Solvens am Rotationsverdampfer entfernt. Säulenchromatographische Reinigung (Essigester/Methanol, 9/1 und Chloroform/Methanol, 7/1) liefert 10,9 g (32,7 mmol, 87 %) 1-(2'-Desoxy-β-D-threo-pentofuranosyl)-5-(E)-(2-bromvinyl)-2,4-(1H ,3H)-pyrimidindion als weißen Feststoff.

### 11.1.2. 1-(5'-Methylsulfonyl-2'-desoxy-β-D-threo-pentofuranosyl)-5-(E)-(2-bromvinyl)-2,4-(1H ,3H)-pyrimidindion

10,9 g (32,7 mmol) 1-(2'-Desoxy-β-D-threo-pentofura-nosyl)-5-(E)-(2-bromvinyl)-2,4-(1H3H)-pyrimidindion werden bei 0°C in 100 ml Pyridin vorgelegt. 3,94 g (34,4 mmol, 1,05 Äq.) Methylsulfonylchlorid werden in 50 ml THF gelöst und langsam zugetropft. Anschließend wird das Reaktionsgemisch weitere 3 h bei 0°C gerührt. Der Ansatz wird auf 300 g Eis gegossen, mit konz. Salzsäure auf pH = 5 eingestellt und das Gemisch mit 3 x 100 ml Essigester extrahiert. Die vereinte organische Phase wird mit 1 M Salzsäure sowie NaCl-Lösung gewaschen, mit Na₂SO₄ getrocknet, filtriert und das Solvens am Rotationsverdampfer entfernt. Es resultieren 11,8 g (28,7 mmol) 1-(5'-Methylsulfonyl-2'-desoxy-β-D-threo-pentofuranosyl)-5-(E)-(2-bromvinyl)-2,4-(1H ,3H)-pyrimidindion als weißer Feststoff. Das Rohprodukt wird ohne weitere Reinigung umgesetzt.

### 11.1.3. 1-(5'-Iod-2',5'-didesoxy-β-D-threo-pento-furanosyl)-5-(E)-(2-bromvinyl)-2,4-(1R ,3H)-pyrimidindion

0,50 g (1,22 mmol) 1-(5'-Methylsulfonyl-2'-desoxy-β-D-threo-pentofuranosyl)-5-(E)-(2-bromvinyl)-2,4-(1H ,3H)-pyrimidindion werden mit 911 mg (6,08 mmol, 5,0 Äq.) Natriumiodid in 10 ml DMF 20 h erhitzt. Das Reaktionsgemisch wird in 50 ml Essigester aufgenommen, mit Wasser und NaCl-Lösung gewaschen, mit Na₂SO₄ getrocknet, filtriert und das Solvens am Rotationsverdampfer entfernt. Säulenchromatographische Reinigung (Chloroform/Methanol, 9/1) liefert 130 mg (293 µmol, 24 %) 1-(5'-Iod-2',5'-didesoxy-β-D-threo-pentofura-nosyl)-5-(E)-(2-bromvinyl)-2,4-(1H,3H)-pyrimidindion als schwach gelblichen Feststoff mit einem Schmelzpunkt von 79 - 81 °C.

¹H-NMR (500 MHz, DMSO-d₆): 2,04 (d, 1H); 2,58 (m, 1H); 3,43-3.53 (m, 2H); 4,16 (m, 1H); 4,26 (m, 1H); 5,44 (d, 1H); 6,06 (dd, 1H); 6,93 (d, 1H); 7,24 (d, 1H); 7,98 (s, 1H); 11,57 (s, 1H) ppm.

### 12. 1-(2',3'-Didehydro-2'-desoxy-2'-fluor-β-D-arabinofuranosyl)-5-ethyluracil als Referenzverbindung

### 12.1. 1-(2'-Desoxy-2'-fluor-5'-O-trityl-β-D-arabino-furanosyl)-5-ethyluracil

22,8 g (83,2 mmol) 1-(2'-Desoxy-2'-fluor-β-D-arabinofuranosyl)-5-ethyluracil werden in 200 ml Pyridin suspendiert und 25,5 g (91,6 mmol) Tritylchlorid zugegeben. Das Reaktionsgemisch wird 24 h bei Raumtemperatur gerührt und anschließend das Solvens am Rotationsverdampfer entfernt. Der Rückstand wird in 300 ml Essigester aufgenommen und mit verdünnter Salzsäure, NaHCO₃-Lsg. und NaCl-Lsg. gewaschen. Die organische Phase wird mit Na₂SO₄ getrocknet, filtriert und das Solvens am Rotationsverdampfer entfernt. Säulenchromatographische Reinigung (Chloroform/Methanol, 9/1) liefert 26,1 g (50,5 mmol, 61 %) 1-(2'-Desoxy-2'-fluor-5'-O-trityl-β-D-arabinofurano-syl)-5-ethyluracil als weißen Feststoff.

### 12.2. 2,3'-Anhydro-1-(2'-desoxy-2'-fluor-5'-O-trityl-β-D-arabinofuranosyl)-5-ethyluracil

26,1 g (50,5 mmol) 1-(2'-Desoxy-2'-fluor-5'-O-trityl-β-D-arabinofuranosyl)-5-ethyluracil werden in 300 ml DMF vorgelegt und 19,9 g (75,7 mmol) PPH₃ zugegeben. Anschließend werden 15,3 g (75,7 mmol) Diisopropylazodicarboxylat in 50 ml DMF gelöst und zugetropft. Nach zwei Stunde wird das Solvens am Rotationsverdampfer entfernt, der Rückstand in 1 l Diethylether aufgenommen und 16 h kräftig verrührt. Der resultierende Feststoff wird abgesaugt und 3x mit 50 ml Diethylether gewaschen. Umkristallisation aus Cyclohexan/Essigester liefert 25,5 g (50,5 mmol, 100 %) an 2,3'-Anhydro-1-(2'-desoxy-2'-fluor-5'-O-trityl-β-D-arabinofuranosyl)-5-ethyluracil als weißer Feststoff.

### 12.3. 1-(2',3'-Didehydro-2'-desoxy-2'-fluor-5'-O-trityl-β-D-arabinofuranosyl)-5-ethyluracil

25,5 g (50,5 mmol) 2,3'-Anhydro-1-(2'-desoxy-2'-fluor-5'-O-trityl-β-D-arabinofuranosyl)-5-ethyluracil werden in 600 ml Ethanol gelöst, 125 ml Wasser und 62,5 ml (126 mmol) 2 M NaOH-Lsg. zugegeben und das Reaktionsgemisch wird 2 h unter Rückfluss erhitzt. Das Ethanol wird weitgehend am Rotationsverdampfer entfernt. Der Rückstand wird mit verdünnter Salzsäure neutralisiert und mit Essigester extrahiert. Die vereinigten organischen Phasen werden mit NaCl-Lsg. gewaschen, mit Na₂SO₄ getrocknet, filtriert und das Solvens am Rotationsverdampfer entfernt. Säulenchromatographische Reinigung (Chloroform/Methanol, 95/5) liefert 19,6 g (39,2 mmol, 78 %) 1-(2',3'-Didehydro-2'-desoxy-2'-fluor-5'-O-trityl-β-D-arabinofuranosyl)-5-ethyluracil als weißen Feststoff.

### 12.4. 1-(2',3'-Didehydro-2'-desoxy-2'-fluor-β-D-arabinofuranosyl)-5-ethyluracil

5,00 g (10,0 mmol) 1-(2',3'-Didehydro-2'-desoxy-2'-fluor-5'-O-trityl-β-D-arabinofuranosyl)-5-ethyluracil werden in 50 ml Dioxan gelöst und auf 0°C gekühlt. 4,2 ml (20,0 mmol) 4,8 M HCl in Dioxan werden langsam zugetropft. Das Kühlbad wird entfernt und das Reaktionsgemisch 2 h gerührt. Anschließend wird das Solvens am Rotationsverdampfer entfernt. Säulenchromatographische Reinigung (Chloroform/Methanol, 9/1) liefert 2,06 g (8,04 mmol, 80 %) 1-(2',3'-Didehydro-2'-desoxy-2'-fluor-β-D-arabinofuranosyl)-5-ethyluracil als weißen Feststoff mit einem Schmelzpunkt von 145-146°C.

¹H-NMR (300 MHz, DMSO-d₆): 1,02 (t, 3H) ; 2,20 (q, 2H); 3,55-3,65 (m, 2H); 4,76-4,84 (m, 1H); 5,16 (t, 1H) ; 5, 95-6, 05 (m, 1H); 6,75-6,80 (m, 1H); 7,86 (s, 1H); 11,42 (s, 1H).

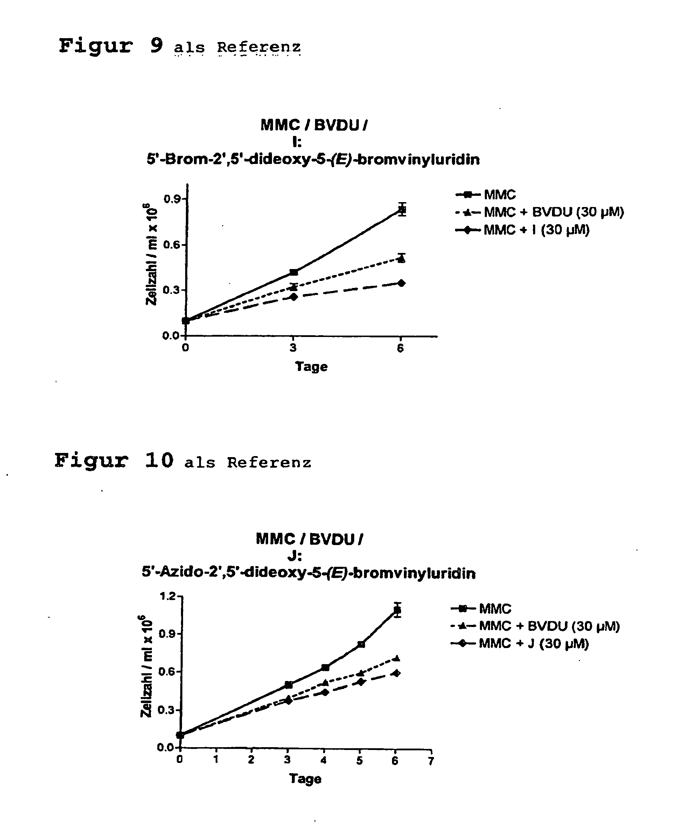

Die Fig. 12 zeigt die Ergebnisse einer weiteren erfindungsgemäßen Verbindungen. Fig. 9 bis 11 zeigen die Ergebnisse von Referenzverbindungen.

Fig. 9 zeigt die Ergebnisse von 5'-Brom-2',5'-dideoxy-5-(E)-bromvinyluridin in Kombination mit Mitomycin C (MMC) im Vergleich zu MMC alleine und MMC in Kombination mit BVDU.

Fig. 10 zeigt die Ergebnisse von 5'-Azido-2',5'-dideoxy-5-(E)-bromvinyluridin in Kombination mit Mitomycin C (MMC) im Vergleich zu MMC alleine und MMC in Kombination mit BVDU.

Fig. 11 zeigt die Ergebnisse von 1-(3'Azido-2',3'-dideoxy-β-D-erythro-pentofuranosyl)-5-(E)-(2-bromvinyl)-2,4(1H, 3H)-pyrimidindion in Kombination mit Mitomycin C (MMC) im Vergleich zu MMC alleine und MMC in Kombination mit BVDU.

Fig. 12 zeigt die Ergebnisse von 3'-Cl-BVDU in Kombination mit Mitomycin C (MMC) im Vergleich zu MMC alleine und MMC in Kombination mit BVDU.

## Patentansprüche

1. Nukleoside der allgemeinen Formel I 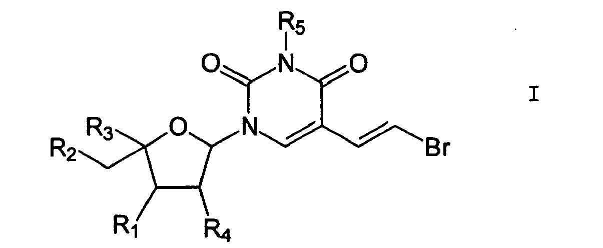 mit
R₁ = Halogen,
R₂ ausgewählt aus der Gruppe bestehend aus H, Halogen, OR₈, CN, N₃, NR₆R₇ und über ein Sauerstoffatom gebundenen Prodrugresten, wobei
der Prodrugrest ausgewählt ist aus der Gruppe bestehend aus 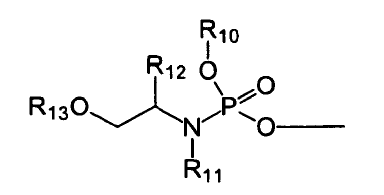 mit
R₁₀ und R₁₁ unabhängig voneinander geradkettiger oder verzweigter C₁-C₈-Alkyl, Aryl, der auch Heteroatome aufweisen kann, R₁₂ = geradkettiger oder verzweigter C₁-C₈-Alkyl, Aralkylen, Aryl, der auch Heteroatome aufweisen kann, R₁₃ = geradkettiger oder verzweigter C₁-C₈-Alkyl, 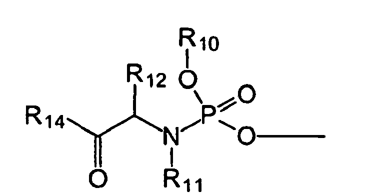 mit
R₁₀ und R₁₁ unabhängig voneinander geradkettiger oder verzweigter C₁-C₈-Alkyl, Aryl, der auch Heteroatome aufweisen kann, R₁₂ = geradkettiger oder verzweigter C₁-C₈-Alkyl, Aralkylen, Aryl, der auch Heteroatome aufweisen kann, R₁₃ = geradkettiger oder verzweigter C₁-C₈-Alkyl, R₁₄ = O-C₁-C₈-Alkyl, NH-C₁-C₈-Alkyl, N-(C₁-C₈-Alkyl)₂, 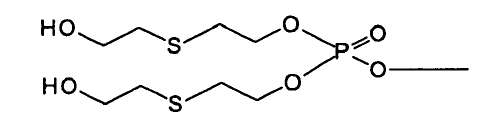 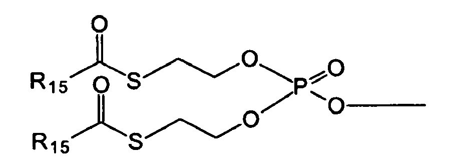 mit
R₁₅ = geradkettiger oder verzweigter C₁-C₈-Alkyl, 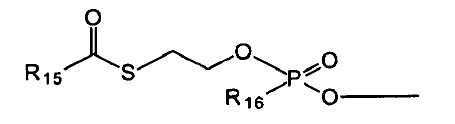 mit
R₁₅ = geradkettiger oder verzweigter C₁-C₈-Alkyl,
R₁₆ = geradkettiger oder verzweigter C₁-C₈-Alkyl, O-Aryl 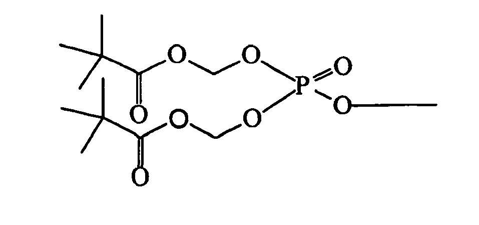 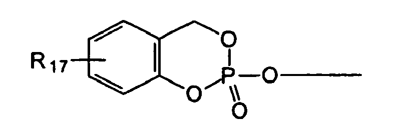 mit
R₁₇ = geradkettiger oder verzweigter C₁-C₈-Alkyl, Halogen, 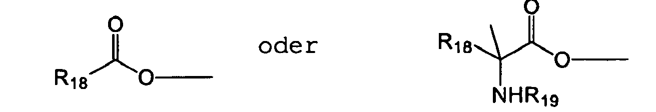 mit
R₁₈ = geradkettig oder verzweigt C₁-C₈-Alkyl, Aryl oder Heteroaryl, R₁₉ = Alkyl-CO-, Aryl-CO-, Aminosäure oder Peptid, 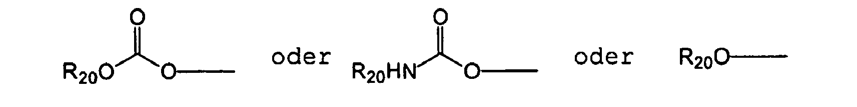 mit
R₂₀ = geradkettig oder verzweigt C₁-C₈-Alkyl,
R₃ = H, geradkettiges oder verzweigtes C₁-C₈-Alkyl, geradkettiges oder verzweigtes C₁-C₈-Alkylen,
R₄ = H, Halogen, OR₈, N₃, NR₆R₇ oder R₄ gemeinsam mit R₁ eine zweite Bindung zwischen den zu R₁ und R₄ benachbarten C-Atomen darstellen,
R₅ = H, C₁-C₈-Alkyl oder Aryl und
R₆, R₇ und R₈ unabhängig voneinander H, geradkettiges oder verzweigtes C₁-C₈-Alkyl oder Acetyl, wobei die Verbindungen mit folgenden Resten ausgeschlossen sind:
R₁ = F, R₂ = OH, R₃ = R₄ = R₅ = H,
R₁ = F, R₂ = O-Acetyl, R₃ = R₄ = R₅ = H,
R₁ = F, R₂ = Triphosphat, R₃ = R₄ = R₅ = H,
R₁ = Cl, R₂ = OH, R₃ = R₄ = R₅ = H.

2. Nukleosid nach Anspruch 1, **gekennzeichnet durch** die Formel II: 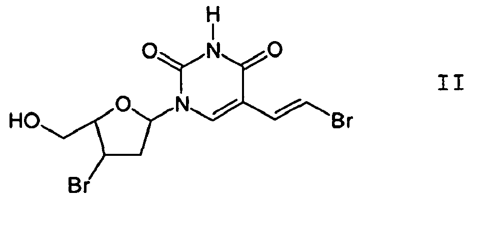 oder
**durch** die Formel III: 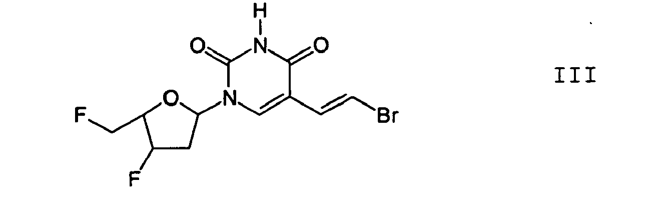 oder
**durch** die Formel IV: 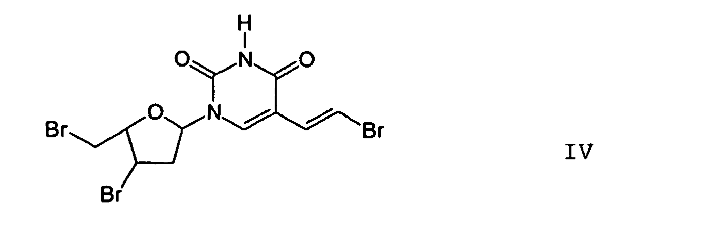 oder
**durch** die Formel V: 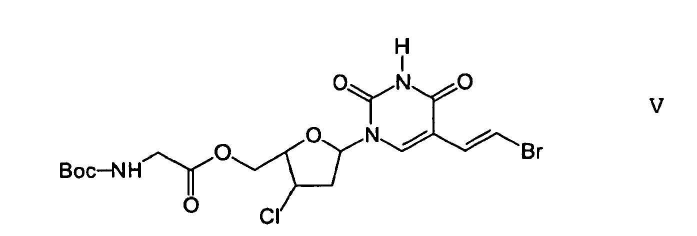 oder
**durch** die Formel VI: 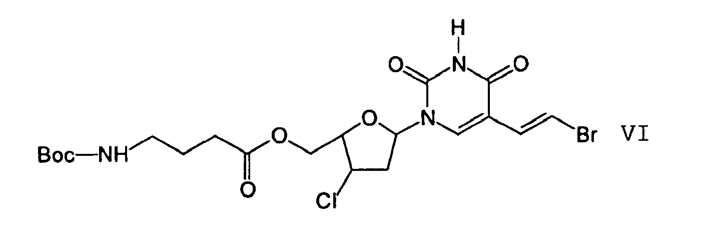 oder
**durch** die Formel VII: 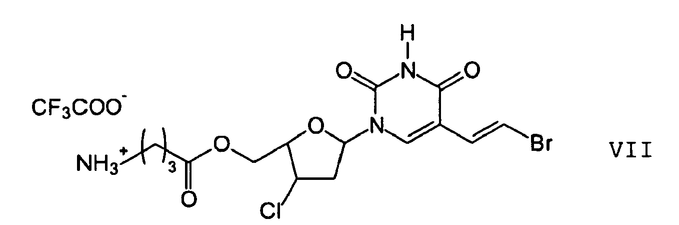 oder
**durch** die Formel VIII:

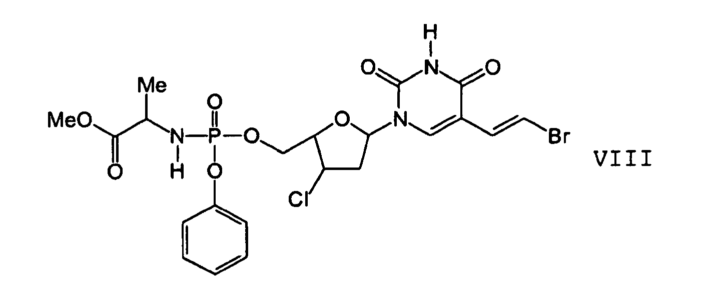

3. Nukleosid der allgemeinen Formel IX 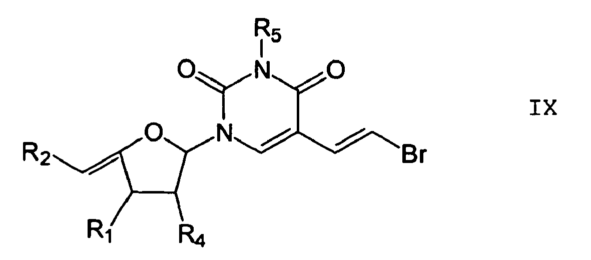 mit
R₁ und R₂ unabhängig voneinander ausgewählt aus der Gruppe bestehend aus H, Halogen, OR₈, CN, N₃, NR₆R₇ und über ein Sauerstoffatom gebundenen Prodrugresten,
R₄ = Halogen,
R₅ = H, C₁-C₈-Alkyl oder Aryl und
R₆, R₇ und R₈ unabhängig voneinander H, geradkettiges oder verzweigtes C₁-C₈-Alkyl oder Acetyl.

4. Arzneimittel enthaltend mindestens ein Nukleosid nach einem der vorhergehenden Ansprüche.

5. Arzneimittel nach Anspruch 4, **dadurch gekennzeichnet, dass** das Arzneimittel zusätzlich mindestens ein Zytostatikum enthält.

6. Verwendung von mindestens einem Nukleosid der allgemeinen Formel I 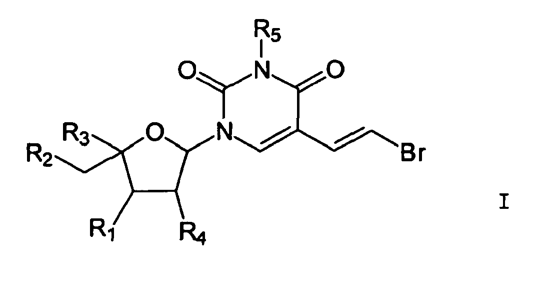 mit
R₁ = Halogen,
R₂ ausgewählt aus der Gruppe bestehend aus H, Halogen, OR₈, CN, N₃, NR₆R₇ und über ein Sauerstoffatom gebundenen Prodrugresten,
R₃ = H, geradkettiges oder verzweigtes C₁-C₈-Alkyl, geradkettiges oder verzweigtes C₁-C₈-Alkylen,
R₄ = H, Halogen, OR₈, N₃, NR₆R₇ oder R₄ gemeinsam mit R₁ eine zweite Bindung zwischen den zu R₁ und R₄ benachbarten C-Atomen darstellen,
R₅ = H, C₁-C₈-Alkyl oder Aryl und
R₆, R₇ und R₈ unabhängig voneinander H, geradkettiges oder verzweigtes C₁-C₈-Alkyl oder Acetyl,
sowie mindestens einem Zytostatikum zur Herstellung eines Arzneimittels zur Unterdrückung oder Reduzierung der Resistenzenbildung bei der Zytostatikabehandlung.

7. Verwendung nach Anspruch 6,
**dadurch gekennzeichnet, dass** der Prodrugrest ausgewählt ist aus der Gruppe bestehend aus 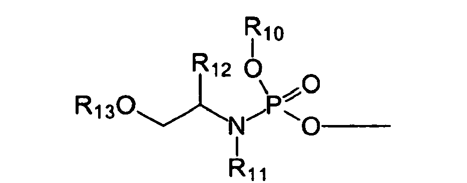 mit
R₁₀ und R₁₁ unabhängig voneinander geradkettiger oder verzweigter C₁-C₈-Alkyl, Aryl, der auch Heteroatome aufweisen kann, R₁₂ = geradkettiger oder verzweigter C₁-C₈-Alkyl, Aralkylen, Aryl, der auch Heteroatome aufweisen kann, R₁₃ = geradkettiger oder verzweigter C₁-C₈-Alkyl, 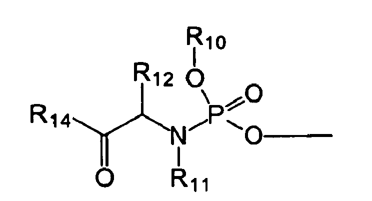 mit
R₁₀ und R₁₁ unabhängig voneinander geradkettiger oder verzweigter C₁-C₈-Alkyl, Aryl, der auch Heteroatome aufweisen kann, R₁₂ = geradkettiger oder verzweigter C₁-C₈-Alkyl, Aralkylen, Aryl, der auch Heteroatome aufweisen kann, R₁₃ = geradkettiger oder verzweigter C₁-C₈-Alkyl, R₁₄ = O-C₁-C₈-Alkyl, NH-C₁-C₈-Alkyl, N-(C₁-C₈-Alkyl)₂, 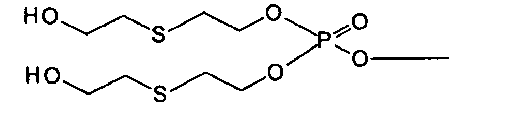 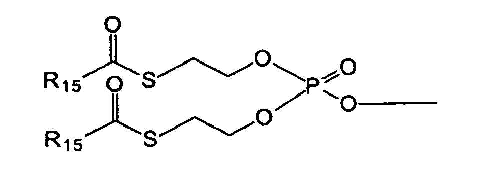 mit
R₁₅ = geradkettiger oder verzweigter C₁-C₈-Alkyl, 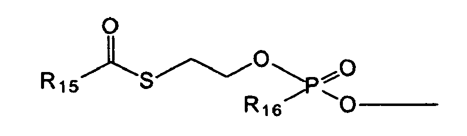 mit
R₁₅ = geradkettiger oder verzweigter C₁-C₈-Alkyl,
R₁₆ = geradkettiger oder verzweigter C₁-C₈-Alkyl, O-Aryl 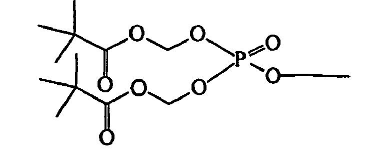 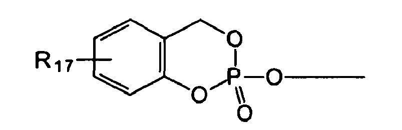 mit
R₁₇ = geradkettiger oder verzweigter C₁-C₈-Alkyl, Halogen, 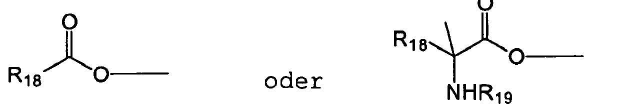 mit
R₁₈ = geradkettig oder verzweigt C₁-C₈-Alkyl, Aryl oder Heteroaryl, R₁₉ = Alkyl-CO-, Aryl-CO-oder Aminosäure, 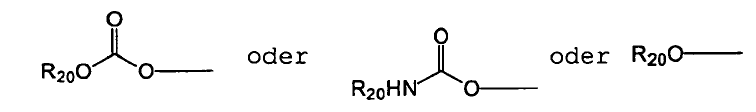 mit
R₂₀ = geradkettig oder verzweigt C₁-C₈-Alkyl.

8. Verwendung von mindestens einem Nukleosid der allgemeinen Formel IX nach Anspruch 3 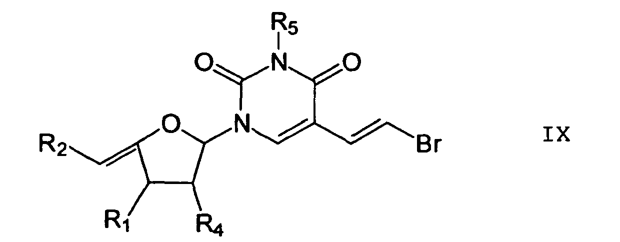 mit
R₁ und R₂ unabhängig voneinander ausgewählt aus der Gruppe bestehend aus H, Halogen, OR₈, CN, N₃, NR₆R₇ und über ein Sauerstoffatom gebundenen Prodrugresten,
R₄ = Halogen,
R₅ = H, C₁-C₈-Alkyl oder Aryl und
R₆, R₇ und R₈ unabhängig voneinander H, geradkettiges oder verzweigtes C₁-C₈-Alkyl oder Acetyl,
sowie mindestens einem Zytostatikum zur Herstellung eines Arzneimittels zur Unterdrückung oder Reduzierung der Resistenzenbildung bei der Zytostatikabehandlung.

9. Verwendung nach einem der Ansprüche 6 oder 7, **dadurch gekennzeichnet, dass** Nukleoside der Formel X 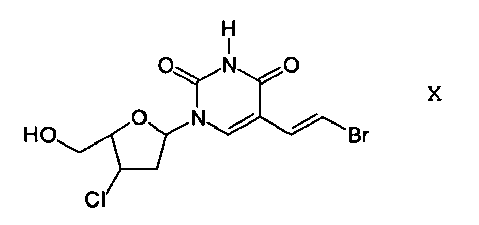 eingesetzt werden.

10. Verwendung nach einem der Ansprüche 6 bis 9, **dadurch gekennzeichnet, dass** das mindestens eine Nukleosid und das mindestens eine Zytostatikum in einer einzigen Formulierung oder dass das mindestens eine Nukleosid und das mindestens eine Zytostatikum in getrennten Formulierungen verwendet werden.

11. Verwendung von mindestens einem Nukleosid der allgemeinen Formel I 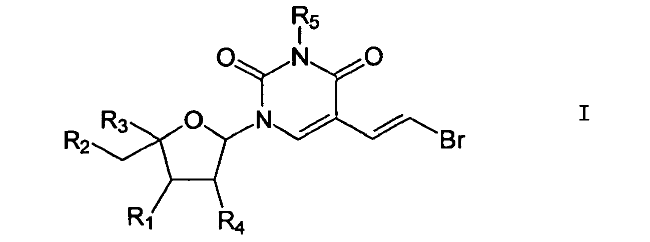 mit
R₁ = Halogen,
R₂ ausgewählt aus der Gruppe bestehend aus H, Halogen, OR₈, CN, N₃, NR₆R₇ und über ein Sauerstoffatom gebundenen Prodrugresten,
R₃ = H, geradkettiges oder verzweigtes C₁-C₈-Alkyl, geradkettiges oder verzweigtes C₁-C₈-Alkylen,
R₄ = H, Halogen, OR₈, N₃ oder NR₆R₇,
R₅ = H, C₁-C₈-Alkyl oder Aryl und
R₆, R₇ und R₈ unabhängig voneinander H, geradkettiges oder verzweigtes C₁-C₈-Alkyl oder Acetyl,
sowie mindestens einem aktiven Wirkstoff zur Herstellung eines Arzneimittels zur resistenzfreien Therapie von durch Bakterien, Plasmodien oder Leishmanien verursachten Infektionskrankheiten.

12. Verwendung nach Anspruch 11,
**dadurch gekennzeichnet, dass** das mindestens eine Nukleosid und das mindestens eine Zytostatikum in einer einzigen Formulierung verwendet werden.

## Claims

1. Nucleosides of the general Formula I 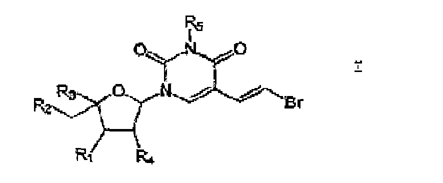 where
R₁ = Halogen,
R₂ is selected from the group consisting of H, Halogen, OR₈, CN, N₃, NR₆R₇ and prodrug residues bonded via an oxygen atom, wherein
the prodrug residue is selected from the group consisting of 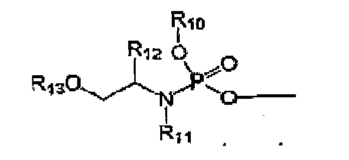 where
R₁₀ and R₁₁ mutually independently represent linear or branched C₁-C₈ alkyl, aryl, which can also have heteroatoms, R₁₂ = linear or branched C₁-C₈ alkyl, aralkyls, aryl, which can also have heteroatoms, R₁₃ = linear or branched C₁-C₈ alkyl, 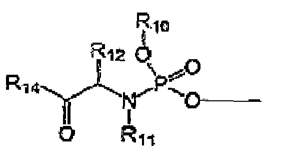 where
R₁₀ and R₁₁ mutually independently represent linear or branched C₁-C₈ alkyl, aryl, which can also have heteroatoms, R₁₂ = linear or branched C₁-C₈ alkyl, aralkyls, aryl, which can also have heteroatoms, R₁₃ = linear or branched C₁-C₈ alkyl, R₁₄ = O-C₁-C₈ alkyl, NH-C₁-C₈ alkyl, N-(C₁-C₈ alkyl)₂, 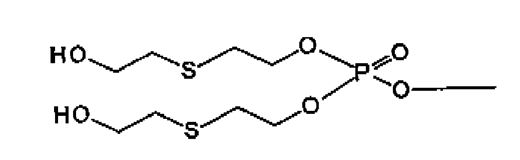 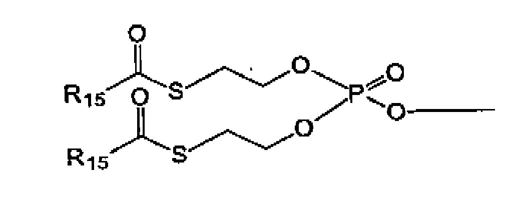 where
R₁₅ = linear or branched C₁-C₈ alkyl, 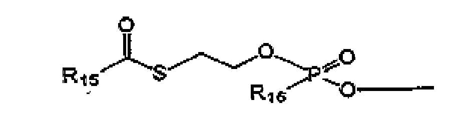 where
R₁₅ = linear or branched C₁-C₈ alkyl,
R₁₆ = linear or branched C₁-C₈ alkyl, O aryl 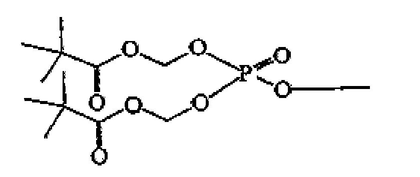 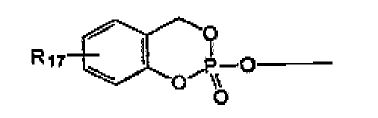 where
R₁₇ = linear or branched C₁-C₈ alkyl, halogen, 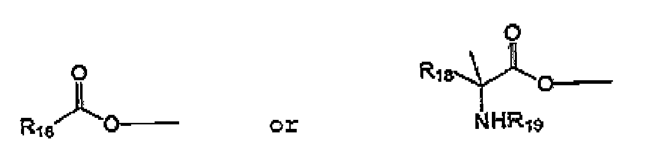 where
R₁₈ = linear or branched C₁-C₈ alkyl, aryl or heteroaryl, R₁₉ = alkyl-CO-, aryl-CO-, amino acid or peptide, 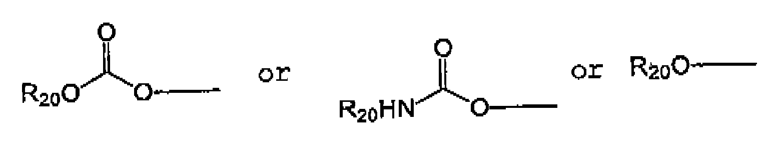 where
R₂₀ = linear or branched C₁-C₈ alkyl,
R₃ = H, linear or branched C₁-C₈ alkyl, linear or branched C₁-C₈ alkylene,
R₄ = H, halogen, OR₈, N₃, NR₆R₇, or R₄ together with R₁ represent a second bond between the C atoms neighbouring R₁ and R₄,
R₅ = H, C₁-C₈ alkyl or aryl and R₆, R₇, and R₈ mutually independently represent H, linear or branched C₁-C₈ alkyl or acetyl, wherein compounds with the following residues are excluded:
R₁ = F, R₂ = OH, R₃ = R₄ = R₅ = H,
R₁ = F, R₂ = O acetyl, R₃ = R₄ = R₅ = H,
R₁ = F, R₂ = triphosphate, R₃ = R₄ = R₅ = H,
R₁ = Cl, R₂ = OH, R₃ = R₄ = R₅ = H.

2. Nucleoside according to claim 1, **characterised by** Formula II: 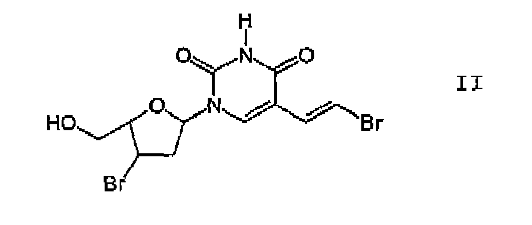 or
by Formula III: 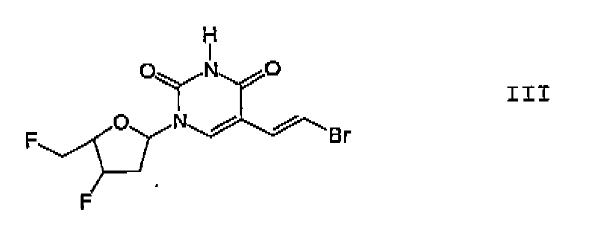 or
by Formula IV: 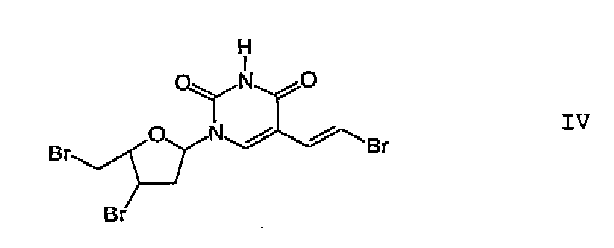 or
by Formula V: 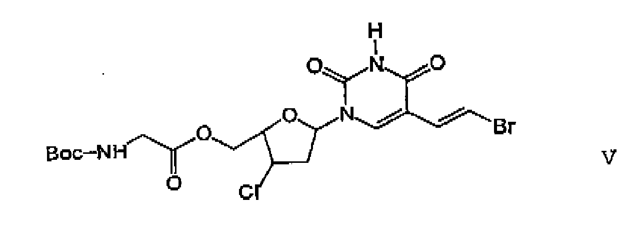 or
by Formula VI: 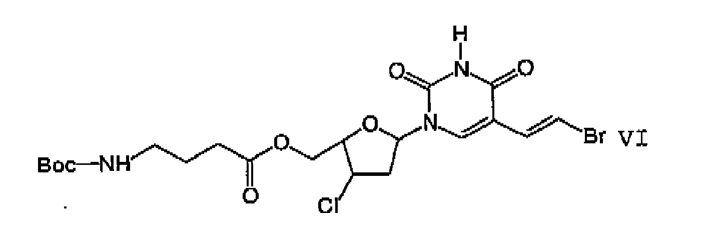 or
by Formula VII: 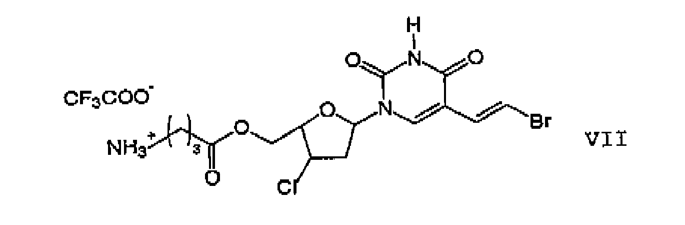 or
by Formula VIII:

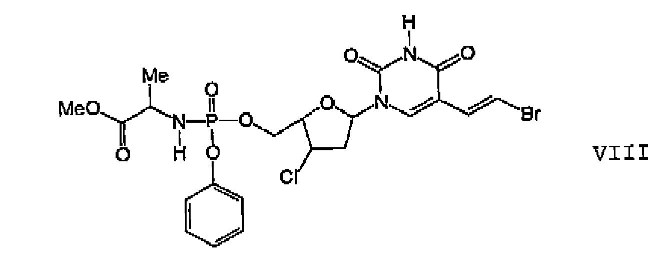

3. Nucleoside of the general Formula IX 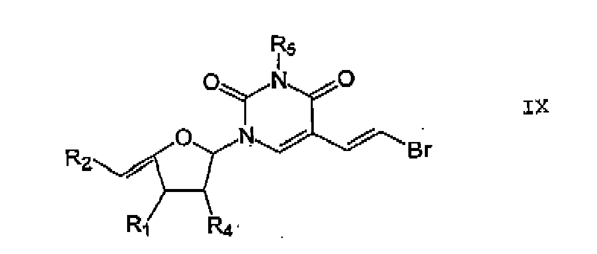 where
R₁ and R₂ are selected independently of each other from the group consisting of H, halogen, OR₈, CN, N₃, NR₆R₇ and prodrug residues bonded via an oxygen atom,
R₄ = halogen,
R₅ = H, C₁-C₈ alkyl or aryl and R₆, R₇ and R₈ mutually independently represent H, linear or branched C₁-C₈ alkyl or acetyl.

4. Medicine containing at least one nucleoside according to one of the preceding claims.

5. Medicine according to claim 4, **characterised in that** the medicine additionally contains at least one cytostatic.

6. Use of at least one nucleoside of the general Formula I 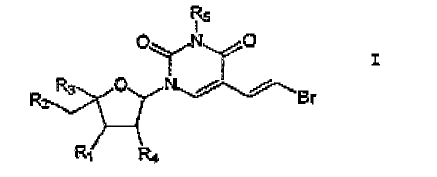 where
R₁ = halogen,
R₂ is selected from the group consisting of H, halogen, OR₈, CN, N₃, NR₆R₇ and prodrug residues bonded via an oxygen atom, R₃ = H, linear or branched C₁-C₈ alkyl, linear or branched C₁-C₈ alkylene,
R₄ = H, halogen, OR₈, N₃, NR₆R₇ or R₄ together with R₁ represent a second bond between the C atoms neighbouring R₁ and R₄,
R₅ = H, C₁-C₈ alkyl or aryl and
R₆, R₇ and R₈ mutually independently represent H, linear or branched C₁-C₈ alkyl or acetyl, and at least one cytostatic for producing a medicine for suppressing or reducing the build-up of resistance during cytostatic treatment.

7. Use according to claim 6,
**characterised in that** the prodrug residue is selected from the group consisting of 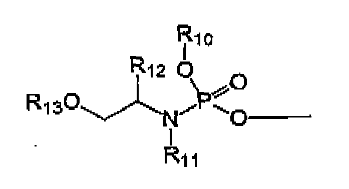 where
R₁₀ and R₁₁ mutually independently represent linear or branched C₁-C₈ alkyl, aryl, which can also have heteroatoms, R₁₂ = linear or branched C₁-C₈ alkyl, aralkyls, aryl, which can also have heteroatoms, R₁₃ = linear or branched C₁-C₈ alkyl, 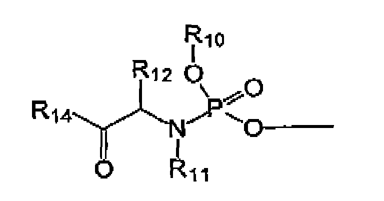 where
R₁₀ and R₁₁ mutually independently represent linear or branched C₁-C₈ alkyl, aryl, which can also have heteroatoms, R₁₂ = linear or branched C₁-C₈ alkyl, aralkyls, aryl, which can also have heteroatoms, R₁₃ = linear or branched C₁-C₈ alkyl, R₁₄ = O-C₁-C₈ alkyl, NH-C₁-C₈ alkyl, N-(C₁-C₈ alkyl)₂, 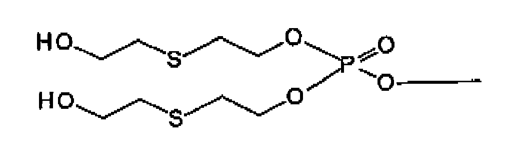 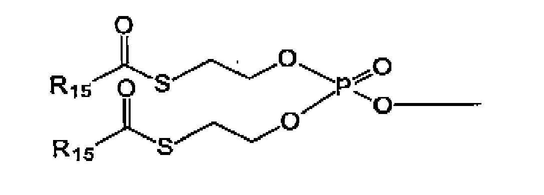 where
R₁₅ = linear or branched C₁-C₈ alkyl, 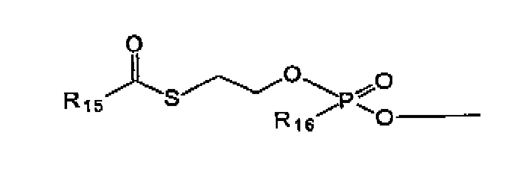 where
R₁₅ = linear or branched C₁-C₈ alkyl,
R₁₆ = linear or branched C₁-C₈ alkyl,
O aryl 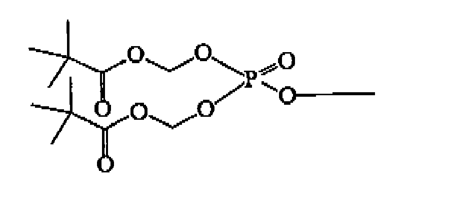 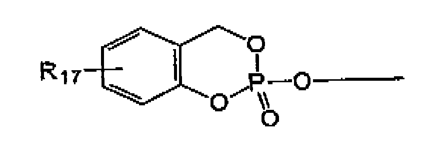 where
R₁₇ = linear or branched C₁-C₈ alkyl, halogen 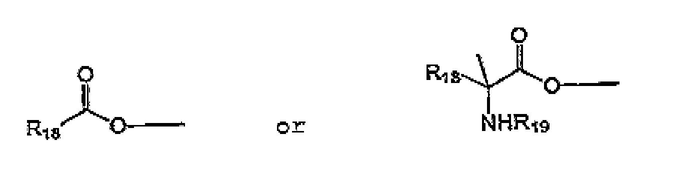 where
R₁₆ = linear or branched C₁-C₈ alkyl, aryl or heteroaryl, R₁₉ = alkyl-CO-, aryl-CO- or amino acid, 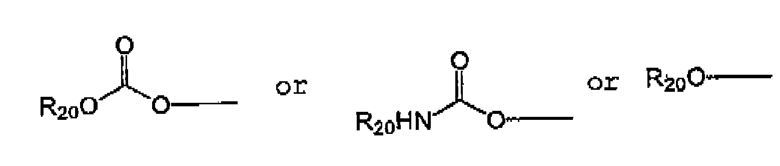 where
R₂₀ = linear or branched C₁-C₈ alkyl.

8. Use of at least one nucleoside of the general Formula IX according to claim 3 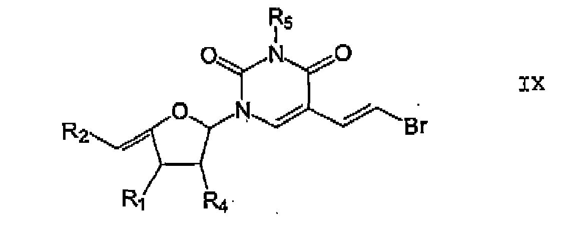 where
R₁ and R₂ are selected independently of each other from the group consisting of H, halogen, OR₈, CN, N₃, NR₆R₇ and prodrug residues bonded via an oxygen atom,
R₄ = halogen,
R₅ = H, C₁-C₈ alkyl or aryl and
R₆, R₇ and R₈ mutually independently represent H, linear or branched C₁-C₈ alkyl or acetyl,
and at least one cytostatic for producing a medicine for suppressing or reducing the build-up of resistance during cytostatic treatment.

9. Use according to one of claims 6 or 7, **characterised in that** nucleosides of the Formula X 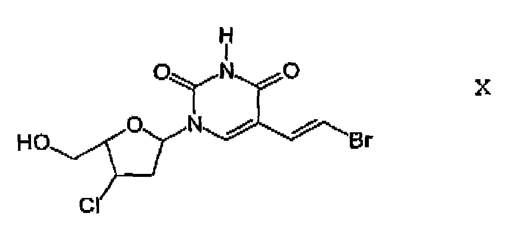 are deployed.

10. Use according to one of claims 6 to 9, **characterised in that** the at least one nucleoside and the at least one cytostatic are used in a single formulation, or the at least one nucleoside and the at least one cytostatic are used in separate formulations.

11. Use of at least one nucleoside of the general Formula I 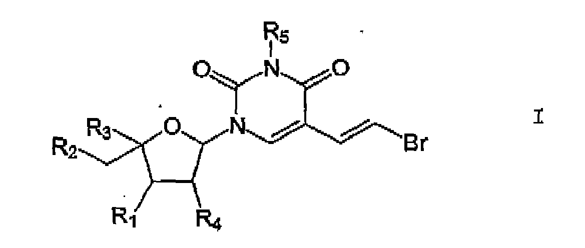 where
R₁ = halogen,
R₂ is selected from the group consisting of H, halogen, OR₈, CN, N₃, NR₆R₇ and prodrug residues bonded via oxygen atoms,
R₃ = H, linear or branched C₁-C₈ alkyl, linear or branched C₁-C₈ alkylene,
R₄ = H, halogen, OR₈, N₃ or NR₆R₇,
R₅ = H, C₁-C₈ alkyl or aryl and
R₆, R₇ and R₈ mutually independently represent H, linear or branched C₁-C₈ alkyl or acetyl,
and at least one active agent for producing a medicine for resistance-free therapy of infectious diseases caused by bacteria, plasmodia or Leishmania.

12. Use according to claim 11,
**characterised in that** the at least one nucleoside and the at least one cytostatic are used in a single formulation.

## Revendications

1. Nucléosides de formule générale I 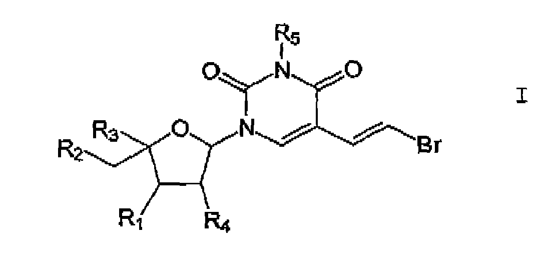 dans laquelle
R₁ est un atome d'halogène,
R₂ est choisi dans le groupe consistant en H, un halogène, OR₈, CN, N₃, NR₆R₇ et les résidus prodrogues liés par l'intermédiaire d'un atome d'oxygène,
le résidu prodrogue étant choisi dans le groupe consistant en 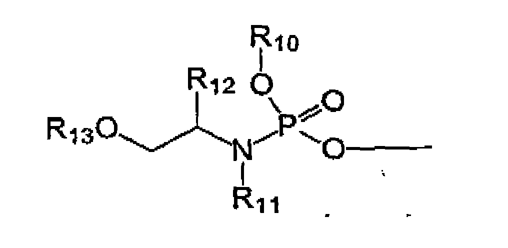 où
R₁₀ et R₁₁ représentent chacun indépendamment de l'autre un groupe alkyle en C₁-C₈ à chaîne droite ou ramifiée, aryle pouvant aussi comprendre des hétéroatomes, R₁₂ est un groupe alkyle en C₁-C₈ à chaîne droite ou ramifiée, aralkylène, aryle pouvant aussi comprendre des hétéroatomes, R₁₃ est un groupe alkyle en C₁-C₈ à chaîne droite ou ramifiée 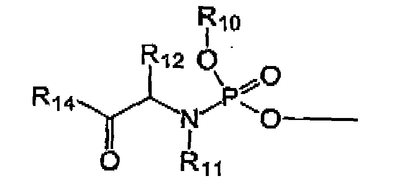 où
R₁₀ et R₁₁ représentent chacun indépendamment de l'autre un groupe alkyle en C₁-C₈ à chaîne droite ou ramifiée, aryle pouvant aussi comprendre des hétéroatomes, R₁₂ est un groupe alkyle en C₁-C₈ à chaîne droite ou ramifiée, aralkylène, aryle pouvant aussi comprendre des hétéroatomes, R₁₃ est un groupe alkyle en C₁-C₈ à chaîne droite ou ramifiée, R₁₄ est un groupe O-(alkyle en C₁-C₈), NH-(alkyle en C₁-C₈), N-(alkyle en C₁-C₈)₂, 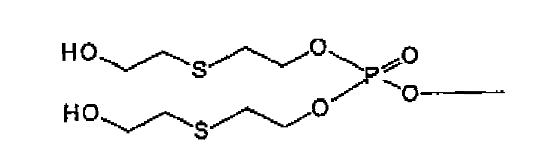 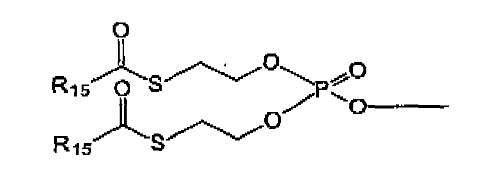 où
R₁₅ est un groupe alkyle en C₁-C₈ à chaîne droite ou ramifiée, 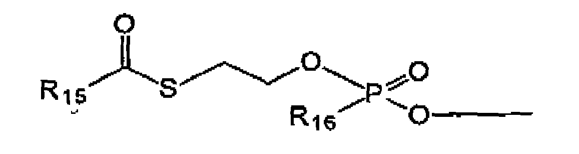 où
R₁₅ est un groupe alkyle en C₁-C₈ à chaîne droite ou ramifiée,
R₁₆ est un groupe alkyle en C₁-C₈ à chaîne droite ou ramifiée, O-aryle, 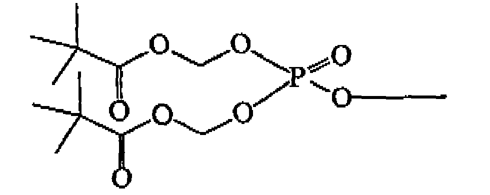 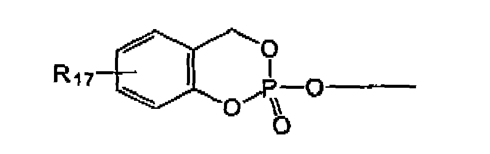 où
R₁₇ est un groupe alkyle en C₁-C₈ à chaîne droite ou ramifiée, halogéno, 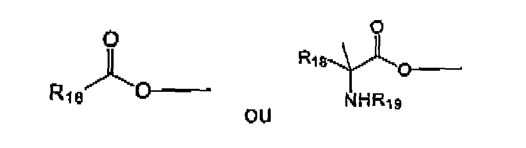 où,
R₁₈ est un groupe alkyle en C₁-C₈ à chaîne droite ou ramifiée, aryle ou hétéroaryle, R₁₉ est un groupe alkyl-CO-, aryl-CO-, un acide aminé ou un peptide, 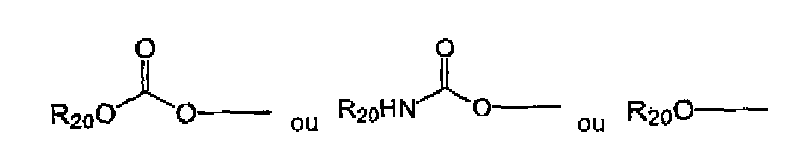 où
R₂₀ est un groupe alkyle en C₁-C₈ à chaîne droite ou ramifiée,
R₃ est H, un groupe alkyle en C₁-C₈ à chaîne droite ou ramifiée, alkylène en C₁-C₈ à chaîne droite ou ramifiée,
R₄ est H, un halogène, OR₈, N₃, NR₆R₇. ou R₄ et R₁ représentent ensemble une deuxième liaison entre les atomes de carbone voisins de R₁ et de R₄,
R₅ est H, un groupe alkyle en C₁-C₈ ou aryle, et
R₆, R₇ et R₈ représentent chacun indépendamment de l'autre H, un groupe alkyle en C₁-C₈ à chaîne droite ou ramifiée ou acétyle, les composés ayant les radicaux suivants étant exclus :
R₁ = F, R₂ = OH, R₃ = R₄ = R₅ = H,
R₁ = F, R₂ = O-acétyle, R₃ = R₄ = R₅ = H,
R₁ = F, R₂ = triphosphate, R₃ = R₄ = R₅ = H,
R₁ = Cl, R₂ = OH, R₃ = R₄ = R₅ = H.

2. Nucléoside selon la revendication 1, **caractérisés par** la formule II : 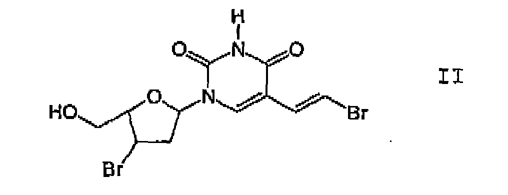 ou par la formule III : 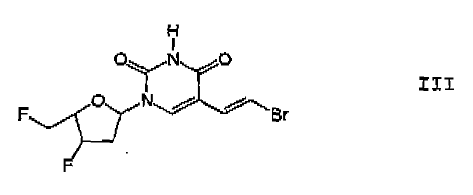 ou par la formule IV : 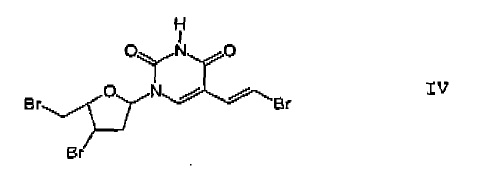 ou par la formule V : 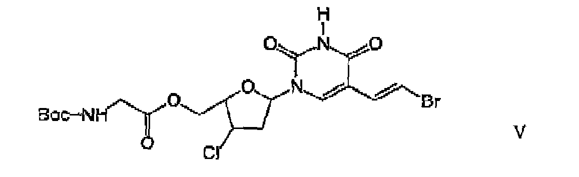 ou par la formule VI : 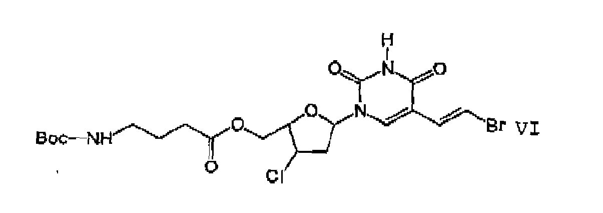 ou par la formule VII : 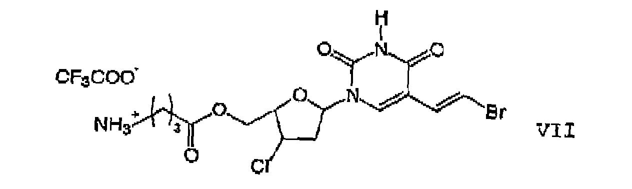 ou par la formule VIII

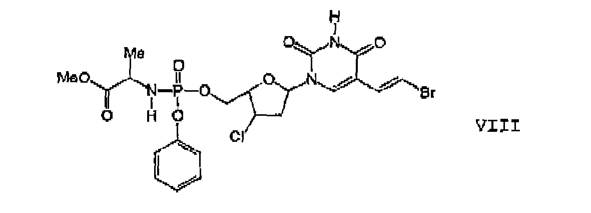

3. Nucléoside de formule générale IX 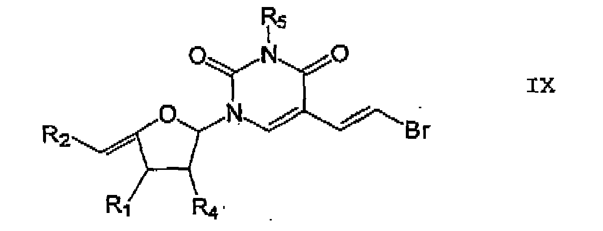 dans laquelle
R₁ et R₂ sont chacun indépendamment de l'autre choisis dans le groupe consistant en H, les halogènes, OR₈, CN, N₃, NR₆R₇ et les résidus prodrogues liés par l'intermédiaire de l'atome d'oxygène,
R₄ est un halogène,
R₅ est H, un groupe alkyle en C₁-C₈ ou aryle, et
R₆, R₇ et R₈ représentent chacun indépendamment des autres H, un groupe alkyle en C₁-C₈ à chaîne droite ou ramifiée, ou acétyle.

4. Médicament contenant au moins un nucléoside selon l'une des revendications précédentes.

5. Médicament selon la revendication 4, **caractérisé en ce que** le médicament contient en outre au moins un cytostatique.

6. Utilisation d'au moins un nucléoside de formule générale I 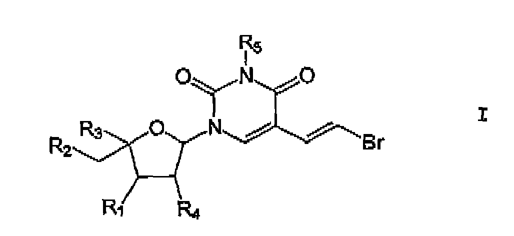 dans laquelle
R₁ est un halogène,
R₂ est choisi dans le groupe consistant en H, les halogènes, OR₈, CN, N₃, NR₆R₇ et les résidus prodrogues liés par l'intermédiaire d'un atome d'oxygène,
R₃ est H, un groupe alkyle en C₁-C₈ à chaîne droite ou ramifiée, alkylène en C₁-C₈ à chaîne droite ou ramifiée,
R₄ est H, un halogène, OR₈, N₃, NR₆R₇, ou R₄ et R₁ forment ensemble une deuxième liaison entre les atomes de carbone voisins de R₁ et R₄,
R₅ est H, un groupe alkyle en C₁-C₈ ou aryle, et
R₆, R₇ et R₈ représentent chacun indépendamment des autres H, un groupe alkyle en C₁-C₈ à chaîne droite ou ramifiée, ou acétyle,
ainsi que d'au moins un cytostatique pour la fabrication d'un médicament pour supprimer ou réduire la formation de résistances lors du traitement par des cytostatiques.

7. Utilisation selon la revendication 6, **caractérisée en ce que** le résidu prodrogue est choisi dans le groupe consistant en 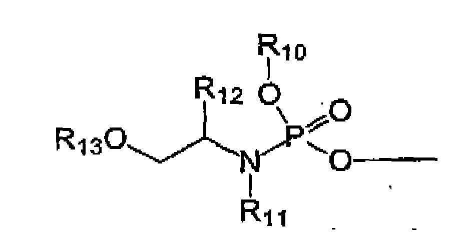 où
R₁₀ et R₁₁ représentent chacun indépendamment de l'autre un groupe alkyle en C₁-C₈ à chaîne droite ou ramifiée, aryle pouvant aussi comprendre des hétéroatomes, R₁₂ est un groupe alkyle en C₁-C₈ à chaîne droite ou ramifiée, aralkylène, aryle pouvant aussi comprendre des hétéroatomes, R₁₃ est un groupe alkyle en C₁-C₈ à chaîne droite ou ramifiée 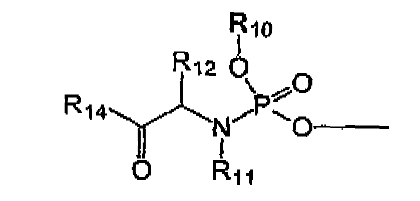 où
R₁₀ et R₁₁ représentent chacun indépendamment de l'autre un groupe alkyle en C₁-C₈ à chaîne droite ou ramifiée, aryle pouvant aussi comprendre des hétéroatomes, R₁₂ est un groupe alkyle en C₁-C₈ à chaîne droite ou ramifiée, aralkylène, aryle pouvant aussi comprendre des hétéroatomes, R₁₃ est un groupe alkyle en C₁-C₈ à chaîne droite ou ramifiée, R₁₄ est un groupe O-(alkyle en C₁-C₈), NH-(alkyle en C₁-C₈), N-(alkyle en C₁-C₈)₂, 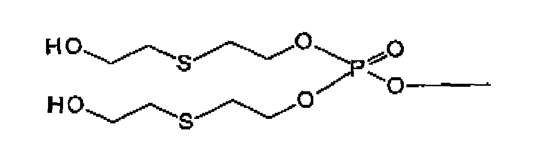 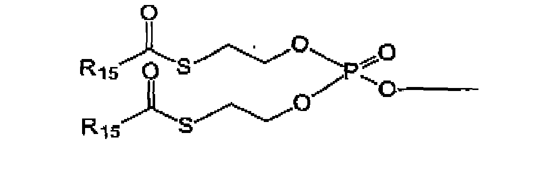 où
R₁₅ est un groupe alkyle en C₁-C₈ à chaîne droite ou ramifiée, 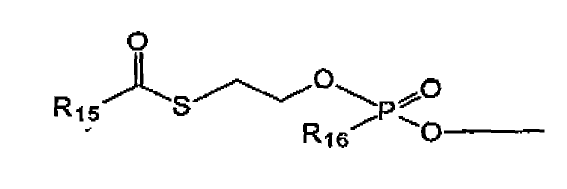 où
R₁₅ est un groupe alkyle en C₁-C₈ à chaîne droite ou ramifiée,
R₁₆ est un groupe alkyle en C₁-C₈ à chaîne droite ou ramifiée, O-aryle, 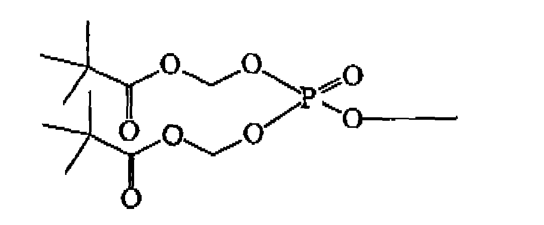 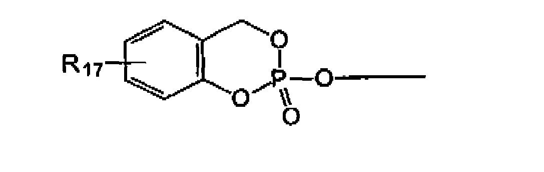 où
R₁₇ est un groupe alkyle en C₁-C₈ à chaîne droite ou ramifiée, halogéno, 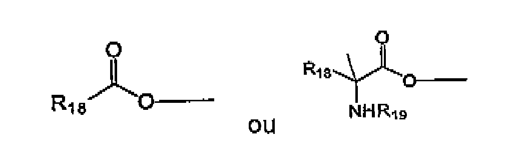 où,
R₁₈ est un groupe alkyle en C₁-C₈ à chaîne droite ou ramifiée, aryle ou hétéroaryle, R₁₉ est un groupe alkyl-CO-, aryl-CO- ou un acide aminé, 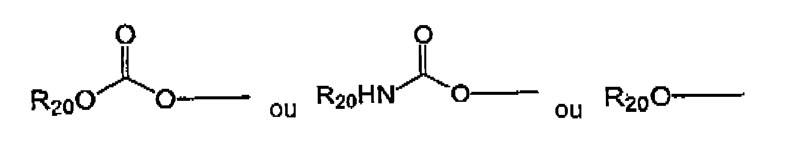 où
R₂₀ est un groupe alkyle en C₁-C₈ à chaîne droite ou ramifiée.

8. Utilisation d'au moins un nucléoside de formule générale IX selon la revendication 3, 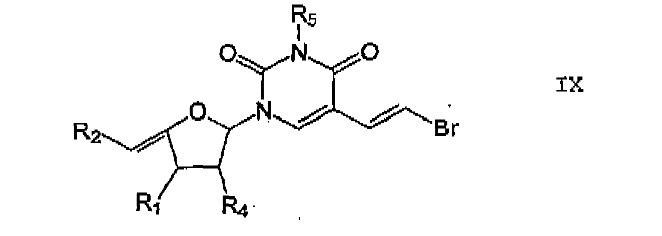 dans laquelle
R₁ et R₂ sont chacun indépendamment de l'autre choisis dans le groupe consistant en H, les halogènes, OR₈, CN, N₃, NR₆R₇ et les résidus prodrogues liés par l'intermédiaire de l'atome d'oxygène,
R₄ est un halogène,
R₅ est H, un groupe alkyle en C₁-C₈ ou aryle, et
R₆, R₇ et R₈ représentent chacun indépendamment des autres H, un groupe alkyle en C₁-C₈ à chaîne droite ou ramifiée, ou acétyle,
ainsi que d'au moins un cytostatique pour fabriquer un médicament pour la suppression ou la réduction de la formation de résistances lors du traitement par des cytostatiques.

9. Utilisation selon l'une des revendications 6 ou 7, **caractérisée en ce qu'**on utilise des nucléosides de formule X

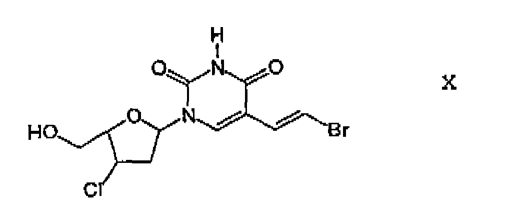

10. Utilisation selon l'une des revendications 6 à 9, **caractérisée en ce que** le ou les nucléosides et le ou les cytostatiques sont utilisés dans une formulation unique, ou que le ou les nucléosides et le ou les cytostatiques sont utilisés dans des formulations distinctes.

11. Utilisation d'au moins un nucléoside de formule générale I 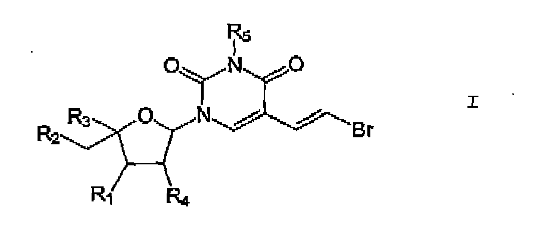 dans laquelle
R₁ est un halogène,
R₂ est choisi dans le groupe consistant en H, les halogènes, OR₈, CN, N₃, NR₆R₇ et les résidus prodrogues liés par l'intermédiaire d'un atome d'oxygène,
R₃ est H, un groupe alkyle en C₁-C₈ à chaîne droite ou ramifiée, alkylène en C₁-C₈ à chaîne droite ou ramifiée,
R₄ est H, un halogène, OR₈, N₃ ou NR₆R₇,
R₅ est H, un groupe alkyle en C₁-C₈ ou aryle, et
R₆, R₇ et R₈ représentent chacun indépendamment des autres H, un groupe alkyle en C₁-C₈ à chaîne droite ou ramifiée ou acétyle,
ainsi que d'au moins un principe actif pour fabriquer un médicament pour le traitement sans formation de résistances de maladies infectieuses provoquées par des bactéries, des plasmodiums ou des leishmanies.

12. Utilisation selon la revendication 11, **caractérisée en ce que** le ou les nucléosides et le ou les cytostatiques sont utilisés dans une formulation unique.
